# EUROPEAN PATENT APPLICATION

(11) **EP 4 806 372 A2**
(43) Date of publication of application: **16.09.2026**
(21) Application number: 26193787.4
(22) Date of filing: 13.02.2024
(51) Int. Cl.: A61B 17/12

(54) **VASO-OCCLUSIVE ELECTROLYTIC DETACHMENT DETECTION**

(30) Priority: 21.02.2023 US 202363486183 P
(62) Document number(s) of the earlier application(s) in accordance with Art. 76 EPC: 24711083.6 / 4 669 218
(71) Applicant: Stryker Corporation, Kalamazoo, MI 49002 (US); Stryker European Operations Limited, Carrigtwohill, Co. Cork T45 HX08 (IE)
(72) Inventor: BASHIRI, Mehran, San Carlos, 94070 (US); COHEN, Brandon, Kalamazoo, 49002 (US); LEE, Andrew S., San Jose, 95124 (US)
(74) Representative: Gillespie, Richard

(57) **Abstract**

An electrolytic detachment device for use with a delivery wire attached to a vaso-occlusive device via an electrolytically severable joint. The detachment device is configured for assessing if a successful electrolytic detachment event has occurred based on electrical parameter information, and resetting the electrical parameter information if the measured elapsed time reaches an elapsed time threshold. The detachment device is also configured for assessing if the successful electrolytic detachment event has occurred during a fixed time period of an electrolytic detachment cycle based on the generated electrical parameter information, and extending the electrolytic detachment cycle only if the successful electrolytic detachment event has been assessed to have not occurred during the fixed time period. The detachment device is also configured for incrementally increasing an electrolytic work threshold to which measured electrolytic work can be compared to assess if the electrolytic detachment event has occurred.

## Description

### FIELD OF THE INVENTION

The present disclosure relates generally to medical devices and intravascular medical procedures and more particularly, to devices and methods for detecting electrolytic detachment of vaso-occlusive devices.

### BACKGROUND

An aneurysm, which is a localized, blood-filled, dilation of a blood vessel that typically assumes a sac or balloon-like configuration that extends from a blood vessel and is caused by disease, blood flow/pressure exerted in the vessel, and/or weakening of the vessel wall, can rupture and cause hemorrhage, stroke (e.g., an intracranial aneurysm) and other damaging consequences to a patient. Approximately 25,000 intracranial aneurysms rupture each year in North America.

There are a variety of approaches to treat a ruptured or non-ruptured aneurysm including, e.g., an endovascular approach that involves delivering vaso-occlusive devices through an endovascular catheter into the aneurysm. Vaso-occlusive devices are commonly composed of self-expanding materials, so that when the devices are deployed from the delivery system into the target location in a patient, the unconstrained devices expand without requiring assistance. Self-expanding vaso-occlusive devices may be biased so as to expand upon release from the delivery catheter and/or include a shape-memory component that allows the device to expand upon exposure to a predetermined condition. Some vaso-occlusive devices may be characterized as hybrid devices, which have some characteristics of both self-expandable materials and non-self-expandable materials.

A typical endovascular approach includes two major steps. The first step involves the introduction of the catheter to the aneurysm site using devices, such as described in U.S. Patent Nos. 4,739,768 and 4,884,575, which are expressly incorporated herein by reference. The second step involves loading a vaso-occlusive device into the catheter via a delivery device (e.g., a delivery wire) in a collapsed or radially compressed delivery configuration and then introduced into an aneurysmal sac. In some embodiment, multiple vaso-occlusive devices (e.g., two) may be concurrently loaded and then serially introduced into the aneurysmal sac. Once delivered within the aneurysmal sac, the vaso-occlusive device may then be placed into an expanded configuration, filling and occluding the aneurysmal sac. Additional vaso-occlusive devices can be serially located into the catheter via delivery devices and delivered within the aneurysmal sac until the aneurysmal sac is completely filled with vaso-occlusive devices.

Vaso-occlusive devices may have a variety of sizes and shapes; however, vaso-occlusive devices for treatment of an aneurysm usually assume a spherical secondary configuration when deployed within the aneurysmal sac. When implanted within the aneurysmal sac, the vaso-occlusive device may further reinforce the inner walls of the aneurysmal sac while occluding the aneurysm, reducing the probability of rupture or preventing further rupture of the aneurysm. Vaso-occlusive devices can be composed from a variety of materials, including polymers (e.g., non-bioerodable and bioerodable plastics) and metals. Vaso-occlusive devices can be made from shape memory or superelastic materials, such as shape memory metals (e.g., shape memory Nitinol) and polymers (e.g., polyurethane). Such shape memory vaso-occlusive devices can be induced (e.g., by temperature, electrical or magnetic field or light) to take on a shape (e.g., a radially expanded shape) after delivery to a treatment site. Superelastic embolic materials, such as superelastic Nitinol, take on a shape after delivery without the need for an inductive stimulus. Other commonly used materials include stainless steel, platinum and elgiloy. Drug delivery vaso-occlusive devices can carry, and/or the surface of the device, can be coated with a bioactive or therapeutic agent (e.g., thrombosis inducing agent).

Commonly used vaso-occlusive devices include soft, helically wound coils formed by winding a platinum (or platinum alloy) wire strand about a "primary" mandrel. The coil is then wrapped around a larger, "secondary" mandrel, and heat treated to impart a secondary shape. For example, U.S. Pat. No. 4,994,069, which is expressly incorporated herein by reference, describes a vaso-occlusive device that assumes a linear, helical primary shape when stretched for placement through the lumen of a delivery catheter, and a folded, convoluted secondary shape when released from the delivery catheter and deposited in the vasculature. In order to better frame and fill aneurysms, complex three-dimensional secondary shapes can be imparted on vaso-occlusive devices and the stiffness/flexibility of vaso-occlusive devices can be modified. Other three-dimensional embolic coils have been described in U.S. Patent Nos. 5,624,461 (i.e., three-dimensional in-filling embolic coil), 5,639,277 (i.e., vaso-occlusive coils having twisted helical shapes) and 5,649,949 (i.e., variable cross-section conical vaso-occlusive coils). Vaso-occlusive coils having little or no inherent secondary shape have also been described, such as in U.S. Patent Nos. 5,690,666 and 5,826,587. Spherical shaped vaso-occlusive devices are described in U.S. Patent No. 5,645,558, which discloses that one or more strands can be wound to form a substantially hollow spherical or ovoid shape comprising overlapping strands when deployed in an aneurysm. Other vaso-occlusive devices that assume spherical shapes when deployed are described in U.S. Patent No. 8,998,947, which discloses tubular mesh having petal-like sections to form a substantially spherical shape having overlapping petals-like sections when deployed in an aneurysm, and U.S. Publication No. 2014/020060, which discloses a semi-spherical dome-like scaffolding that invert when opposing forces are exerted at the dome of the aneurysm, which is then filled with vaso-occlusive material.

Vaso-occlusive coils, such as those described in U.S. Pat. No. 4,994,069, may be delivered within the aneurysmal sac in a variety of manners, with 3-10 vaso-occlusive coils being typically delivered into a single aneurysmal sac. One highly desirable means of delivering a vaso-occlusive coil into an aneurysmal sac employs an electrolytic detachment procedure, such as that described in U.S. Patent No. 5,122,136, which is expressly incorporated herein by reference. After loading an electrically conductive delivery wire (e.g., composed of stainless steel) with an attached vaso-occlusive coil within the delivery catheter and distally advancing the delivery wire to insert the vaso-occlusive coil into the aneurysmal sac, such electrolytic detachment procedure involves severing the vaso-occlusive coil from the distal end of delivery wire by the application of a small electric current through the delivery wire to an electrolytically severable joint between the vaso-occlusive coil and the distal end of the delivery wire, thereby permanently delivering the detached vaso-occlusive coil into the aneurysmal sac.

When electrolytic detachment of the vaso-occlusive coil from the delivery wire occurs (a "successful electrolytic detachment event" has occurred), it is desirable to report such successful electrolytic detachment event has occurred at the time that it occurs, so that the physician may be prompted to remove the delivery wire from the delivery catheter and load the next vaso-occlusive coil and associated delivery wire into the delivery catheter for electrolytic detachment and delivery into the aneurysmal sac. Alternatively, if a successful electrolytic detachment event has not occurred within a reasonable amount of time, it is desirable to report to the physician such non-occurrence of a successful electrolytic detachment event non-detachment so that the physician may make another attempt at electrolytically detaching the vaso-occlusive coil from the delivery wire. Thus, typical electrolytic detachment devices include means for reporting whether or not a successful electrolytic detachment event has occurred to the physician. Some known electrolytic detachment detection systems measure one or more direct current (DC) and/or alternating current (AC) electrical parameters (e.g., impedance, voltage, etc.) during application of the small electrical current to the electrolytically severable joint and analyzing sudden changes in these electrical parameters to determine the likelihood that a successful electrolytic detachment event has occurred. Examples of such electrolytic detachment detection systems are described in U.S. Patent Nos. 5,643,254 and 6,397,850, which are expressly incorporated herein.

A handheld electrolytic detachment device, such as the InZone^{®} Detachment System, manufactured by Stryker^{®} Neurovascular, may be employed to deliver the small electrical current to the electrolytically severable joint between the vaso-occlusive coil and the distal end of the delivery wire, and detect and report detachment of the vaso-occlusive coil from the delivery wire. One embodiment of such a handheld electrolytic detachment device includes a power terminal to which the proximal end of the delivery wire is coupled (e.g., via insertion into a power port), and a ground terminal to which a ground electrode placed in contact with the patient (e.g., via percutaneous insertion into the patient) is coupled via an electrical cable (e.g., via insertion of a connector of the electrical cable into a ground port).

The handheld electrolytic detachment device may be operated (e.g., by depressing a button) on handle to perform one or more electrolytic detachment cycles (i.e., deliver one or more cycles of electrical current) to the electrolytically severable joint between the vaso-occlusive coil and the distal end of the delivery wire. The handheld electrolytic detachment device may, during performance of each electrolytic detachment cycle, measure and compare cumulative current delivered to the electrolytically severable joint (as a proxy to work electrolytic work performed by the handheld electrolytic detachment device) to a threshold value to determine the likelihood that the vaso-occlusive coil has electrolytically detached from the delivery wire. That is, it is assumed that if a certain amount of electrolytic work has been performed by the handheld electrolytic detachment device, then the vaso-occlusive coil must have electrolytically detached from the delivery wire.

Although desirable, it is not guaranteed that all vaso-occlusive coils will electrolytically detach from the delivery wire occur within the initial electrolytic detachment cycle. In particular, because the electrolytic detachment process is highly dependent on the environment surrounding the vaso-occlusive coil, the elapsed time between the time that electrical current is initially delivered to the electrolytically severable joint and the time that the vaso-occlusive coil electrolytically detaches from the delivery wire will vary. For example, the presence of high nonionic contrast, the presence of other embolic agents, contact between the electrolytically severable joint and previously delivered vaso-occlusive coils, the presence of thrombus on the electrolytically severable joint, poor longitudinal alignment between delivery wire and delivery catheter that may not fully expose the electrolytically severable joint to blood necessary for electrolysis to occur, problems with grounding, such as a corroded electrical cable or poor electrical contact between the ground electrode and the patient, etc., may adversely affect the elapsed time needed for successful detachment of the vaso-occlusive coil from the deliver wire. If the environmental electrolytic conditions are ideal, initiation of the electrolytic detachment cycle will quickly result in successful detachment of the vaso-occlusive coil from the delivery wire. For a particular set of vaso-occlusive coils, initiation of the electrolytic detachment cycle will likely result in successful detachment of the vaso-occlusive coil from the delivery wire within five seconds.

However, if one or more of the environment conditions are not ideal, a longer elapsed time will be needed, sometimes longer than five seconds. In fact, empirical studies have shown that 30% of successful electrolytic detachments of some types of vaso-occlusive coils from their corresponding delivery wires occur after five seconds. Thus, in approximately 30% of electrolytic detachment cases, if the electrolytic detachment cycle is only five seconds in length, at least one additional electrolytic detachment cycle would need to be performed to effect successful electrolytic detachment of the vaso-occlusive device from the delivery wire. Although electrolytic detachment devices may be designed to have an electrolytic detachment cycle time of greater than five seconds in length (e.g., one minute in length) that ensures that successful detachment of the vaso-occlusive coil from the delivery wire will occur at the end of the electrolytic detachment cycle. However, by extending the electrolytic detachment cycle time, many, if not most, of the electrolytic detachment procedures will be unnecessarily extended for those electrolytic detachment cycle that effect successful electrolytic detachments of the vaso-occlusive coils well before the electrolytic detachment cycle time. Thus, an electrolytic detachment cycle of five seconds has been determined to be optimal for certain types of vaso-occlusive coils.

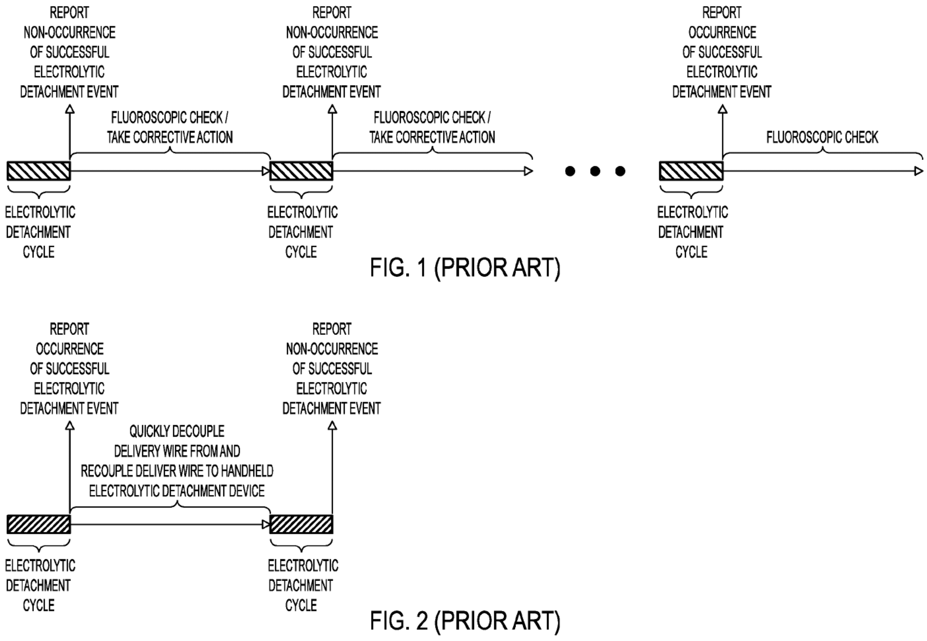

As such, an electrolytic detachment device may have to be sequentially operated multiple times to electrolytically detach a vaso-occlusive coil from the delivery wire. For example, as shown in **Fig. 1****,** the handheld electrolytic detachment device may be operated (e.g., by repeatedly depressing a button) to perform an exemplary sequence of fixed electrolytic detachment cycles (i.e., serially deliver cycles of electrical current during fixed time periods (e.g., five second time periods to the electrolytically severable joint between the vaso-occlusive coil and the distal end of the delivery wire), with the last electrolytic detachment cycle successfully resulting in electrolytic detachment of the vaso-occlusive coil from the delivery wire. Prior to initiating such exemplary electrolytic detachment sequence, it is assumed that the vaso-occlusive coil has been inserted into the aneurysmal sac of the patient, as briefly described above, and that the proximal end of the delivery wire has been inserted into the power port of the handheld electrolytic detachment device, and the electrical cable connected to the ground electrode placed in contact with the patient is inserted into the ground port of the handheld electrolytic detachment device.

During each electrolytic detachment cycle, the handheld electrolytic detachment device measures and analyzes one or more electrical parameters in the electrical circuit between the delivery wire and ground electrode, and then, at the end of the electrolytic detachment cycle, determines and reports whether or not the vaso-occlusive coil has successfully electrolytically detached from the delivery wire. As discussed above, such measured electrical parameters may at least comprise cumulative current (although other types of electrical parameters (e.g., moving impedance averages) may be measured and analyzed as well) and compared to a threshold value to determine the likelihood that the vaso-occlusive coil has electrolytically detached from the delivery wire. Based on this comparison, the handheld electrolytic detachment device will report to the physician at the end of the electrolytic detachment cycle as to whether or not a successful electrolytic detachment event has occurred.

Thus, as illustrated in **Fig. 1****,** at the end of each electrolytic detachment cycle prior to the last electrolytic detachment cycle, the handheld electrolytic detachment device has assessed that the vaso-occlusive coil has not electrolytically detached from the delivery wire and reported to the physician that a successful electrolytic detachment event has not occurred, thereby prompting the physician to initiate a subsequent electrolytic detachment cycle via operation of the electrolytic detachment cycle. In contrast, at the end of the last electrolytic detachment cycle, the handheld electrolytic detachment device has assessed that the vaso-occlusive coil has electrolytically detached from the delivery wire and reported to the physician that a successful electrolytic detachment event has occurred, thereby prompting the physician to perform an electrolytic detachment procedure for a new vaso-occlusive coil.

Because the electrolytic detachment of the vaso-occlusive coil from the delivery wire does not always occur at the end of an electrolytic detachment cycle, prudent practice dictates that the physician always confirm after the completion of each electrolytic detachment cycle that the vaso-occlusive coil has, in fact, either been electrolytically detached from the delivery wire or has not been electrolytically detached from the delivery wire.

For example, in the case where the handheld electrolytic detachment device detects and reports to the physician that a successful electrolytic detachment event has not occurred at the end of a particular electrolytic detachment cycle, the physician may confirm that the vaso-occlusive coil has, in fact, not been electrolytically detached from the delivery wire. In particular, the physician may remove the proximal end of the delivery wire from the handheld electrolytic detachment device, slightly pull the delivery wire in the proximal direction (e.g., 1-2 mm), and confirm, under fluoroscopy, that the vaso-occlusive coil has moved with the delivery wire, and thus, has not been successfully electrolytically detached from the delivery wire. Notably, movement of the vaso-occlusive coil during this confirmation procedure may favorably change the environmental electrolytic conditions, thereby increasing the likelihood that the vaso-occlusive coil may successfully electrolytically detach from the delivery wire during the next electrolytic detachment attempt. The physician may take other corrective actions, e.g., ensuring that the delivery wire is properly aligned with the delivery catheter, such that the electrolytically severable joint is fully exposed to blood, operating a flushing system to clear any contrast agent surrounding the vaso-occlusive device, checking the ground electrode to ensure that it is in stable contact with the patient, etc.

In the case where the handheld electrolytic detachment device detects and reports that a successful electrolytic detachment event has occurred at the end of a particular electrolytic detachment cycle, the physician may confirm that the vaso-occlusive coil has, in fact, been detached from the delivery wire even in the case the handheld electrolytic detachment device has assessed and reported a successful electrolytic detachment event to the physician. For example, the physician may decouple the proximal end of the delivery wire from the handheld electrolytic detachment device, slightly pull the delivery wire in the proximal direction (e.g., 1-2mm), and confirm, under fluoroscopy, that the vaso-occlusive coil has not moved with the delivery wire, and thus, has, indeed, successfully electrolytically detached from the delivery wire.

Thus, as illustrated in **Fig. 1****,** the physician may perform fluoroscopic visualization checks and take corrective action immediately after each electrolytic detachment cycle for which the handheld electrolytic detachment device has reported that a successful electrolytic detachment event has not occurred, and perform only a fluoroscopic visualization check (without corrective action) after the last electrolytic detachment cycle for which the handheld electrolytic detachment device has reported that a successful electrolytic detachment event has occurred.

If fluoroscopic visualization reveals that the vaso-occlusive coil has actually moved with the delivery wire, and thus, has not been successfully electrolytically detached from the delivery wire (i.e., a false positive electrolytic detachment detection has occurred), the physician may slightly push the delivery wire in the distal direction to reposition the vaso-occlusive coil fully within the aneurysmal sac, reinsert the proximal end of the delivery wire into the power port of the handheld electrolytic detachment device, and operate the handheld electrolytic detachment device again to perform another electrolytic detachment cycle. Thus, instead of performing only a fluoroscopic visualization check at the end of the last electrolytic detachment cycle illustrated in **Fig. 1****,** the physician may perform a fluoroscopic visualization check and take corrective action immediately after the current electrolytic detachment cycle, and operate the handheld electrolytic detachment device to continue the electrolytic detachment procedure.

In some cases where the handheld electrolytic detachment device detects and reports to the physician that a successful electrolytic detachment event has occurred at the end of the current electrolytic detachment cycle ( whether or not such positive electrolytic detachment detection is true), a "double-press" physician might, out of an abundance of caution, operate the handheld electrolytic detachment device again to perform an additional electrolytic detachment cycle without performing a fluoroscopic visualization confirmation of successful electrolytic detachment of the vaso-occlusive coil from the delivery wire. In these cases, such "double-press" physicians may decouple the proximal end of the delivery wire from the handheld electrolytic detachment device, and then quickly recouple (without performing a fluoroscopic visualization confirmation) the proximal end of the delivery wire to the handheld electrolytic detachment device, as illustrated **Fig. 2****.**

In the case where the handheld electrolytic detachment device detects and reports to the physician that a successful electrolytic detachment event has not occurred at the end of the current electrolytic detachment cycle (whether or not such negative electrolytic detachment detection is true), the physician may confirm that the vaso-occlusive coil has, in fact, not been detached from the delivery wire. Again, the physician may remove the proximal end of the delivery wire from the handheld electrolytic detachment device, slightly pull the delivery wire in the proximal direction (e.g., 1-2mm), and confirm, under fluoroscopy, that the vaso-occlusive coil has moved with the delivery wire, and thus, has not, indeed, successfully electrolytically detached from the delivery wire. The mere physical manipulation of the vaso-occlusive coil serves as a corrective action that favorably modifies the electrolytic conditions. The physician may take other corrective actions, e.g., ensuring that the delivery wire is properly aligned with the delivery catheter, such that the electrolytically severable joint is fully exposed to blood, operating a flushing system to clear any contrast agent surrounding the vaso-occlusive device, checking the ground electrode to ensure that it is in stable contact with the patient, etc. Rather than immediately performing a fluoroscopic visualization confirmation and/or taking corrective action, a "double-press" physician might simply operate the handheld electrolytic detachment device again to perform an additional electrolytic detachment cycle. Again, in these cases, such "double-press" physicians may decouple the proximal end of the delivery wire from the handheld electrolytic detachment device, and then quickly recouple (without performing a fluoroscopic visualization confirmation and/or taking corrective action) the proximal end of the delivery wire to the handheld electrolytic detachment device, as illustrated **Fig. 2****.**

If the vaso-occlusive coil has not been successfully detached from the delivery wire (e.g., after fluoroscopic visualization reveals that there was a false positive electrolytic detachment detection or a true negative electrolytic detachment detection), the physician may recouple the proximal end of the delivery wire to the handheld electrolytic detachment device, and operate the handheld electrolytic detachment device again to perform an electrolytic detachment cycle, thereby continuing the electrolytic detachment procedure in another attempt to electrolytically detach the vaso-occlusive coil from the delivery wire. Such electrolytic detachment attempts may be repeated until the vaso-occlusive coil, in fact, electrolytically detaches from the delivery wire.

Because fluoroscopic confirmation that a vaso-occlusive coil has either successfully electrolytically detached from the delivery wire (in the case of a reported successful electrolytic detachment) or not successfully electrolytically detached from the delivery wire (in the case of a reported unsuccessful electrolytic detachment) is time-consuming, thereby potentially extending the procedure time, it is desirable that fluoroscopic confirmation be performed only one time for each vaso-occlusive coil and that manipulation of the delivery wire with the intention of improving the environment conditions be minimized if not eliminated altogether.

It is possible to design an electrolytic detachment device to have a variable electrolytic detachment cycle time (e.g., the electrolytic detachment cycle time may not have a fixed period of time, e.g., five seconds), such that electrical current delivered to the electrolytically severable joint is terminated upon detection and reporting of a successful electrolytic detachment of the vaso-occlusive coil from the delivery wire, thereby minimizing the number of electrolytic detachment cycles needed (mostly likely to only one) and associated fluoroscopic visualization confirmations. However, the use of a fixed electrolytic detachment cycle minimizes the occurrence of false positive electrolytic detachment detections (i.e., the detection of a successful electrolytic detachment of a vaso-occlusive coil from the delivery wire when, in fact, the vaso-occlusive coil has not electrolytically detached from the delivery wire). That is, by delivering electrical current to the electrolytically severable joint for an entire fixed electrolytic detachment cycle, there is a greater chance that the vaso-occlusive coil, in fact, electrolytically detaches from the delivery wire compared to terminating the delivery of electrical current to the electrolytically severable joint during a variable electrolytic detachment cycle upon detection and reporting of a successful electrolytic detachment of the vaso-occlusive coil from the delivery wire.

Furthermore, if variable electrolytic detachment cycles are used, in certain cases when the environmental electrolytic conditions are not ideal, it may not be desirable to wait for the vaso-occlusive coil to electrolytically detach from the delivery wire. That is, rather than continuing to delivery electrical current to the electrolytically severable joint during non-ideal environmental electrolytic conditions, it may be desirable to use a fixed electrolytic detachment cycle that unconditionally terminates at a known end time to allow the physician to alter the environmental electrolytic conditions, e.g., by realigning the delivery wire and delivery catheter, in an attempt to render the environmental electrolytic conditions more ideal, and then attempting another fixed electrolytic detachment cycle under the more ideal environmental electrolytic conditions. Thus, by having a fixed electrolytic detachment cycle with an appropriate unconditional end time, the physician will be prompted at the termination of the fixed electrolytic detachment cycle to make corrective actions (i.e., to attempt to render the environmental electrolytic conditions more ideal) prior to continuing with electrical current delivery to the electrolytically severable joint.

There, thus, remains a need to minimize the number of electrolytic detachment cycles needed to successfully electrolytically detach a given vaso-occlusive coil from the delivery wire, while minimizing the number of false positive electrolytic detachment detection while also prompting the physician to perform corrective actions when needed.

Because it is presumed that the vaso-occlusive coil will electrolytically detach from the delivery wire by the end of the initial electrolytic detachment cycle (despite the fact that a minority of vaso-occlusive coils may not electrolytically detach from the delivery wire by the end of the initial electrolytic detachment cycle), the handheld electrolytic detachment device anticipates that the next electrolytic detachment cycle will be applied to a new distally attached vaso-occlusive coil. To prevent electrolytic detachment detection information (e.g., the measured cumulative current and any thresholds) related to the current vaso-occlusive coil from being transferred to the new vaso-occlusive coil, the handheld electrolytic detachment device may automatically reset the electrolytic detachment detection information that may be used to detect the electrolytic detachment of the new vaso-occlusive coil from the delivery wire. Such reset might occur when the proximal end of the delivery wire associated with the new vaso-occlusive coil is coupled to the handheld electrolytic detachment device and/or when the handheld electrolytic detachment device is operated to deliver another electrolytic detachment cycle. In this manner, the handheld electrolytic detachment device will recognize that no electrolytic work has been performed at the beginning of the next electrolytic detachment cycle preformed in an attempt to electrolytically detach the new vaso-occlusive coil from delivery wire.

While this will not be problematic in the case where the vaso-occlusive coil has, in fact, electrolytically detached from the delivery wire during the current electrolytic detachment cycle, and such successful electrolytic detachment has been confirmed to have occurred, in the case where the current vaso-occlusive coil has not, in fact, electrolytically detached from the delivery wire during the current electrolytic detachment cycle, prematurely resetting the electrolytic detachment detection information may adversely affect the ability of the handheld electrolytic detachment device to accurately determine whether or not the current vaso-occlusive coil has electrolytically detached from the delivery wire during the next electrolytic detachment cycle. For example, if the state of the measured cumulative current is not carried over to the next electrolytic detachment cycle, the measured cumulative current that the handheld electrolytic detachment device compares to the cumulative current threshold might be too small to ever meet the cumulative current threshold to which it will be compared, thereby preventing the handheld electrolytic detachment device from detecting successful electrolytic detachment of the vaso-occlusive coil from the delivery wire during the next electrolytic detachment cycle, and as such, incorrectly reporting that a successful electrolytic detachment event has not occurred to the physician (i.e., resulting in a false negative electrolytic detachment detection). Such premature resetting of the electrical parameter information may result from recoupling the same delivery wire to the handheld electrolytic detachment device (e.g., when fluoroscopic visualization does not confirm that the vaso-occlusive device has successfully detached from the delivery wire in the case of a false positive electrolytic detachment detection, or when fluoroscopic visualization confirms that the vaso-occlusive device has not successfully detached from the delivery wire in the case of a true negative electrolytic detachment detection, or when the handheld electrolytic detachment device is merely operated one more time to perform another electrolytic detachment cycle without fluoroscopic visualization confirmation (e.g., in a "double-press event" in the case of a true negative electrolytic detachment detection or a false positive electrolytic detachment cycle detection).

In the case where the current vaso-occlusive coil has, in fact, electrolytically detached from the delivery wire during the current electrolytic detachment cycle, premature resetting of the electrical parameter information may still occur if the same delivery wire is immediately recoupled to the handheld electrolytic detachment device (without fluoroscopic visualization conformation) and the handheld electrolytic device is operated one more time to perform another electrolytic detachment cycle on the currently detached vaso-occlusive coil (e.g., in a double-press event in the case of a true positive electrolytic detachment detection or a false negative electrolytic detachment cycle detection), thereby preventing the handheld electrolytic detachment device from detecting successful electrolytic detachment of the vaso-occlusive coil from the delivery wire during the next electrolytic detachment cycle, and as such, incorrectly reporting that a successful electrolytic detachment event has not occurred to the physician (i.e., resulting in a false negative electrolytic detachment detection).

Furthermore, in the case where a successful electrolytic detachment event has been reported to occur during the current electrolytic detachment cycle (regardless of whether the current vaso-occlusive coil has, in fact, electrolytically detached from the delivery wire during the current electrolytic detachment cycle), a sequence that initially reports that a successful electrolytic detachment event has occurred and then subsequently reports that a successful electrolytic detachment has not occurred, as illustrated in **Fig. 2****,** may be confusing for the physician.

One solution that might prevent premature resetting of the electrolytic detachment detection information is to provide the handheld electrolytic detachment device with a control (e.g., a button) that the physician may manually actuate to reset the electrolytic detachment detection information, thereby ensuring that such electrolytic detachment detection information is only reset after the current vaso-occlusive coil has been confirmed to have been electrolytically detached from the delivery wire. However, this requires an additional operational step in the electrolytic detachment procedure that the physician may not remember to perform or may otherwise not want to perform.

There, thus, remains a need to provide an electrolytic detachment device that automatically resets the electrolytic detachment information only after it has been deemed that a successful electrolytic detachment event has, in fact, occurred.

The handheld electrolytic detachment device presumes that the electrolytic work needed to electrolytically detach a vaso-occlusive coil from the delivery wire remains the same throughout the entire electrolytic detachment procedure, and thus, the threshold to which the handheld electrolytic detachment device compares the measured cumulative current is constant. However, it has been discovered that a significant portion of the electrolytic work (i.e., the cumulative current) is wasted on parasitic reactions in the vicinity of the vaso-occlusive coil, which increases as the electrolytic detachment procedure proceeds in time. However, since the threshold to which the measured cumulative current is compared is constant, the measured cumulative current may prematurely reach such threshold, thereby causing the handheld electrolytic detachment device to detect and report a false positive electrolytic detachment detection (i.e., incorrectly detect and report that the vaso-occlusive coil has electrolytically detached from the delivery wire).

There, thus, remains a need to minimize the chance of a false positive electrolytic detachment detection based on the measurement of electrolytic work performed by the handheld electrolytic detachment device (i.e., measured cumulative current).

### SUMMARY

In accordance with the present inventions, an electrolytic detachment device for use with an electrically conductive delivery wire having a distal end to which a respective vaso-occlusive device is attached and an electrolytically severable joint located proximal to the vaso-occlusive device is provided.

The electrolytic detachment device comprises a power terminal to which a proximal end of the delivery wire is configured for being electrically coupled, and from which the proximal end of the delivery wire is configured for being electrically decoupled. The power terminal may be, e.g., a power port into which the proximal end of the delivery wire is configured for being inserted. In one embodiment, the electrolytic detachment device further comprises a ground terminal to which a ground electrode in electrical contact with the patient is configured for being electrically coupled via an electrical cable.

The electrolytic detachment device further comprises electrical current delivery circuitry configured for delivering electrical current to the electrolytically severable joint of the vaso-occlusive assembly while the vaso-occlusive device is disposed within a vasculature of a patient, such that the vaso-occlusive device electrolytically detaches from the distal end of the delivery wire. The electrolytic detachment device further comprises an electrolytic detachment detection circuit configured for generating electrical parameter information (e.g., a measured cumulative electrical parameter, such as measured cumulative current, or measured electrolytic work performed by the electrical current delivery circuitry) indicative of a successful electrolytic detachment event.

The electrolytic detachment device further comprises a controller configured for assessing if the successful electrolytic detachment event has occurred based on the generated electrical parameter information. In one embodiment, the controller is configured for generating a first user-discernible notification if the successful electrolytic detachment event has been assessed to have occurred, and the controller is configured for generating a second user-discernible notification different from the first user-discernible notification if the successful electrolytic detachment event has been assessed to have not occurred. In another embodiment, the electrolytic detachment device further comprises an electrical current delivery actuator, in which case, the controller may be configured for operating the electrical current delivery circuitry to deliver the electrical current to the electrolytically severable joint during an electrolytic detachment cycle in response to a single actuation of the electrical current delivery actuator, and operating the electrical current delivery circuitry to deliver the electrical current to the electrolytically severable joint during another electrolytic detachment cycle in response to another single actuation of the electrical current delivery actuator.

In accordance with a first aspect of the present inventions, the electrolytic detachment device further comprises a timer configured for measuring an elapsed time between a delivery wire decoupling event and a subsequent delivery wire coupling event. The controller is further configured resetting the electrical parameter information if the measured elapsed time reaches a first elapsed time threshold (e.g., equal to or greater than three seconds, e.g., in the range of 25-45 seconds). In one embodiment, the controller is configured for resetting the electrical parameter information if the measured elapsed time reaches the first elapsed time threshold and the successful electrolytic detachment event has been assessed to have occurred, for resetting the electrical parameter information if the measured elapsed time reaches a second elapsed time threshold longer than the first elapsed time threshold and the successful electrolytic detachment event has been assessed to have not occurred. In this case, the first elapsed time threshold may be, e.g., in the range of 3-10 seconds, and the second elapsed time threshold may be, e.g., in the range of 25-45 seconds.

In accordance with a second aspect of the present inventions, the controller is configured for operating the electrical current delivery circuitry to deliver the electrical current to the electrolytically severable joint during an electrolytic detachment cycle having a fixed time period, and extending the electrolytic detachment cycle only if the successful electrolytic detachment event has been assessed to have not occurred during the fixed time period.

In one embodiment, the fixed time period is an initial fixed time period, in which case, the controller is configured for extending the electrolytic detachment cycle by a subsequent variable time period. The controller may be configured for incrementally assessing if the successful electrolytic detachment event has occurred during the subsequent variable time period, and terminating the electrolytic detachment cycle when the successful electrolytic detachment event is assessed to have occurred. The subsequent variable time period may have a plurality time increments, each of which is shorter than the initial fixed time period of the electrolytic detachment cycle. In this case, the controller may be configured for incrementally assessing that if the successful electrolytic detachment event has occurred respectively during the plurality of time increments, and terminating the electrolytic detachment cycle at the end of the time increment at which the successful electrolytic detachment event has been assessed to have occurred.

In another embodiment, the controller is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint. For example, if the electrical parameter information comprises measured electrolytic work performed by the electrical current delivery circuitry, the controller may be configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.

In still another embodiment, the electrolytic detachment device further comprises a counter configured for tracking a number of completed electrical detachment cycles, in which case, the controller may be configured for not extending the electrolytic detachment cycle if the tracked number of completed electrolytic detachment cycles exceeds a predefined number of electrolytic detachment cycles.

In accordance with a third aspect of the present inventions, the electrolytic detachment detection circuit is configured for incrementally measuring electrolytic work performed by the electrical current delivery circuitry over time (e.g., by incrementally measuring cumulative current delivered by the electrical current delivery circuitry over time), thereby generating a plurality of measured electrolytic work values, and the controller is further configured incrementally increasing an electrolytic work threshold over time, thereby generating a plurality of electrolytic work threshold values, respectively comparing the plurality of measured electrolytic work values to the plurality of electrolytic work threshold values, and assessing if a successful electrolytic detachment event has occurred based on the comparisons.

In accordance with the present inventions, a vaso-occlusive treatment system is provided.

The vaso-occlusive treatment system comprises a delivery catheter configured for being introduced into a vasculature of a patient. The delivery catheter comprises an elongate sheath body, an inner lumen extending through the elongate sheath body, and a distal port in respective communication with the inner lumen.

The vaso-occlusive treatment system further comprises a vaso-occlusive assembly configured for being disposed within inner lumen of the delivery catheter. The vaso-occlusive assembly comprises a delivery wire and a vaso-occlusive device (e.g., a vaso-occlusive coil) detachably coupled to the delivery wire via an electrolytically severable joint. The vaso-occlusive device configured for being deployed out from the distal port of the delivery catheter into the vasculature of the patient.

The vaso-occlusive treatment system further comprises an electrolytic detachment device (e.g., a handheld electrolytic detachment device) to which a proximal end of the delivery wire of the vaso-occlusive assembly is configured for being electrically coupled, and from which the proximal end of the delivery wire of the vaso-occlusive assembly is configured for being electrically decoupled, delivering electrical current to the electrolytically severable joint of the vaso-occlusive assembly while the vaso-occlusive device is disposed within the vasculature of the patient, such that the vaso-occlusive device electrolytically detaches from the distal end of the delivery wire, generating electrical parameter information (e.g., a measured cumulative electrical parameter, such as measured cumulative current, or measured electrolytic work performed by the electrolytic detachment device) indicative of a successful electrolytic detachment event, and assessing if the successful electrolytic detachment event has occurred based on the generated electrical parameter information.

In one embodiment, the vaso-occlusive treatment system further comprises a ground electrode configured for being placed in electrical contact with the patient and for being electrically coupled to the electrolytic detachment device via an electrical cable.

In another embodiment, the electrolytic detachment device is configured for generating a first user-discernible notification if the successful electrolytic detachment event has been assessed to have occurred, and for generating a second user-discernible notification different from the first user-discernible notification if the successful electrolytic detachment event has been assessed to have not occurred. In still another embodiment, the electrolytic detachment device is configured for delivering the electrical current to the electrolytically severable joint during an electrolytic detachment cycle in response to a single actuation of the electrolytic detachment device, and delivering the electrical current to the electrolytically severable joint during another electrolytic detachment cycle in response to another single actuation of the electrolytic detachment device.

In accordance with a fourth aspect of the present invention, the electrolytic detachment device is further configured for measuring an elapsed time between a delivery wire decoupling event and a subsequent delivery wire coupling event, and resetting the electrical parameter information if the measured elapsed time reaches a first elapsed time threshold (e.g., equal to or greater than three seconds, e.g., in the range of 25-45 seconds).

In one embodiment, the electrolytic detachment device is configured for resetting the electrical parameter information if the measured elapsed time reaches the first elapsed time threshold and the successful electrolytic detachment event has been assessed to have occurred, and for resetting the electrical parameter information if the measured elapsed time reaches a second elapsed time threshold longer than the first elapsed time threshold and the successful electrolytic detachment event has been assessed to have not occurred. In this case, the first elapsed time threshold may be, e.g., in the range of 3-10 seconds, and the second elapsed time threshold may be, e.g., in the range of 25-45 seconds. In one example, the delivery catheter may comprise another inner lumen extending through the elongate sheath body, and another distal port in respective communication with the other inner lumen. The vaso-occlusive treatment system may further comprise another vaso-occlusive assembly configured for being disposed within inner lumen of the delivery catheter, with the other vaso-occlusive assembly comprising a delivery wire and a vaso-occlusive device detachably coupled to the delivery wire via an electrolytically severable joint, and the other vaso-occlusive device configured for being deployed out from the other distal port of the delivery catheter into the vasculature of the patient. The proximal end of the delivery wire of the other vaso-occlusive assembly may be configured for being electrically coupled to the electrolytic detachment device, and the proximal end of the delivery wire of the other vaso-occlusive assembly may be configured for being electrically decoupled from the electrolytic detachment device. The electrolytic detachment device may be configured for delivering electrical current to the electrolytically severable joint of the vaso-occlusive assembly while the other vaso-occlusive device is disposed within the vasculature of the patient, such that the vaso-occlusive device electrolytically detaches from the distal end of the delivery wire. The delivery wire decoupling event comprises decoupling the delivery wire of the vaso-occlusive assembly from the electrolytic detachment device, and the subsequent delivery wire coupling event comprises either recoupling the delivery wire of the vaso-occlusive assembly to the electrolytic detachment device or coupling the delivery wire of the other vaso-occlusive assembly to the electrolytic detachment device.

In accordance with a fifth aspect of the present inventions, the electrolytic detachment device is configured delivering electrical current, during an electrolytic detachment cycle having a fixed time period, to the electrolytically severable joint of the vaso-occlusive assembly while the vaso-occlusive device is disposed within the vasculature of the patient, such that the vaso-occlusive device electrolytically detaches from the distal end of the delivery wire, and extending the electrolytic detachment cycle only if the successful electrolytic detachment event has been assessed to have not occurred during the fixed time period.

In one embodiment, the fixed time period is an initial fixed time period, in which case, the electrolytic detachment device is configured for extending the electrolytic detachment cycle by a subsequent variable time period. The electrolytic detachment device may be configured for incrementally assessing if the successful electrolytic detachment event has occurred during the subsequent variable time period, and terminating the electrolytic detachment cycle when the successful electrolytic detachment event is assessed to have occurred. The subsequent variable time period may have a plurality time increments, each of which is shorter than the initial fixed time period of the electrolytic detachment cycle. In this case, the electrolytic detachment device may be configured for incrementally assessing that if the successful electrolytic detachment event has occurred respectively during the plurality of time increments, and terminating the electrolytic detachment cycle at the end of the time increment at which the successful electrolytic detachment event has been assessed to have occurred.

In another embodiment, the electrolytic detachment device is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint. For example, if the electrical parameter information comprises measured electrolytic work performed by the electrical current delivery circuitry, the electrolytic detachment device may be configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.

In still another embodiment, the electrolytic detachment device is configured for tracking a number of completed electrical detachment cycles, in which case, the electrolytic detachment device may be configured for not extending the electrolytic detachment cycle if the tracked number of completed electrolytic detachment cycles exceeds a predefined number of electrolytic detachment cycles.

In accordance with a sixth aspect of the present inventions, the electrolytic detachment device is configured for incrementally measuring electrolytic work performed by the electrolytic detachment device over time (e.g., by incrementally measuring cumulative current delivered by the electrolytic detachment device over time), thereby generating a plurality of measured electrolytic work values, and the electrolytic detachment device is further configured incrementally increasing an electrolytic work threshold over time, thereby generating a plurality of electrolytic work threshold values, respectively comparing the plurality of measured electrolytic work values to the plurality of electrolytic work threshold values, and assessing if a successful electrolytic detachment event has occurred based on the comparisons.

In accordance with the present inventions, a method of occluding a vasculature of a patient using a vaso-occlusive assembly comprising a delivery wire and a vaso-occlusive device (e.g., a vaso-occlusive coil) detachably coupled to a distal end of the delivery wire via an electrolytically severable joint is provided.

The method comprises introducing a delivery catheter configured into a vasculature of a patient, disposing the vaso-occlusive assembly within the delivery catheter, such that the vaso-occlusive device is disposed outside of the delivery catheter within the vasculature of the patient (e.g., an aneurysmal sac in the vasculature of the patient), electrically coupling a proximal end of the delivery wire of the vaso-occlusive assembly to a power source, delivering electrical current from the power source to the electrolytically severable joint of the vaso-occlusive assembly while the vaso-occlusive device is disposed within the vasculature of the patient, generating electrical parameter information (e.g., a measured cumulative electrical parameter, such as measured cumulative current, or measured electrolytic work performed by the power source) indicative of a successful electrolytic detachment event, and assessing if a successful electrolytic detachment event has occurred based on the generated electrical parameter information. One method further comprises contacting a ground electrode to the patient, and electrically coupling the ground electrode to the power source, such that the electrical current is delivered between the delivery wire and the ground electrode. Another method further comprises generating a first user-discernible notification if the successful electrolytic detachment event has been assessed to have occurred, and generating a second user-discernible notification different from the first user-discernible notification if the successful electrolytic detachment event has been assessed to have not occurred. In still another method, the electrical current is delivered to the electrolytically severable joint during an electrolytic detachment cycle, and delivered to the electrolytically severable joint during another electrolytic detachment cycle.

In accordance with a seventh aspect of the present inventions, the method further comprises measuring an elapsed time between a delivery wire decoupling event and a subsequent delivery wire coupling event, and resetting the electrical parameter information if the measured elapsed time reaches a first elapsed time threshold (e.g., equal to or greater than three seconds, e.g., in the range of 25-45 seconds).

In one method, the electrical parameter information is reset if the measured elapsed time reaches the first elapsed time threshold and the successful electrolytic detachment event has been assessed to have occurred, and the electrical parameter information is reset if the measured elapsed time reaches a second elapsed time threshold longer than the first elapsed time threshold and the successful electrolytic detachment event has been assessed to have not occurred. In this case, the first elapsed time threshold may be, e.g., in the range of 3-10 seconds, and the second elapsed time threshold may be, e.g., in the range of 25-45 seconds.

In one example, the method further comprises disposing another vaso-occlusive assembly within the delivery catheter, such that both the vaso-occlusive device and other vaso-occlusive device are disposed outside of the delivery catheter within the vasculature of the patient, with the other vaso-occlusive assembly comprising a delivery wire and a vaso-occlusive device detachably coupled to a distal end of the delivery wire via an electrolytically severable joint. The method further comprises electrically coupling a proximal end of the delivery wire of the other vaso-occlusive assembly to the power source after assessing if the successful electrolytic detachment event has occurred, and resetting the electrical parameter information after the proximal end of the delivery wire of the other vaso-occlusive assembly has been electrically coupled to the power source.

In accordance with an eighth aspect of the present inventions, the electrical current is delivered during an electrolytic detachment cycle having a fixed time period from the power source to the electrolytically severable joint of the vaso-occlusive assembly while the vaso-occlusive device is disposed within the vasculature of the patient, and the method comprises assessing if the successful electrolytic detachment event has occurred during the fixed time period of the electrolytic detachment cycle based on the generated electrical parameter information, and extending the electrolytic detachment cycle only if the successful electrolytic detachment event has been assessed to have not occurred during the fixed time period.

In one method, the fixed time period is an initial fixed time period, in which case, the electrolytic detachment cycle may be extended by a subsequent variable time period. The method may further comprise incrementally assessing if the successful electrolytic detachment event has occurred during the subsequent variable time period, and terminating the electrolytic detachment cycle when the successful electrolytic detachment event is assessed to have occurred. The subsequent variable time period may have a plurality time increments, each of which is shorter than the initial fixed time period of the electrolytic detachment cycle, in which case, if the successful electrolytic detachment event has occurred is incrementally assessed respectively during the plurality of time increments, and the electrolytic detachment cycle is terminated at the end of the time increment at which the successful electrolytic detachment event has been assessed to have occurred.

Another method further comprises determining if an electrolytic fault has occurred during the delivery of the electrical current to the electrolytically severable joint, in which case, the electrolytic detachment cycle is extended only if the electrolytic fault has not been determined to have occurred during the delivery of the electrical current to the electrolytically severable joint. For example, if the electrical parameter information comprises measured electrolytic work performed by the power source, the electrolytic fault may be determined to have occurred during the delivery of the electrical current to the electrolytically severable joint if the measured electrolytic work is outside of an electrolytic work range defined by a minimum electrolytic work limit and a maximum electrolytic work limit.

Still another method further comprises tracking a number of completed electrical detachment cycles, in which case, the electrolytic detachment cycle is not extended if the tracked number of completed electrolytic detachment cycles exceeds a predefined limit of electrolytic detachment cycles.

In accordance with a ninth aspect of the present inventions, the method comprises incrementally measuring electrolytic work performed by the power source over time (e.g., by incrementally measuring cumulative current delivered by the power source over time), thereby generating a plurality of measured electrolytic work values, incrementally increasing an electrolytic work threshold over time, thereby generating a plurality of electrolytic work threshold values, respectively comparing the plurality of measured electrolytic work values to the plurality of electrolytic work threshold values, and assessing if a successful electrolytic detachment event has occurred based on the comparisons.

Other and further aspects and features of embodiments will become apparent from the ensuing detailed description in view of the accompanying figures.

### BRIEF DESCRIPTION OF THE DRAWINGS

The drawings illustrate the design and utility of preferred embodiments of the disclosed inventions, in which similar elements are referred to by common reference numerals. It should be noted that the figures are not drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention, which is defined only by the appended claims and their equivalents. In addition, an illustrated embodiment of the disclosed inventions needs not have all the aspects or advantages shown. Further, an aspect or an advantage described in conjunction with a particular embodiment of the disclosed inventions is not necessarily limited to that embodiment and can be practiced in any other embodiments even if not so illustrated.

In order to better appreciate how the above-recited and other advantages and objects of the disclosed inventions are obtained, a more particular description of the disclosed inventions briefly described above will be rendered by reference to specific embodiments thereof, which are illustrated in the accompanying drawings. Understanding that these drawings depict only typical embodiments of the invention and are not therefore to be considered limiting of its scope, the invention will be described and explained with additional specificity and detail through the use of the accompanying drawings in which:
**Fig. 1** is a plan view of an electrolytic detachment procedure performed by a prior art electrolytic detachment device to electrolytically detach a vaso-occlusive device from a delivery wire;
**Fig. 2** is a plan view of another electrolytic detachment procedure performed by a prior art electrolytic detachment device to electrolytically detach a vaso-occlusive device from a delivery wire;
**Fig. 3** is a plan view of a vaso-occlusive treatment system constructed in accordance with one embodiment of the disclosed inventions, particularly showing a vaso-occlusive device in a delivery configuration;
**Fig. 4** is a plan view of the vaso-occlusive treatment system of **Fig. 3****,** particularly showing the vaso-occlusive device in a deployed configuration;
**Fig. 5** is a close-up, partially-cutaway, cross-sectional view of the distal ends of a vaso-occlusive assembly of the vaso-occlusive treatment system of **Fig. 3****;**
**Fig. 6** is a cross-sectional view of a single-lumen delivery catheter and vaso-occlusive assembly of the vaso-occlusive treatment system of **Fig. 3****;**
**Fig. 7** is a cross-sectional view of an altemative embodiment of a dual-lumen delivery catheter and two vaso-occlusive assemblies that can be used in the vaso-occlusive treatment system of **Fig. 3****;**
**Fig. 8** is a top view of a hand-held electrolytic detachment device of the vaso-occlusive treatment system of **Fig. 3****;**
**Fig. 9** **is** a block diagram of electronic componentry contained in the hand-held electrolytic detachment device of **Fig. 8****;**
**Fig. 10** is a plan view of the use of the vaso-occlusive treatment system of **Fig. 3** for delivering a vaso-occlusive device within an aneurysmal sac of a patient, prior to electrolytic detachment of the vaso-occlusive device from the delivery wire;
**Fig. 11** is a plan view of the use of the vaso-occlusive treatment system of **Fig. 3** for delivering a vaso-occlusive device within an aneurysmal sac of a patient, subsequent to electrolytic detachment of the vaso-occlusive device from the delivery wire;
**Fig. 12** is a plan view of an extended electrolytic detachment cycle performed by the hand-held electrolytic detachment device of **Fig. 8****;**
**Figs. 13A-13G** are plan views of an incrementally extended electrolytic detachment cycle performed by the hand-held electrolytic detachment device of **Fig. 8****;**
**Fig. 14** is one sequence of electrolytic detachment cycles that can be performed by the hand-held electrolytic detachment device of **Fig. 8****;**
**Fig. 15** is another sequence of electrolytic detachment cycles that can be performed by the hand-held electrolytic detachment device of **Fig. 8****;**
**Fig. 16** is a plan view of an extended electrolytic detachment cycle performed by the hand-held electrolytic detachment device of **Fig. 8****,** particularly showing the timing of a sequence of electrolytic detachment event assessments performed during extended electrolytic detachment cycle;
**Fig. 17** is a timing diagram illustrating an incrementally increased electrolytic work threshold over time used by the hand-held electrolytic detachment device of **Fig. 8** in assessing an occurrence of an electrolytic detachment event during the extended electrolytic detachment cycle;
**Fig. 18** is a plot illustrating the fitting of the incrementally increased electrolytic work threshold of **Fig. 17** to experimental electrolytic detachment points;
**Fig. 19** is a flow diagram illustrating one method of performing an electrolytic detachment procedure by the hand-held electrolytic detachment device of **Fig. 8****;** and
**Fig. 20** is a flow diagram illustrating another method of performing an electrolytic detachment procedure by the hand-held electrolytic detachment device of **Fig. 8****.**

### DETAILED DESCRIPTION OF THE ILLUSTRATED EMBODIMENTS

The present disclosure is directed to an electrolytic detachment device for use in a vaso-occlusive treatment system that delivers vaso-occlusive devices (e.g., vaso-occlusive coils) within the vasculature of a patient (e.g., within an aneurysmal sac) via an electrolytic detachment procedure.

The electrolytic detachment device described herein may minimize the number of electrolytic detachment cycles (and associated fluoroscopic visualization confirmations) needed to successfully electrolytically detach a given vaso-occlusive coil from the delivery wire, while minimizing the number of false electrolytic detachment positives while also prompting the physician to perform corrective actions when needed.

In particular, in the case where a successful electrolytic detachment event (i.e., the vaso-occlusive device has electrolytically detached from the delivery wire) has not been detected and reported at the end of an electrolytic detachment cycle, the electrolytic detachment device will automatically extend (if it is assessed that such cycle extension will facilitate electrolytic detachment of the vaso-occlusive device from the delivery wire), such electrolytic detachment cycle with the anticipation that a successful electrolytic detachment event will soon occur without having to manipulate the delivery wire to favorably change the environmental electrolytic conditions. In this manner, the extended electrolytic detachment cycle may have an initial fixed time period and a subsequent variable time period. Such electrolytic detachment cycle may be incrementally extended by several time increments, each of which is significantly smaller than the time period during which the electrolytic detachment cycle extends. In this manner, electrolytic detachment of the vaso-occlusive device from the delivery wire can be assessed during each time increment, and the extended electrolytic detachment cycle quickly terminated (i.e., the electrolytic detachment cycle will not be further extended) once a successful electrolytic detachment event has been assessed to have occurred. In some embodiments, the electrolytic detachment device may extend multiple electrolytic detachment cycles up to a certain limit, after which such extension is deemed to not be beneficial.

The length of the initial fixed time period of the extended electrolytic detachment cycle may be selected (e.g., five seconds) to minimize the chance of a false electrolytic detachment positive (i.e., by setting a minimum length of time that electrical current is delivered by the electrolytic detachment device). In essence, the initial fixed time period of extended electrolytic detachment cycle serves as, and has the same advantages of, a fixed electrolytic detachment cycle in the case where a successful electrolytic detachment event has been detected and reported, and such electrolytic detachment cycle will not be automatically extended. The length of the subsequent variable time period of the extended electrolytic detachment cycle may be selected (e.g., up to five seconds) to be long enough to effect a significant number of successful electrolytic detachment events that have not occurred during the initial fixed time period of the extended electrolytic detachment cycle, while being short enough to allow the extended electrolytic detachment cycle to terminate within a reasonable amount of time where it would be futile to continue the extended electrolytic detachment cycle due to unfavorable environmental electrolytic conditions. Thus, it can be appreciated that, in the case that a successful electrolytic detachment event, that did not occur during the initial fixed time period of the extended electrolytic detachment cycle, does occur during the subsequent variable time period of the electrolytic detachment cycle, the physician will not be prompted to take corrective action when it is not needed and will be prompted only one time to perform a fluoroscopic confirmation check upon reporting of the successful electrolytic detachment event, thereby decreasing the electrolytic detachment procedure time. By placing a maximum limit on the extended electrolytic detachment cycle, the extended electrolytic detachment cycle will eventually terminate, thereby prompting the physician to take corrective action when it is needed.

The electrolytic detachment device described herein may also automatically reset electrolytic detachment detection information (i.e., information used to assess whether or not a successful electrolytic detachment event has occurred) only when it is deemed that a successful electrolytic detachment event has, in fact, occurred. Such successful electrolytic detachment event can be deemed to have occurred based on a measured elapsed time between a delivery wire decoupling event (i.e., when a delivery wire is decoupled from the electrolytic detachment device) and a subsequent delivery wire coupling event (i.e., when a delivery wire is coupled to the electrolytic detachment device (either the current delivery wire being recoupled or a new delivery wire being coupled). In essence, the electrolytic detachment device uses such measured elapsed time as a proxy for physician actions that are indicative of whether a successful electrolytic detachment event has occurred. Such measured elapsed time can be compared to one or more elapsed time thresholds, and a successful electrolytic detachment event can be assumed to have occurred if the measured elapsed time reaches an elapsed time threshold.

In one embodiment, irrespective of whether or not a successful electrolytic detachment event has been detected and reported, the measured elapsed time between a delivery wire decoupling event and a subsequent delivery wire coupling event may be compared to a single elapsed time threshold.

In this case, the electrolytic detachment device assumes that if the measured elapsed time between the delivery wire decoupling event and the subsequent delivery wire coupled event is less than the elapsed time threshold, the physician has either fluoroscopically visually assessed that a successful electrolytic detachment event has not occurred, possibly performed additional corrective action, and has quickly recoupled the proximal end of the delivery wire to the electrolytic detachment case or the physician has quickly decoupled and then recoupled the proximal end of the delivery wire to and from the electrolytic detachment device prior to initiating a subsequent electrolytic detachment cycle for the current vaso-occlusive device (i.e., a double-press event will occur). In these cases (i.e., either the physician has performed a fluoroscopic check and/or taken corrective action or the physician will perform a double-press event), the measured elapsed time will not have reached the elapsed time threshold. As such, the electrolytic detachment device will not automatically reset the electrolytically detachment detection information, thereby minimizing the chance that a false electrolytic detachment negative will be detected and reported for the current vaso-occlusive device at the end of the next electrolytic detachment cycle (in the case of either a fluoroscopic check and/or corrective action or a double-press event) or preventing a confusing false electrolytic detection negative from being detected and reported after a true electrolytic detection positive has already been detected and reported (in the case of a double-press event).

In contrast, the electrolytic detachment device assumes that if the measured elapsed time between the delivery wire decoupling event and the subsequent delivery wire coupled event reaches the elapsed time threshold, the physician has fluoroscopically visually assessed that a successful electrolytic detachment event has occurred and has loaded a new delivery wire with a new vaso-occlusive device into the delivery catheter and then coupled the new delivery wire to the electrolytic detachment device. As such, the electrolytic detachment device will automatically reset the electrolytically detachment detection information for the new vaso-occlusive device. It follows that the single elapsed time threshold must be selected to temporally distinguish between a mere fluoroscopic visualization check and a fluoroscopic visualization check followed by a loading of a delivery wire with a new vaso-occlusive coil into delivery catheter. For example, such single elapsed time threshold may be in the range of 25-45 seconds, e.g., 30 seconds.

In another embodiment, the measured elapsed time between a delivery wire decoupling event and a subsequent delivery wire coupling event may be compared to two different elapsed time thresholds, depending on whether or not a successful electrolytic detachment event has been detected and reported.

In particular, if a successful electrolytic detachment event has been detected and reported, the electrolytic detachment device assumes that if the measured elapsed time between the delivery wire decoupling event and the subsequent delivery wire coupled event is less than a relatively short elapsed time threshold, the physician has quickly decoupled and then recoupled the proximal end of the delivery wire to and from the electrolytic detachment device prior to initiating a subsequent electrolytic detachment cycle for the current vaso-occlusive device (i.e., a double-press event will occur). As such, the electrolytic detachment device will not automatically reset the electrolytically detachment detection information to prevent a confusing false electrolytic detection negative from being detected and reported after a true electrolytic detection positive has already been detected and reported. In contrast, the electrolytic detachment device assumes that if the measured elapsed time between the delivery wire decoupling event and the subsequent delivery wire coupled event reaches the relatively short elapsed time threshold, the physician has not decoupled and recoupled the delivery wire with the current vaso-occlusive device to the electrolytic detachment device (i.e., a double-press event will not occur), and that the physician has, instead, performed a fluoroscopic visualization check (either followed or not followed by loading one or more new vaso-occlusive devices into a delivery catheter). As such, the electrolytic detachment device will automatically reset the electrolytically detachment detection information for the new vaso-occlusive device. In the embodiment described below, the relatively short elapsed time threshold is selected to temporally distinguish between quickly coupling and recoupling the same delivery wire to and from the electrolytic detachment device and performing a fluoroscopic visualization check. For example, such relatively short elapsed time threshold may be 3-10 seconds, e.g., 5 seconds.

If a successful electrolytic detachment event has not been detected and reported, the electrolytic detachment device assumes that if the measured elapsed time between the delivery wire decoupling event and the subsequent delivery wire coupled event is less than the elapsed time threshold, the physician has fluoroscopically visually confirmed that a successful electrolytic detachment event has not occurred, possibly performed additional corrective action, and has quickly recoupled the proximal end of the delivery wire to the electrolytic detachment case, in which case, the measured elapsed time will not have reached the relatively high elapsed time threshold. As such, the electrolytic detachment device will not automatically reset the electrolytically detachment detection information, thereby minimizing the chance that a false electrolytic detachment negative will be detected and reported for the current vaso-occlusive device at the end of the next electrolytic detachment cycle. In contrast, the electrolytic detachment device assumes that if the measured elapsed time between the delivery wire decoupling event and the subsequent delivery wire coupled event reaches the relatively high elapsed time threshold, the physician has fluoroscopically visually assessed that a successful electrolytic detachment event has actually occurred and has loaded a new delivery wire with a new vaso-occlusive device into the delivery catheter and then coupled the new delivery wire to the electrolytic detachment device. As such, the electrolytic detachment device will automatically reset the electrolytically detachment detection information for the new vaso-occlusive device. It follows that the relatively high elapsed time threshold must be selected to temporally distinguish between a mere fluoroscopic visualization check and a fluoroscopic visualization check followed by a loading of a delivery wire with a new vaso-occlusive coil into delivery catheter. For example, such relatively high elapsed time threshold may be in the range of 25-45 seconds, e.g., 30 seconds.

The electrolytic detachment device described herein may also minimize the chance of a false electrolytic detachment positive based on the measurement of electrolytic work performed by the electrolytic detachment device (i.e., indicated by the measured cumulative current). In particular, the electrolytic detachment device incrementally increases an electrolytic work threshold (e.g., cumulative current threshold) over time to which the measured electrolytic work is compared to assess whether a successful electrolytic detachment event has occurred. The electrolytic work threshold may be incrementally increased over the electrolytic detachment cycle or a portion thereof (e.g., at the beginning of each variable time period of an extended electrolytic detachment cycle), and the measured electrolytic work may be compared to the increased electrolytic work threshold at the end of the electrolytic detachment cycle to assess whether the electrolytic detachment event has occurred. In this manner, the incremental increases in the electrolytic work threshold will be commensurate with the increased quantity of electrolytic work required to effect successful detachment of the vaso-occlusive device from the delivery wire, thereby decreasing the detection and reporting of false electrolytic detachment positives.

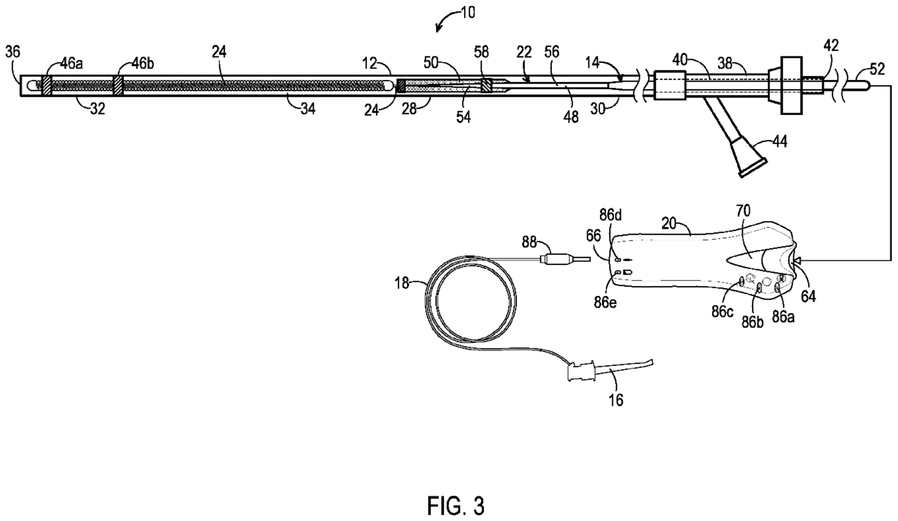

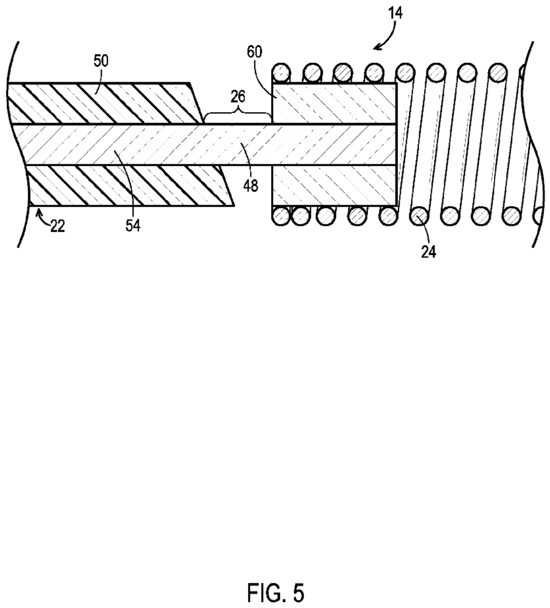

Referring to **Figs. 3-6****,** one embodiment of a vaso-occlusive treatment system 10 constructed in accordance with the disclosed inventions will now be described. The vaso-occlusive treatment system 10 comprises a delivery catheter 12, a vaso-occlusive assembly 14 slidably disposed within the delivery catheter 12, a ground electrode 16 configured for being placed in contact with a patient, an electrical cable 18 configured for being removably affixed to the ground electrode 16, and an electrical power source in the form of an electrolytic detachment device 20 to which the vaso-occlusive assembly 14 is removably affixed, and to which the ground electrode 16 is removably affixed via the electrical cable 18. As will be discussed in further detail below, the vaso-occlusive assembly 14 comprises a delivery wire 22 and a vaso-occlusive device 24 detachable coupled to the delivery wire 22 via an electrolytically severable joint 26.

The delivery catheter 12 has a tubular configuration, and can, e.g., take the form of a micro-catheter, a sheath, or the like. The delivery catheter 12 comprises an elongate sheath body 28 having a proximal portion 30 and a distal portion 32, and an inner lumen 34 (shown partially in phantom) extending through the sheath body 28 between the proximal portion 30 and the distal portion 32, and in which the vaso-occlusive assembly 14 is housed. The free end of the proximal portion 30 of the sheath body 28 remains outside of the patient and accessible to an operator (e.g., clinician or physician), while the remainder of the sheath body 28, including the distal portion 32, is sized and dimensioned to reach remote locations of the vasculature of the patient. The sheath body 28 has a suitable length for accessing a target tissue site within the patient from a vascular access point. The target tissue site depends on the medical procedure for which the delivery catheter 12 is used. For example, if the delivery catheter 12 is used to access vasculature in a brain of a patient from a femoral artery access point at the groin of the patient, the overall length of the sheath body 28 may be 125cm-200cm. The outer diameter of the sheath body 28 may be in the range of 3F-10F. In one embodiment, the outer diameter of the sheath body 28 may be uniform along the length of the sheath body 28. In another embodiment, the outer diameter of the sheath body 28 may taper in either a gradual fashion or a step-wise fashion from a first outer diameter of the proximal portion 30 to a second outer diameter at the distal portion 32 to facilitate navigation in tortuous vasculature. Although depicted as having a generally round cross-sectional shape, it can be appreciated that the sheath body 28 can include other cross-sectional shapes or combinations of shapes, e.g., oval, rectangular, triangular, polygonal, and the like.

The delivery catheter 12 may include one or more, or a plurality of regions along its length having different configurations and/or characteristics. For example, the distal portion 32 of the sheath body 28 may have an outer diameter less than the outer diameter of the proximal portion 30 of the sheath body 28 to reduce the profile of the distal portion 32 and facilitate navigation in tortuous vasculature. Furthermore, the distal portion 32 may be more flexible than the proximal portion 30. Generally, the proximal portion 30 may be formed from material that is stiffer than the distal portion 32 of the sheath body 28, so that the proximal portion 30 has sufficient pushability to advance through the patient's vascular system, while the distal portion 32 may be formed of a more flexible material so that the distal portion 32 may remain flexible and track more easily over a guidewire to access remote locations in tortuous regions of the vasculature. The sheath body 28 may be composed of suitable polymeric materials, metals and/or alloys, such as polyethylene, stainless steel or other suitable biocompatible materials or combinations thereof. In some instances, the proximal portion 30 may include a reinforcement layer, such a braided layer or coiled layer to enhance the pushability of the sheath body 28. The sheath body 28 may include a transition region between the proximal portion 30 and the distal portion 32.

The delivery catheter 12 comprises a distal port 36 in communication with the inner lumen 34 of the delivery catheter 12 and from which the vaso-occlusive device 24 is deployed. The delivery catheter 12 further comprises a proximal adapter 38 affixed to the proximal portion 30 of the sheath body 28 using suitable means, e.g., adhesive, welding, etc. The proximal adapter 38 comprises a central bore 40 (shown in phantom) in communication with the lumen 34 of the delivery catheter 12. The central bore 40 terminates in a proximal port 42 for allowing loading of the vaso-occlusive assembly 14 into the delivery catheter 12. The proximal adapter 38 further comprises a side port 44 in fluid communication with the central bore 40 for introducing fluids into the inner lumen 34 of the delivery catheter 12, e.g., to vaso-occlusive assembly 14, to introduce contrast into the vasculature of the patient, and/or to introduce saline into the vasculature of the patient, e.g., to flush out contrast prior to electrolytic detachment and delivery of the vaso-occlusive device 24 into the vasculature of the patient.

The delivery catheter 12 further comprises one or more radiopaque marker bands 46 (in this case, two distal and proximal bands 46a, 46b) disposed on the distal portion 32 of the delivery catheter 12 proximate the distal port 36, which can be identified using medical imaging technology (e.g., fluoroscopy). The distal band 46a may be used to locate the distal tip of the delivery catheter 12 within the patient's vasculature system, while the proximal band 46b may be used to locate the delivery catheter 12 relative to the partially or fully deployed vaso-occlusive device 24, such that the delivery catheter 12 and delivery wire 22 may be longitudinally aligned to ensure that the electrolytically severable joint 26 is located just distal to the distal port 36 of the delivery catheter 12 in contact with bodily fluids in the vasculature of the patient to facilitate electrolytic detachment of the vaso-occlusive device 24 from the delivery wire 22, as will be discussed in further detail below. The radiopaque marker bands 46 may be composed of a suitable radiopaque material, e.g., gold, platinum, palladium, tantalum, tungsten alloy, polymer material loaded with a radiopaque filler, and the like.

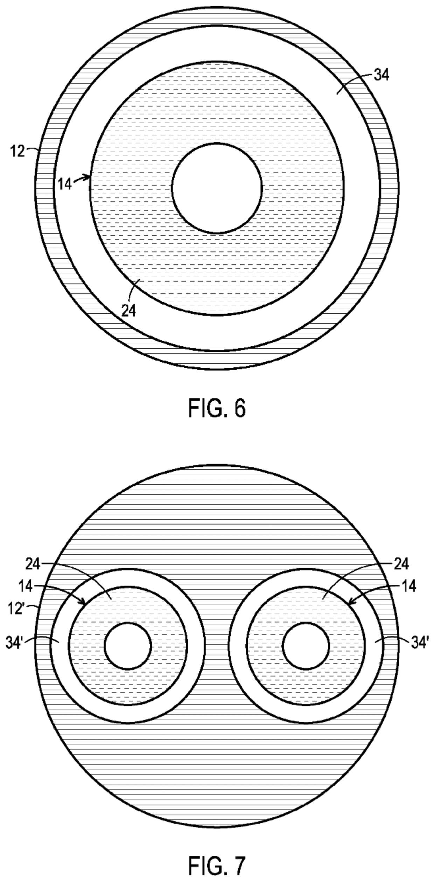

As illustrated in **Fig. 7****,** an altemative embodiment of a delivery catheter 12' is similar to the delivery catheter 12 illustrated in **Fig. 6****,** with the exception that the delivery catheter 12' comprises two inner lumens 34' in which two vaso-occlusive assemblies 14 can be respectively disposed, and two distal ports (not shown) out which the two vaso-occlusive assemblies 14 may be respectively deployed.

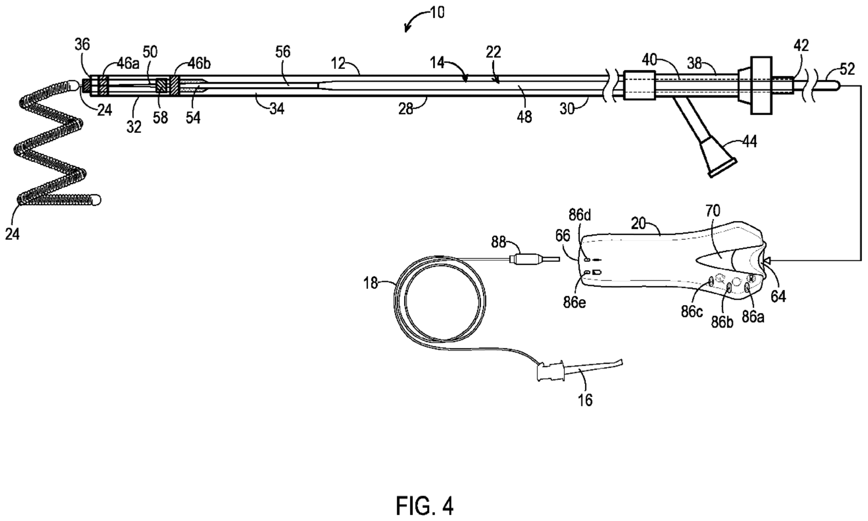

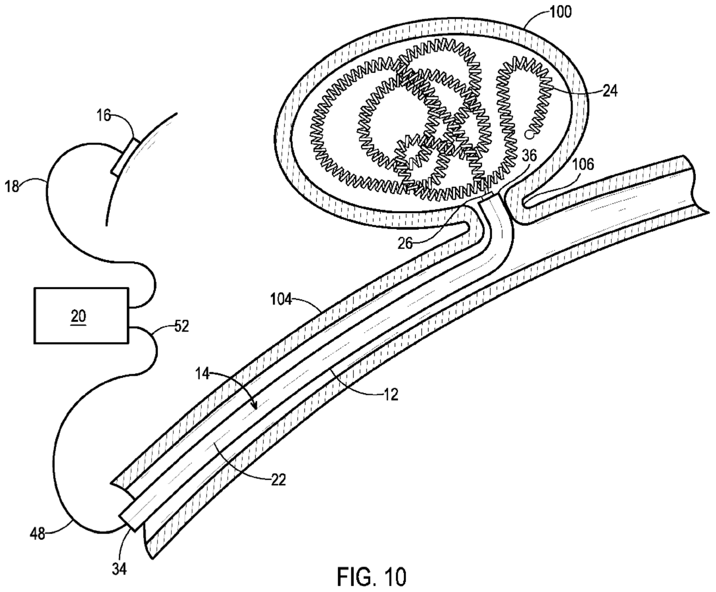

In general, the vaso-occlusive device 24 has a delivery configuration when restrained within a delivery catheter 12 **(****Fig. 3****)** (or alternatively, the delivery catheter 12') and has a deployed configuration that conforms to the interior shape of an aneurysmal sac 100 (shown in **Figs. 10-11****)** when deployed from the delivery catheter 12 **(****Fig. 4****)** (or alternatively, the delivery catheter 12') into the aneurysmal sac 100. The vaso-occlusive device 24 may be pre-biased to form a cylinder, a cone, or other desired envelope. The vaso-occlusive device 24 may be extremely soft and its overall shape easily deformed. In the illustrated embodiment, the vaso-occlusive device 24 is shown as a helical coil formed of a wire having a suitable diameter, e.g., 2-6 mils. The diameter of the vaso-occlusive device 24, when in the delivery configuration, may be, e.g., 10-30 mils. The vaso-occlusive device 24 may have any suitable length desirable and appropriate for the site to be occluded, e.g., 1-50 cm. In alternative embodiments, the vaso-occlusive device 24 may take the form of a structure other than a coil, e.g., a braid. The vaso-occlusive device 24 may optionally be covered or connected with fibrous materials tied to the outside of the coil or braid. The vaso-occlusive device 24 may be composed of a suitable biocompatible and radio-opaque material, such as platinum, gold, tungsten, iridium, or alloys thereof or other metals. In the illustrated embodiment, the vaso-occlusive device 24 has an end cap or tip that prevents punctures of the aneurysmal sac 100 when delivered therein.

The delivery wire 22 may be a coil, wire, tendon, or the like (e.g., a conventional guidewire, torqueable cable tube, or a hypotube), having a sufficient columnar strength to permit pushing of the vaso-occlusive device 24 into the aneurysmal sac 100. The delivery wire 22 may have a suitable outer diameter, e.g., 10-30 mils, and a suitable length, e.g., 50-300 cm. The material used to construct the delivery wire 22 is chosen to impart varying flexibility and stiffness characteristics to different portions of the delivery wire 22. For example, the delivery wire 22 may be formed of different materials along its length, for example materials having different moduli of elasticity, resulting in a difference in flexibility.

In the illustrated embodiment, the delivery wire 22 generally comprises a core wire 48 composed of an electrically conductive material, such as, e.g., stainless steel, and a sleeve 50 composed of an electrically insulative material, such as, e.g., polytetrafluoroethylene, polyurethane, polyethylene, polypropylene, or other suitable polymeric material. The core wire 48 has a proximal portion 52 that extends proximal from the proximal portion 30 of the delivery catheter 12 for manipulation by the physician, a distal portion 54 to which the vaso-occlusive device 24 is attached, and a medial portion 56 disposed between the proximal portion 52 and the distal portion 54. The proximal portion 52 of the core wire 48 is enlarged to ergonomically facilitate manipulation of the delivery wire 22 by the physician. The proximal portion 52 of the core wire 48 distally tapers downward to the medial portion 56. The distal portion 54 of the core wire 48 extends from the medial portion 56 and distally tapers further downward to provide flexibility to the distal end of the delivery wire 22. The delivery wire 22 may comprise a coil (not shown) affixed around the distal portion 54 of the core wire 48 to provide some columnar strength to the distal end of the delivery wire 22, while not detrimentally affecting the flexibility of the tapered distal portion 54 of the core wire 48. The sleeve 50 is disposed over the distal portion 54 of the core wire 48, and as discussed in further detail, serves to electrically isolate the portion of the distal portion 54 of the core wire 48 and that is proximal to the electrolytically severable joint 26, as well as the coil of the delivery wire 22, from the blood in the vasculature of the patient. The delivery wire 22 further comprises a radiopaque marker band 58 disposed over the sleeve 50, which can be identified using medical imaging technology (e.g., fluoroscopy). The marker band 58 may be used to locate the delivery catheter 12 relative to the partially or fully deployed vaso-occlusive device 24 (by aligning it relative to the proximal marker 46b of the delivery catheter 12), such that the delivery catheter 12 and delivery wire 22 may be longitudinally aligned to ensure that the electrolytically severable joint 26 is located just distal to the distal port 36 of the delivery catheter 12 in contact with bodily fluids in the vasculature of the patient to facilitate electrolytic detachment of the vaso-occlusive device 24 from the delivery wire 22, as will be discussed in further detail below. The radiopaque marker band 58 may be composed of a suitable radiopaque material, e.g., gold, platinum, palladium, tantalum, tungsten alloy, polymer material loaded with a radiopaque filler, and the like.

The vaso-occlusive device 24 is affixed to the distal portion 56 of the core wire 48 via an electrolytically-resistant bushing 60. The electrolytically severable joint 26 takes the form of an electrolytically degradable segment for electrolytically decoupling the vaso-occlusive device 24 from the delivery wire 22, and is located on the core wire 48 between the electrically insulative sleeve 50 and the vaso-occlusive device 24. Thus, when electrical current is supplied to the core wire 48, the electrical current flows to the electrolytically severable joint 26. However, the electrolytically severable joint 26 is not electrically insulated, and is, thus, more susceptible to electrolytic dissolution in blood than the portion of the core wire 48 covered with the electrically insulative sleeve 50 and the vaso-occlusive device 24. Thus, the electrolytically severable joint 26 will substantially or completely dissolve upon release of the vaso-occlusive device 24. Preferably, the length of the electrolytically severable joint 26 is not much greater than the diameter of the electrolytically severable joint 26. For example, the electrolytically joint 26 may be as short as 0.010 inches, and typically no longer than 0.15 inches in length.

In the illustrated embodiment, the ground electrode 16 takes the form of a metallic clip that is configured for being removably attached to a hypodermic needle (not shown) that has been percutaneously inserted into the patient, e.g., into the thigh or groin of the patient, such that the ground electrode 16 is electrically coupled to the patient, thereby completing an electrical circuit that electrically couples the vaso-occlusive assembly 14 to the ground electrode 16 through the electrically conductive patient.

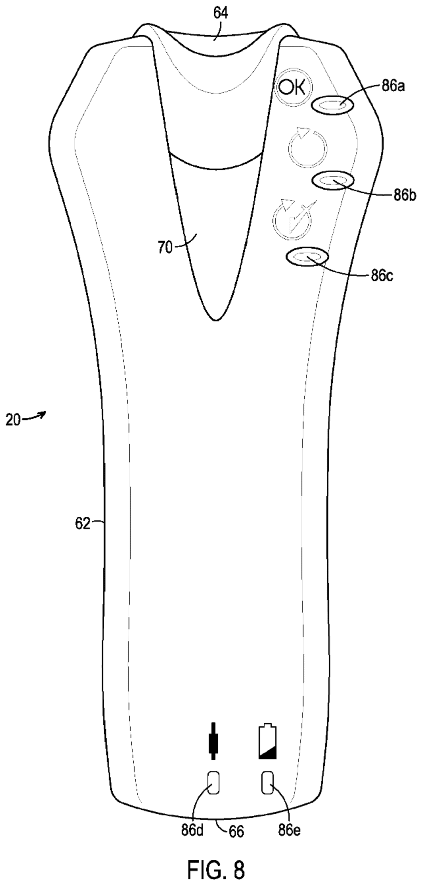

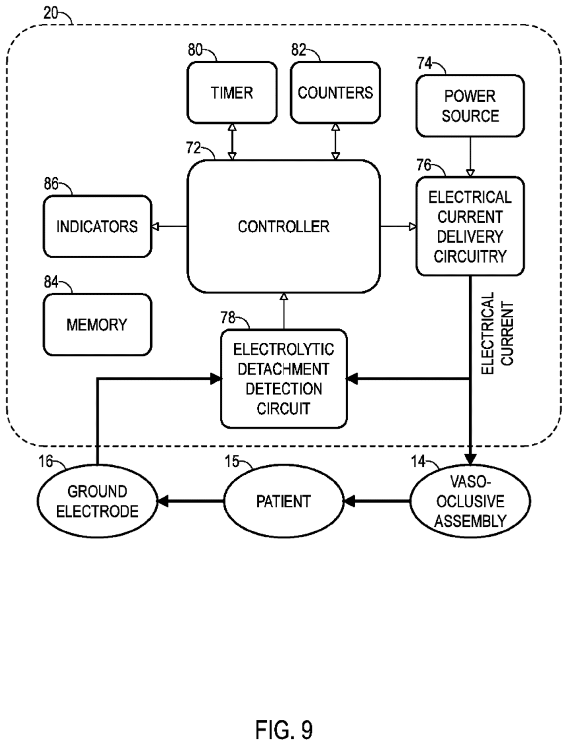

Referring to **Fig. 8****,** the electrolytic detachment device 20 may be operated by the physician to perform an electrolytic detachment procedure. The electrolytic detachment device 20 comprises an outer casing 62; a power terminal 64 to which the core wire 48 is electrically coupled; a ground terminal 66 to which the ground electrode 16 is electrically coupled via the electrical cable 18; electronic componentry 68 (shown in **Fig. 9****)** contained within the outer casing 62 for delivering electrical current to electrolytic severable joint 26 of the vaso-occlusive assembly 14 in a controlled manner; and an electrolytic detachment actuator 70 affixed to the outer casing 62 for manually initiating the flow of electrical current from the electrolytic detachment device 20 to the electrolytic severable joint 26 of the vaso-occlusive assembly 14.

The outer casing 62 is composed of a suitable material, e.g., Acrylonitrile Butadiene Styrene (ABS) or polycarbonate, is a shaped and sized to be ergonomically held by a physician with one hand. In the illustrated embodiment, the power terminal 64 takes the form of a port (e.g., a funnel) in which the proximal portion 52 of the core wire 48 may be alternately inserted and removed, while the ground terminal 66 takes the form of a port in which a corresponding plug 88 (shown in **Figs. 3-4****)** of the electrical cable 18 may be alternately inserted and removed. Insertion of the proximal portion 52 of the core wire 48 into the power port 64 (or broadly speaking, the coupling of the delivery wire 22 to the power terminal 64) constitutes a "delivery wire coupling event," whereas the removal of the proximal portion 52 of the core wire 48 from the power port 64 ((or broadly speaking, the decoupling of the delivery wire 22 from the power terminal) constitutes a "delivery wire decoupling event." As will be discussed in further detail below, the electronic componentry 68 is configured for delivering electrical current to the electrolytic severable joint 26 of the vaso-occlusive assembly 14 during one or more electrolytic detachment cycles until the vaso-occlusive device 24 electrolytically detaches from the delivery wire 22, as well as reporting to the physician of various events that occur during the electrolytic detachment procedure.

In the illustrated embodiment, the electrolytic detachment actuator 70 takes the form of a push button, which can be depressed to manually command the electrolytic detachment device 20 to perform an electrolytic detachment cycle (i.e., a time period during which electrical current is delivered from the electronic componentry 68 to the electrolytic severable joint 26 of the vaso-occlusive assembly 14). A single actuation of the push button 70 (that is, quickly depressed and then released) initiates an electrolytic detachment cycle. The push button 70 may be actuated multiple times to initiate a series of electrolytic detachment cycles. That is, the push button 70 may be actuated to initiate a first electrolytic detachment cycle, then after the first electrolytic detachment cycle terminates, the push button 70 may be actuated again to initiate a second electrolytic detachment cycle, and then after the second electrolytic detachment cycle terminates, the push button 70 may be actuated again to initiate a third electrolytic detachment cycle, and so forth.

Referring to **Fig. 9****,** the electronic componentry 68 generally comprises a controller 72, a power source 74, electrical current delivery circuitry 76, an electrolytic detachment detection circuit 78, a timer 80, counters 82, memory 84, and a plurality of indicators 86.

The controller 72 is configured for implementing various functions of the electrolytic detachment device 20, including performing electrolytic detachment cycles by operating the electrical current delivery circuitry 76 to initiate and terminate the delivery of electrical current to the vaso-occlusive assembly 14, assessing the occurrence of a successful electrolytic detachment event based on the electrical parameter information generated by the electrolytic detachment detection circuit 78 and stored in memory 84, notifying the physician of events related to the operation of the electrolytic detachment device 20, and events that occur during an electrolytic detachment cycle, such as, e.g., the occurrence or non-occurrence of a successful electrolytic detachment event. The controller 72 may also be configured for monitoring the operation of the electronic componentry 68 (e.g., monitoring the status of various nodes or other points throughout the electronic componentry 68, e.g., power supply voltages, temperature, battery voltage, and the like) and running self-diagnostic tests on the electronic componentry 68. Significantly, as will be discussed in further detail below, the controller 72 is further configured for extending an electrolytic detachment cycle under certain conditions, resetting the electrical parameter information stored in memory 84, including the measured electrolytic work (indicative by the measured cumulative current), under certain conditions, and adjusting the electrolytic work threshold (in this case, the cumulative current threshold) during an electrolytic detachment cycle or between adjacent electrolytic detachment cycles.

The functionalities of the controller 72 may be implemented using one or more suitable computing devices or digital processors, including, but not limited to, a microcontroller, microprocessor, digital signal processor, graphical processing unit, central processing unit, application specific Integrated circuit (ASIC), field programmable gate array (FPGA), and/or programmable logic unit (PLU). Such computing device(s) or digital processors may be associated with non-transitory computer- or processor- readable medium that stores executable logic or instructions and/or data or information, which when executed, perform the functions of these components. The non-transitory computer- or processor- readable medium may be formed as one or more registers, for example of a microprocessor, FPGA, or ASIC, or can be a type of computer-readable media, namely computer-readable storage media.

The power source 74 is configured for storing and supplying electrical power to the electronic componentry 68. The power source 74 provides electrical power for the electronic componentry 68 and may be, e.g., a non-rechargeable battery having an expected life for a specific number of electrolytic detachment cycles (e.g., 20 cycles), although in alternative embodiments, the power source may be, e.g., a rechargeable battery (e.g., a lithium-ion or lithium-ion polymer battery).

The electrical current delivery circuitry 76 is configured for supplying electrical current to the vaso-occlusive assembly 14. The electrical current delivery circuit 76 is electrically coupled between the power port 64 (shown in **Fig. 8****)** to which the vaso-occlusive assembly 14 is electrically coupled and the ground port 66 (shown in **Fig. 8****)** to which the ground electrode 16 is electrically coupled, and is configured for delivering electrical current at a constant amperage (e.g., 0.5 mA or 1.0 mA) to the vaso-occlusive assembly 14. To this end, the electrical current delivery circuit 76 may comprise one or more current sources, patient isolation capacitors, etc.

The electrolytic detachment detection circuit 78 is configured for measuring an electrical parameter generating electrical parameter information indicative of a successful electrolytic detachment event, and in particular, measuring electrolytic work performed by the electrical current delivery circuit 76 (in this case, cumulative current delivered by the electrical current delivery circuitry 76 to the vaso-occlusive assembly 14). The electrolytic detachment detection circuit 78 is electrically coupled between the power port 64 (shown in **Fig. 8****)** to which the vaso-occlusive assembly 14 is electrically coupled and the ground port 66 (shown in **Fig. 8****)** to which the ground electrode 16 is electrically coupled, and may take the form of any conventional circuit capable of measuring the instantaneous amperage of electrical current over time. The controller 74 may derive the value of the cumulative current delivered to the vaso-occlusive assembly 14 by the electrical current delivery circuitry 76 (i.e., the electrolytic work performed by the electrical current delivery circuitry 76) by integrating the instantaneous amperage of electrical current measured by the electrolytic detachment detection circuit 78 over time.

The timer 80 is configured measuring an elapsed time between a delivery wire coupling event and a subsequent delivery wire decoupling event, while one of the counters 82 is configured for tracking the number of electrolytic detachment cycles extended by the electrolytic detachment device 20, and another one of the counters 82 is configured for counting the number of time increments that an electrolytic detachment cycle has been extended, as will be described in further detail below. Although the timer 80 and counters 82 are illustrated as being external to the controller 72, in alternative embodiments, either or both of the timer 80 or counters 82 may be internal to the controller 76.

The memory 84 is configured for storing information data, including electrical parameter information, such as, e.g., a measured cumulative current value obtained from the electrolytic detachment detection circuit 78 and a cumulative current threshold value to which the measured cumulative current value may be compared to by the controller 72, as will be discussed in further detail below). The memory 84 may include, but is not limited to, RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information and which can be accessed by a computing device. It should be appreciated that those skilled in the art are familiar with the term "controller," and that it may be implemented in software, firmware, hardware, or any suitable combination thereof.

The indicators 86 are configured for reporting different events to the physician during the electrolytic detachment procedure. In the illustrated embodiment, the indicators 86 are disposed on the outer case 62 for notifying the physician of different events during the electrolytic detachment procedure, as illustrated in **Fig. 8****.** In the illustrated embodiment, the electrolytic detachment device 20 automatically powers on in response to the insertion of the proximal portion 52 of the core wire 48 into the power terminal 64 of the electrolytic detachment device 20. In the illustrated embodiment, the indicators 72 encompass a system ready indicator 86a, a current flow indicator 86b, a cycle complete indicator 86c, a grounding indicator 86d, and low-battery indicator 86e. At least some of the indicators 72 provide both visual and aural notifications to the physician.

The system ready indicator 86a shows a solid green light and provides a single beep when the electrolytic detachment device 20 has been powered on and the patient 15 successfully grounded with the ground electrode 16 and electrical cable 18 to report to the physician that the electrolytic detachment device 20 is ready to be operated to electrolytically detach the vaso-occlusive device 24 from the delivery wire 22. The current flow indicator 86b shows solid green light to report to the physician that electrical current is flowing along the delivery wire 22. The cycle complete indicator 86c shows a solid green light and provides three short beeps to report to the physician that the electrolytic detachment device 20 has assessed that a successful electrolytic detachment event has occurred, and shows a blinking green light and provides a single long beep to report to the physician that the electrolytic detachment device 20 has assessed that a successful electrolytic detachment event has not occurred. The ground indicator 86d shows a blinking amber light until the patient 15 is effectively grounding with the ground electrode 16 and electrical cable 18, after which the ground indicator 86d shows a solid amber light. Once the push button 70 is depressed, the ground indicator 86d will tum off. The low battery indicator 86e shows a blinking amber light to notify the physician that the battery in the electrolytic detachment device 20 is running low. Of course, electrolytic detachment device 20 may comprise any combination of indicators other than the combination of indicators 86 illustrated in **Fig. 8****.**

Referring now to **Figs. 10-11****,** one procedure for delivering a vaso-occlusive device 24 into the aneurysmal sac 100 for treating a cerebral aneurysm 102 will now be described. The delivery catheter 12 (or alternatively the delivery catheter 12' illustrated in **Fig. 7****)** may be introduced into a patient using a transfemoral approach, and in particular, by inserting the delivery catheter 12 into the femoral artery near the groin of the patient. Under fluoroscopy, a physician may then guide the delivery catheter 12 through the vasculature of the patient until the distal port 36 of the delivery catheter 12 is located at a target site in a blood vessel 104 just proximal to the aneurysmal sac 100 (and in particular, near a neck 106 of the cerebral aneurysm 102), as illustrated in **Fig. 10****.**

The physician may then introduce the vaso-occlusive assembly 14 through the inner lumen 34 of the single-lumen delivery catheter 12 until the vaso-occlusive device 24 is disposed within the aneurysmal sac 100, while longitudinally aligning the delivery catheter 12 and the delivery wire 22, such that the electrolytically severable joint 26 is located just distal to the distal port 36 of the single-lumen delivery catheter 12 in contact with the blood in the blood vessel 104 (or alternatively, two vaso-occlusive assemblies 14 respectively through the inner lumens 34' of the dual-lumen delivery catheter 12' until both vaso-occlusive devices 24 are disposed within the aneurysmal sac 100, while longitudinally aligning the dual-lumen delivery catheter 12' and the delivery wires, such that the electrolytically severable joints of both vaso-occlusive assemblies 14 are located just distal to the distal ports of the dual-lumen delivery catheter 12' in contact with the blood in the blood vessel 104). Under fluoroscopic visualization, such longitudinal alignment between the delivery catheter 12 (or delivery catheter 12') and the delivery wire 22 (or delivery wires of the vaso-occlusive assemblies 14) may be ensured by positioning the marker band 58 located on the vaso-occlusive assembly 14 (or marker bands located on both vaso-occlusive assemblies 14) to the proximal marker 46b located on the delivery catheter 12, as illustrated in **Fig. 4****.**

The ground electrode 16 is attached to the patient (and in this case, a hypodermic needle (not shown) is inserted into the patient, and the ground electrode 16 is clipped to the hypodermic needle). The proximal portion 52 of the core wire 48 (of the only delivery wire 22 in the case where only one vaso-occlusive assembly 14 is introduced through the inner lumen 34 of the single-lumen delivery catheter 12 or one of the two delivery wires 22 in the case where two vaso-occlusive assemblies 14 are introduced through two inner lumens 34 of the dual-lumen delivery catheter 12') is then coupled to the power terminal 64 of the electrolytic detachment device 20 (and in particular, inserted into the power port 64), while the plug 88 of the electrical cable 18 is coupled to the ground terminal 66 (and in particular, inserted into the ground port 66), as illustrated in **Figs. 3-5****.** As a result, the electrolytic detachment device 20 powers on and the system ready indicator 86a shows a solid green light and provides a single beep, while the ground indicator 86d transitions from a blinking amber light to a solid amber light. The physician may then operate the electrolytic detachment device 20 by actuating the electrolytic detachment actuator, and in particular, by depressing the push button 70 once, thereby delivering electrical current to the electrolytically severable joint 24 during an electrolytic detachment cycle. The current flow indicator 86b shows solid green light, while all the other indicators 86 turn off. At the end of the electrolytic detachment cycle, the cycle complete indicator 86c shows a solid green light.

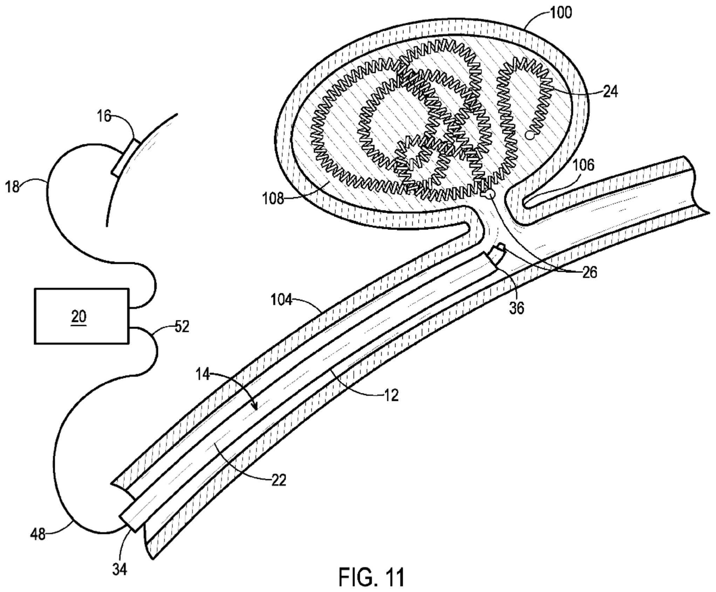

Optimally, the vaso-occlusive device 24 electrolytically detaches from the delivery wire 22 by the end of the electrolytic detachment cycle, as illustrated in **Fig. 11****.** If the electrolytic detachment device 20 detects that a successful electrolytic detachment event has occurred, the cycle complete indicator 86c will provide three short beeps, after which the physician may decouple the proximal portion 52 of the core wire 48 from the power terminal 64 of the electrolytic detachment device 20 (and in particular, removed from the power port 64), and perform a fluoroscopic visualization check (including slightly retracting the delivery wire 22 within the single-lumen delivery catheter 12 (or alternatively, the dual-lumen delivery catheter 12') by pulling the proximal portion 52 of the core wire 48) to ensure that the vaso-occlusive device 24 has, in fact, electrolytically detached from the delivery wire 22.

If the electrolytic detachment device 20 does not detect that a successful electrolytic detachment event has occurred, the cycle complete indicator 86c will provide one long beep, after which the after which the physician may decouple the proximal portion 52 of the core wire 48 from the power terminal 64 of the electrolytic detachment device 20 (and in particular, removed from the power port 64), and perform corrective measures, e.g., physically manipulating the vaso-occlusive device 24 (e.g., by slightly pulling the proximal portion 52 of the core 48), ensuring that the delivery wire 22 is properly aligned with the single-lumen delivery catheter 12 (or alternatively, the dual-lumen delivery catheter 12'), such that the electrolytically severable joint is fully exposed to blood, operating a flushing system to clear any contrast agent surrounding the vaso-occlusive device 24, checking the ground electrode 16 to ensure that it is in stable contact with the patient, etc.

Assuming that the vaso-occlusive device 24 has not, in fact, electrolytically detached from the delivery wire 22 (either after a fluoroscopic visualization check has not confirmed that a successful electrolytic detachment event has occurred despite detection of a successful electrolytic detachment event by the electrolytic detachment device 20 or after the electrolytic detachment device 20 has not detected a successful electrolytic detachment event), the physician may then operate the electrolytic detachment device 20 again by actuating the electrolytic detachment actuator, and in particular, by depressing the push button 70 once again, thereby delivering electrical current to the electrolytically severable joint 26 during another electrolytic detachment cycle.

After the vaso-occlusive device 24 has been confirmed to have been electrolytically detached from the delivery wire 22, the afore-described electrolytic detachment procedure may be repeated for the next vaso-occlusive device 24.

For example, if the single-lumen delivery catheter 12 illustrated in **Fig. 6** is used, a new vaso-occlusive assembly 14 may be introduced through the inner lumen 34 of the delivery catheter 12 until a new vaso-occlusive device 24 is disposed within the aneurysmal sac 100, while longitudinally aligning the delivery catheter 12 and the delivery wire 22, such that the electrolytically severable joint 26 is located just distal to the distal port 36 of the delivery catheter 12 in contact with the blood in the blood vessel 104; the proximal portion 52 of the core wire 48 of the next vaso-occlusive device 24 then coupled to the power terminal 64 of the electrolytic detachment device 20 (and in particular, inserted into the power port 64); and the afore-described electrolytic detachment procedure repeated to deliver the new vaso-occlusive device 24 into the aneurysmal sac 100.

As another example, if the dual-lumen delivery catheter 12' illustrated in **Fig. 7** is used, and if the vaso-occlusive device 24 of only one of the vaso-occlusive assemblies 14 disposed in the respective inner lumen of the delivery catheter 12' has been electrolytically detached from its delivery wire; the proximal portion 52 of the core wire 48 of the other one of the vaso-occlusive assemblies 14 disposed in the respective inner lumen 34' of the delivery catheter 12' is coupled to the power terminal 64 of the electrolytic detachment device 20 (and in particular, inserted into the power port 64); and the afore-described electrolytic detachment procedure repeated to deliver the new vaso-occlusive device 24 into the aneurysmal sac 100.

As still another example, if the dual-lumen delivery catheter 12' illustrated in **Fig. 7** is used, and if the vaso-occlusive devices 24 of both of the vaso-occlusive assemblies 14 disposed in the respective inner lumens of the delivery catheter 12' have been electrolytically detached from their delivery wires, two new vaso-occlusive assemblies 14 may be introduced respectively through the inner lumens 34' of the delivery catheter 12' until two new vaso-occlusive devices 24 are disposed within the aneurysmal sac 100, while longitudinally aligning the delivery catheter 12' and the delivery wires, such that the electrolytically severable joints 26 are located just distal to the distal ports of the delivery catheter 12' in contact with the blood in the blood vessel 104; the proximal portion 52 of the core wire 48 of one of the two vaso-occlusive devices 24 is then coupled to the power terminal 64 of the electrolytic detachment device 20 (and in particular, inserted into the power port 64), and the afore-described electrolytic detachment procedure repeated to deliver the new vaso-occlusive device 24 into the aneurysmal sac 100.

After all vaso-occlusive devices 24 have been delivered into the aneurysmal sac 100 and electrolytically detached from their delivery wires 22, the aneurysmal sac 100 will be sealed from the blood vessel 104. As a result, the blood and vaso-occlusive devices 24 within the aneurysmal sac 100 will congeal into a solid mass 108, as illustrated in **Fig. 11****,** thereby preventing the aneurysmal sac 100 from bursting in response to the blood pressure within the blood vessel 104.

Significantly, as briefly discussed above, the electrolytic detachment device 20 automatically extends the electrolytic detachment cycle if a successful electrolytic event has not been detected and reported to the physician at the end of the electrolytic detachment cycle. Such extension of an electrolytic detachment cycle can be performed in an electrolytic detachment procedure for, e.g., a vaso-occlusive assembly 14 that has been loaded into the single-lumen delivery catheter 12 of **Fig. 6** or either of the vaso-occlusive assemblies 14' loaded into the dual-lumen delivery catheter 12' of **Fig. 7****.**

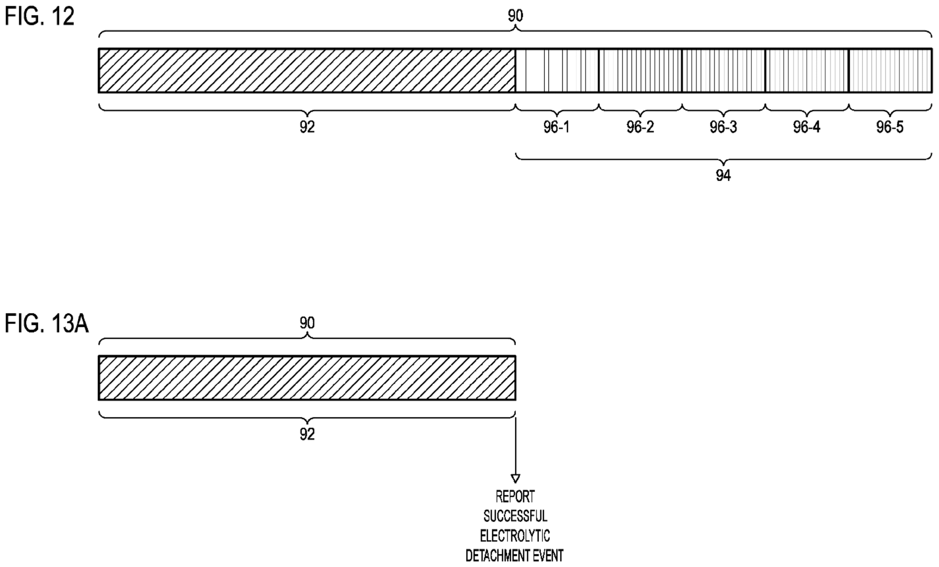

In one embodiment illustrated in **Fig. 12****,** an electrolytic detachment cycle 90 has an initial fixed time period 92 and a subsequent variable time period 94. The length of the initial fixed time period 92 may be selected to minimize the chance of a false electrolytic detachment positive (i.e., by setting a minimum length of time that electrical current is delivered by the electrolytic detachment device 20). In essence, the initial fixed time period 92 serves as, and has the same advantages of, a fixed electrolytic detachment cycle in the case where a successful electrolytic detachment event has been detected and reported, and such electrolytic detachment cycle will not be automatically extended. The length of the subsequent variable time period 94 may be selected to be long enough to effect a significant number of successful electrolytic detachment events that have not occurred during the initial fixed time period 92, while being short enough to allow the extended electrolytic detachment cycle 90 to terminate within a reasonable amount of time where it would be futile to continue the extended electrolytic detachment cycle 90 due to unfavorable environmental electrolytic conditions. In one embodiment, the length of the initial fixed time period 92 may be, e.g., five seconds, while the subsequent variable time period 94 may be, e.g., five seconds, such that the length of the electrolytic detachment cycle 90 may vary from five seconds to ten seconds. Thus, it can be appreciated that, in the case that a successful electrolytic detachment event, that did not occur during the initial fixed time period 92, does occur during the subsequent variable time period 94, the physician will not be prompted to take corrective action when it is not needed and will be prompted only one time to perform a fluoroscopic confirmation check upon reporting of the successful electrolytic detachment event, thereby decreasing the electrolytic detachment procedure time. By placing a maximum limit on the extended electrolytic detachment cycle 90, the extended electrolytic detachment cycle 90 will eventually terminate, thereby prompting the physician to take corrective action when It is needed.

The controller 72 is configured for assessing if a successful electrolytic detachment event has occurred during the initial fixed time period 92 (and in this case, at the end of the initial fixed time period 92) based on the electrical parameter information generated detachment detection circuit 78, and in this case, by comparing the electrolytic work *Wₘₑₐₛ* performed by the electrical current delivery circuitry 76 (using the measured cumulative current delivered by the electrical current delivery circuity 76 as a proxy) to an electrolytic work threshold *Wₜₕ.* The controller 72 is further configured for automatically extending the electrolytic detachment cycle 90 by the subsequent variable time period 94 only if the successful electrolytic detachment event has been assessed to have not occurred during the initial fixed time period 92 (e.g., if the measured electrolytic work *Wₘₑₐₛ* did not reach the electrolytic work threshold *Wₜₕ*).

The controller 72 is configured for incrementally assessing if the successful electrolytic detachment event has occurred during the subsequent variable time period 94, and terminating the electrolytic detachment cycle 90 when the successful electrolytic detachment event has been assessed to have occurred. In the illustrated embodiment, the subsequent variable time period 94 has a plurality of fixed time increments 96, each of which is shorter than the initial fixed time period 92, in which case, the controller 72 is configured for incrementally assessing if a successful electrolytic detachment event has occurred respectively during the fixed time increments 96 (and reporting to the physician whether or not the successful electrolytic detachment event has occurred via the cycle complete indicator 86c), and terminating the electrolytic detachment cycle 90 at the end of the fixed time increments 96 at which the successful electrolytic detachment event has been assessed to have occurred. In this manner, the controller 72 may assess if a successful electrolytic detachment event has occurred during each fixed time increment 96, and the extended electrolytic detachment cycle 90 quickly terminated (i.e., the electrolytic detachment cycle 90 will not be further extended) once a successful electrolytic detachment event has been assessed to have occurred. In the illustrated embodiment, the lengths of the fixed time increments 96 are the same, although in alternative embodiments, the lengths of the fixed time increments 96 may differ from each other.

In one example illustrated in **Fig. 13A****,** the controller 72 has assessed that a successful electrolytic detachment event has occurred during the initial fixed time period 92. Thus, the controller 72 does not automatically extend the electrolytic detachment cycle 90 by the subsequent variable time period 94, immediately terminates the electrolytic detachment cycle 90 at the end of the initial fixed time period 92, and reports to the physician that a successful electrolytic detachment event has occurred. As a result, the completed electrolytic detachment cycle 90 will only have the initial fixed time period 92.

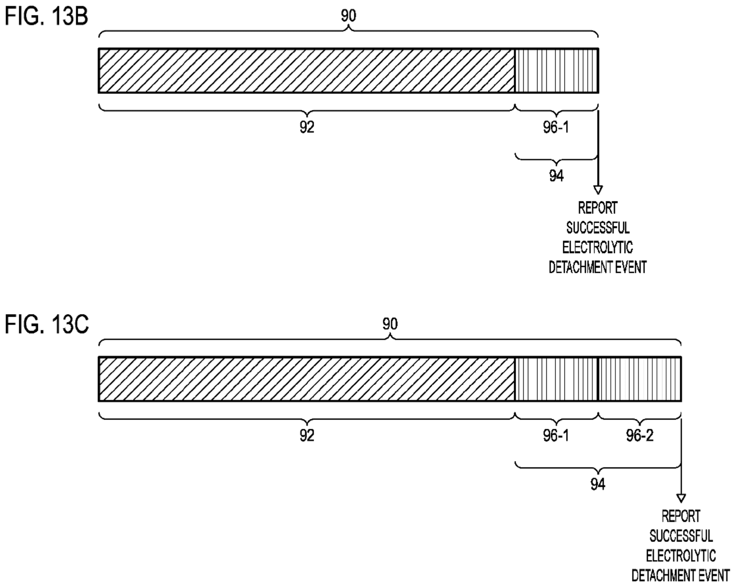

In contrast, as illustrated in **Fig. 13B****,** the controller 72 has assessed that a successful electrolytic detachment event has not occurred during the initial fixed time period 92 of the electrolytic detachment cycle 90. Thus, the controller 72 does not report whether or not the successful electrolytic detachment event has occurred, and instead automatically extends the electrolytic detachment cycle 90 by the subsequent variable time period 94. As further illustrated in **Fig. 13B****,** the controller 72 has assessed that a successful electrolytic detachment event has not occurred during the initial fixed time period 92, but has assessed that a successful electrolytic detachment event has occurred during the first fixed time increment 96-1 of the subsequent variable time period 94. Thus, the controller 72 immediately terminates the electrolytic detachment cycle 90 at the end of the first fixed time increment 96, and reports to the physician that a successful electrolytic detachment event has occurred. As a result, the completed electrolytic detachment cycle 90 will have the initial fixed time period 92 and only the first fixed time increment 96 of the subsequent variable time period 94.

As illustrated in **Fig. 13C****,** the controller 72 has assessed that a successful electrolytic detachment event has not occurred during the initial fixed time period 92 and the first fixed time increment 96-1 of the subsequent variable time period 94, but has assessed that a successful electrolytic detachment event has occurred during the second fixed time increment 96-2 of the subsequent variable time period 94. Thus, the controller 72 immediately terminates the electrolytic detachment cycle 90 at the end of the second fixed time increment 96, and reports to the physician that a successful electrolytic detachment event has occurred. As a result, the completed electrolytic detachment cycle 90 will have the initial fixed time period 92 and the first and second fixed time increments 96-1 and 96-2 of the subsequent variable time period 94.

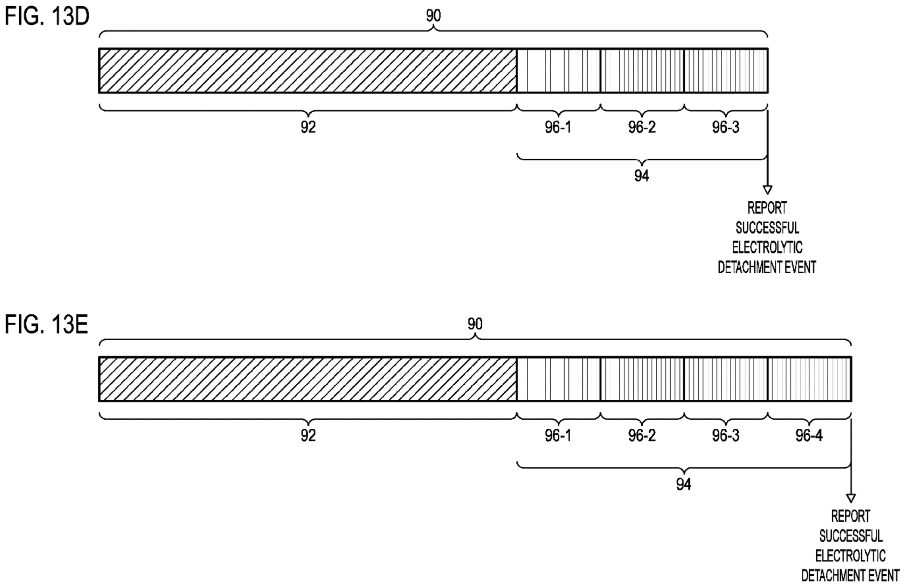

As illustrated in **Fig. 13D****,** the controller 72 has assessed that a successful electrolytic detachment event has not occurred during the initial fixed time period 92 and the first and second fixed time increments 96-1 and 96-2 of the subsequent variable time period 94, but has assessed that a successful electrolytic detachment event has occurred during the third fixed time increment 96-3 of the subsequent variable time period 94. Thus, the controller 72 immediately terminates the electrolytic detachment cycle 90 at the end of the third fixed time increment 96-3, and reports to the physician that a successful electrolytic detachment event has occurred. As a result, the completed electrolytic detachment cycle 90 will have the initial fixed time period 92 and the first through third fixed time increments 96-1 to 96-3 of the subsequent variable time period 94.

As illustrated in **Fig. 13E****,** the controller 72 has assessed that a successful electrolytic detachment event has not occurred during the initial fixed time period 92 and the first through third fixed time increments 96-1 to 96-3 of the subsequent variable time period 94, but has assessed that a successful electrolytic detachment event has occurred during the fourth fixed time increment 96-4 of the subsequent variable time period 94. Thus, the controller 72 immediately terminates the electrolytic detachment cycle 90 at the end of the fourth fixed time increment 96-4, and reports to the physician that a successful electrolytic detachment event has occurred. As a result, the completed electrolytic detachment cycle 90 will have the initial fixed time period 92 and the first through fourth fixed time increments 96-1 to 96-4 of the subsequent variable time period 94.

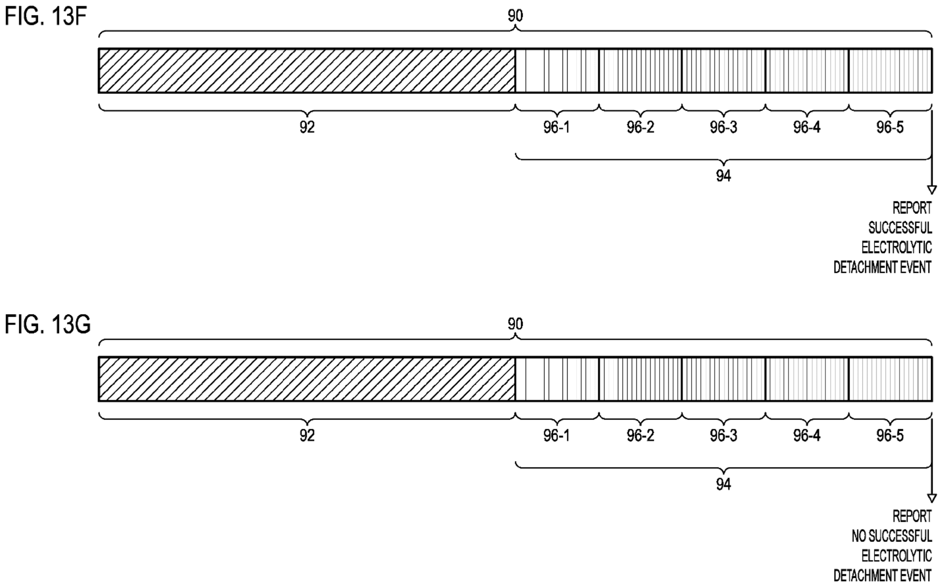

As illustrated in **Fig. 13F****,** the controller 72 has assessed that a successful electrolytic detachment event has not occurred during the initial fixed time period 92 and the first through fourth fixed time increments 96-1 to 96-4 of the subsequent variable time period 94, but has assessed that a successful electrolytic detachment event has occurred during the fifth fixed time increment 96-5 of the subsequent variable time period 94. Thus, the controller 72 immediately terminates the electrolytic detachment cycle 90 at the end of the fifth fixed time increment 96-5, and reports to the physician that a successful electrolytic detachment event has occurred. As a result, the completed electrolytic detachment cycle 90 will have the initial fixed time period 92 and all first through fifth fixed time increments 96-1 to 96-5 of the subsequent variable time period 94.

The controller 72 is configured for only extending an electrolytic detachment cycle 90 up to a pre-defined limit of fixed time increments 96 (in this case, five fixed time increments 96-1 to 96-5). In particular, as briefly discussed above, one of the counters 82 is configured for tracking a number of fixed time increments *N_{TI}* by which the current electrolytic detachment cycle 90 has been extended. Regardless of the occurrence of a successful electrolytic detachment event, the controller 72 is configured for terminating the electrolytic detachment cycle 90 at the end of the current fixed time increment 96 (in this case, the fifth fixed time increment 96-5) once the tracked number of fixed time increments 96 by which the current electrolytic detachment cycle 90 has been extended equals a predefined limit of fixed time increments *N_{TI-lim}* (i.e., the electrolytic detachment cycle 90 will not be extended by another fixed time increment 96 if doing so would cause the tracked number of fixed time increments *N_{TI}* by which the current electrolytic cycle 90 will be extended to exceed the predefined limit of fixed time increments *N*_{*TI*/}*ₗᵢₘ.*

For example, as illustrated in **Fig. 13G****,** the controller 72 has assessed that a successful electrolytic detachment event has not occurred by the end of the fifth fixed time increment 96-5. Thus, the controller 72 immediately terminates the electrolytic detachment cycle 90 at the end of the fifth fixed time increment 96-5, and reports to the physician that a successful electrolytic detachment event has not occurred, since the tracked number of fixed time increments *N_{TI}* by which the current electrolytic detachment cycle 90 has already been extended equals the predefined limit of electrolytic detachment cycles *N_{cycle-lim}=5.*

The controller 72 is configured for only automatically extending a particular electrolytic detachment cycle 90 if it is assessed that such electrolytic detachment cycle extension will significantly facilitate electrolytic detachment of the vaso-occlusive device 24 from the delivery wire 22, e.g., with the anticipation that a successful electrolytic detachment event will soon occur without having to take corrective action.

For example, the controller 72 may take into account the existence of an electrolytic fault in making the assessment of whether automatically extending another electrolytic detachment cycle 90 will significantly facilitate electrolytic detachment of the vaso-occlusive device 24 from the delivery wire 22. In one embodiment, the controller 72 is configured for determining if an electrolytic fault has occurred during the delivery of the electrical current to the electrolytically severable joint 26, and automatically extending the electrolytic detachment cycle 90 only if the electrolytic fault has not been determined to have occurred during the delivery of the electrical current to the electrolytically severable joint 26.

One electrolytic fault may occur when the measured electrolytic work *Wₘₑₐₛ* at the end of the initial fixed time period 92 of the electrolytic detachment cycle 90 is minimal, indicating that not enough electrolytic work is being performed to electrolytically detach the vaso-occlusive device 24 from the deliver wire 22, e.g., if there is an open circuit between the core wire 48 and the ground electrode 16. The controller 72 may compare the measured electrolytic work *Wₘₑₐₛ* to a minimum electrolytic work limit *Wₗᵢₘ₋ₘᵢₙ* at the end of the initial fixed time period 92, and determine that an electrolytic fault has occurred if the measured electrolytic work *Wₘₑₛₛ* is less than the minimum electrolytic work limit *Wₗᵢₘ₋ₘᵢₙ.*

Another electrolytic fault may occur when the cumulative current measured at the end of the initial fixed time period 92 of the electrolytic detachment cycle 90 is excessive, indicating that too much electrical current is being used to perform non-electrolytic work, e.g., if the electrolytically severable joint 26 is in physical contact with a vaso-occlusive device mass within the aneurysmal sac 100. The controller 72 may compare the measured electrolytic work *Wₘₑₐₛ* to a maximum electrolytic work limit *Wₗᵢₘ₋ₘₐₓ* at the end of the initial fixed time period 92, and determine that an electrolytic fault has occurred if the measured electrolytic work *Wₘₑₐₛ* exceeds the maximum electrolytic work limit *Wₗᵢₘ₋ₘₐₓ.*

Thus, despite whether or not the measured electrolytic work *Wₘₑₐₛ* reaches the electrolytic work threshold *Wₜₕ,* the controller 72 may assess that a successful electrolytic detachment event has not occurred in response to an assessed electrolytic fault (in this case, the measured electrolytic work *Wₘₑₐₛ* is outside the range defined by the minimum electrolytic work limit *Wₗᵢₘ₋ₘᵢₙ* and the maximum electrolytic work limit *Wₗᵢₘ₋ₘₐₓ*)*.*

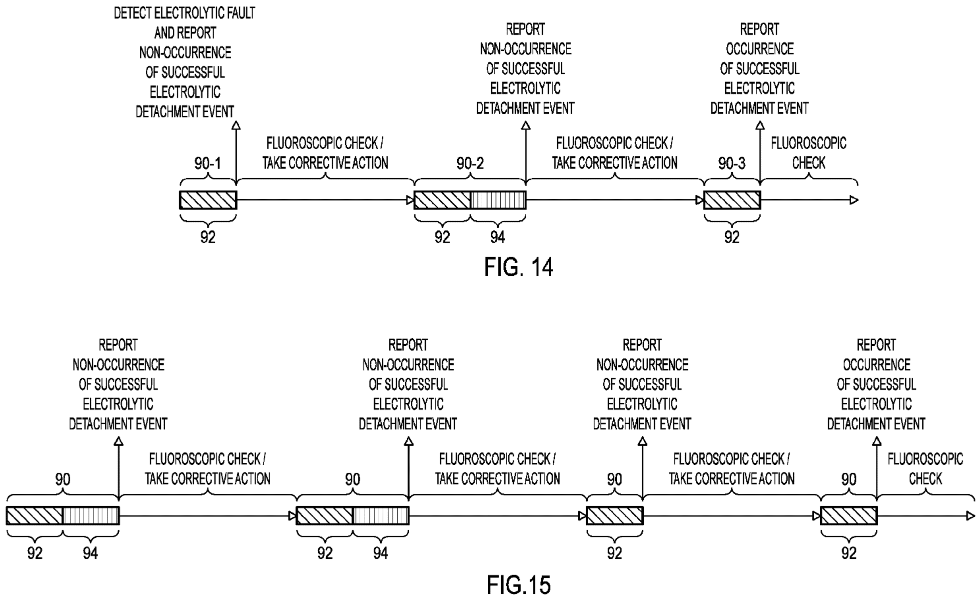

As illustrated in **Fig. 14****,** a series of electrolytic detachment cycles 90 may be performed by the controller 72 in response to single actuations of the push button 70. The controller 72 detects an electrolytic fault for the first electrolytic detachment cycle 90-1, and thus, does not automatically extend the first electrolytic cycle 90-1, but rather reports that a successful electrolytic detachment event has not occurred, thereby prompting the physician to perform a fluoroscopic visualization check and take corrective action, and then initiate a second electrolytic detachment cycle 90-2. Then, the controller 72 does not detect an electrolytic fault for the second electrolytic detachment cycle 90-2, but does assess that a successful electrolytic detachment event has not occurred at the end of the initial fixed time period 92 of the second electrolytic detachment cycle 90-2, and thus, automatically extends the second electrolytic detachment cycle 90-2 by a subsequent variable time period 94. At the end of the second electrolytic detachment cycle 90-2, the controller 72 again assesses and reports that a successful electrolytic event has not occurred, prompting the physician to again perform a fluoroscopic visualization check and take corrective action, and then initiate a third electrolytic detachment cycle 90-3. Then, the controller 72 assesses that a successful electrolytic detachment event has occurred at the end of the initial fixed time period 92 of the third electrolytic detachment cycle 90-3, and thus, does not automatically extend the third electrolytic cycle 90-3, but rather reports that a successful electrolytic detachment event has occurred, thereby prompting the physician to perform a fluoroscopic visualization check to confirm such reported successful electrolytic detachment event.

As another example, the controller 72 may take into account the number of electrolytic detachment cycles that have already been performed in making the assessment of whether automatically extending another electrolytic detachment cycle 90 will significantly facilitate electrolytic detachment of the vaso-occlusive device 24 from the delivery wire 22. In one embodiment, the controller 72 is configured for limiting the number of electrolytic detachment cycles 90 that can be completed. In particular, as briefly discussed above, another one of the counters 82 is configured for tracking a number of electrolytic detachment cycles *N_{cycle}* that have been completed. Regardless of the presence of an electrolytic fault, the controller 72 is configured for not automatically extending an electrolytic detachment cycle 90 if the tracked number of completed electrolytic detachment cycles *N_{cycle}* exceeds a predefined limit of completed electrolytic detachment cycles *N_{cycle-lim}.*

As illustrated in **Fig. 15****,** a series of electrolytic detachment cycles 90 may be performed by the controller 72 in response to single actuations of the push button 70. In this example, the predefined limit of completed electrolytic detachment cycles *N_{cycle-lim}=2,* and thus, the controller 72 automatically extends the first and second electrolytic detachment cycles 90-1 and 90-2, but will not automatically extend the third and fourth electrolytic detachment cycles 90-3 and 90-4.

In particular, the controller 72 assesses that a successful electrolytic detachment event has not occurred at the end of the initial fixed time period 92 of the first electrolytic detachment cycle 90-1, and thus, automatically extends the first electrolytic detachment cycle 90-1 by a subsequent variable time period 94, since the tracked number of completed electrolytic detachment cycles *N_{cycle}* does not exceed the predefined limit of completed electrolytic detachment cycles *N_{cycle-lim}=2*. At the end of the first electrolytic detachment cycle 90-1, the controller 72 assesses and reports that a successful electrolytic event has not occurred, prompting the physician to perform a fluoroscopic visualization check and take corrective action, and then initiate a second electrolytic detachment cycle 90-2. The controller 72 then again assesses that a successful electrolytic detachment event has not occurred at the end of the initial fixed time period 92 of the second electrolytic detachment cycle 90-2, and thus, automatically extends the second electrolytic detachment cycle 90-2 by a subsequent variable time period 94, since the tracked number of completed electrolytic detachment cycles *N_{cycle}* still does not exceed the predefined limit of completed electrolytic detachment cycles *N_{cycle-llm}=2.* At the end of the second electrolytic detachment cycle 90-2, the controller 72 assesses and reports that a successful electrolytic event has not occurred, prompting the physician to again perform a fluoroscopic visualization check and take corrective action, and then initiate a third electrolytic detachment cycle 90-3. The controller 72 then assesses that a successful electrolytic detachment event has not occurred at the end of the initial fixed time period 92 of the third electrolytic detachment cycle 90-3. However, because the tracked number of completed electrolytic detachment cycles *N_{cycle}* does exceed the predefined limit of completed electrolytic detachment cycles *N_{cycle-lim}=2,* the controller 72 does not automatically extend the third electrolytic cycle 90-3, but rather reports that a successful electrolytic detachment event has not occurred, thereby prompting the physician to perform a fluoroscopic visualization check and take corrective action, and then initiate a fourth electrolytic detachment cycle 90-4. Then, the controller 72 assesses that a successful electrolytic detachment event has occurred at the end of the initial fixed time period 92 of the fourth electrolytic detachment cycle 90-4, and thus, does not automatically extend the fourth electrolytic cycle 90-4, but rather reports that a successful electrolytic detachment event has occurred, thereby prompting the physician to perform a fluoroscopic visualization check to confirm such reported successful electrolytic detachment event.

Significantly, as briefly discussed above, the electrolytic detachment device 20 automatically resets the electrolytic detachment detection information (in this case, the measured electrolytic work *Wₘₑₐₛ* and the electrolytic work threshold *Wₜₕ*) that will be subsequently used to detect the electrolytic detachment of a new vaso-occlusive device 24 from the delivery wire 22. The electrolytic detachment device 20 only resets the electrolytic detachment detection information in response to a delivery wire coupling event and only after a certain time has elapsed after a delivery wire decoupling event.

In particular, as briefly discussed above, the timer 80 is configured for measuring an elapsed time *Tₘₑₐₛ* between a delivery wire coupling event and a subsequent delivery wire decoupling event. The controller 72 is configured for resetting the measured electrolytic work *Wₘₑₐₛ* and the electrolytic work threshold *Wₜₕ* if the measured elapsed time *Tₘₑₐₛ* between a delivery wire coupling event and a subsequent delivery wire decoupling event reaches one or more elapsed time thresholds *Tₜₕ* (e.g., equal to or greater than three seconds). In one embodiment, the timer 80 is a countdown timer that counts down from the time threshold(s) *Tₜₕ*, such that the controller 72 may determine that the measured elapsed time *Tₘₑₐₛ* reaches the elapsed time threshold(s) *Tₜₕ* if the timer 80 expires.

In one embodiment of the electrolytic detachment device 20 for use with, e.g., the single-lumen delivery catheter 12 illustrated in Fig. 6, the controller 72 is configured for resetting the measured electrolytic work *Wₘₑₐₛ* and the electrolytic work threshold *Wₜₕ* (which, as briefly discussed above, is modified over time) if the measured elapsed time *Tₘₑₐₛ* reaches a single elapsed time threshold *Tₜₕ*.

The single elapsed time threshold *Tₜₕ* is selected to temporally distinguish between 1) physician actions that merely include quickly decoupling and recoupling the current delivery wire 22 from and to electrolytic detachment device 20 (indicative of a double-press event where the current vaso-occlusive device 24 has electrolytically detached from its delivery wire 22, but will be subjected to one more electrolytic detachment cycle) or merely include a fluoroscopic visualization check and/or taking corrective action (and associated decoupling and recoupling of the current delivery wire 22 from and to the electrolytic detachment device 20) (indicative that the current vaso-occlusive device 24 has not electrolytically detached from its delivery wire 22, and thus, at least one more electrolytic detachment cycle would need to be performed for the current vaso-occlusive device 24) and (2) physician actions that include a fluoroscope check, as well as loading a new vaso-occlusive device 24 into the delivery catheter 12 (and associated decoupling of the current delivery wire 22 from the electrolytic detachment device 20 and coupling of the new delivery wire 22 to the electrolytic detachment device 20) (indicative that the current vaso-occlusive device 24 has electrolytically detached from its delivery wire 22, and that the next electrolytic detachment cycle will be applied to a new vaso-occlusive device 24).

In general, physician actions that include a fluoroscopic visualization check followed by loading a new vaso-occlusive device 24 into the delivery catheter 12 (and associated decoupling of the current delivery wire 22 from the electrolytic detachment device 20 and coupling of the new delivery wire 22 to the electrolytic detachment device 20) will take longer than physician actions that merely include quickly decoupling and recoupling the current delivery wire 22 from and to electrolytic detachment device 20 (in the case of a double press event) or merely include a fluoroscopic visualization check and/or corrective action (and associated decoupling and recoupling of the current delivery wire 22 from and to the electrolytic detachment device 20).

Thus, the single elapsed time threshold *Tₜₕ* may be selected (e.g., in the range of 25-45 seconds), such that a measured elapsed time *Tₘₑₐₛ* between a delivery wire decoupling event and a subsequent delivery wire coupling event that is less than the single elapsed time threshold *Tₜₕ* can be assumed to involve physician actions that merely include quickly decoupling and recoupling the delivery wire 22 from and to electrolytic detachment device 20 (in the case of a double press event) or a fluoroscopic visualization check and/or corrective action (and associated decoupling and recoupling of the current delivery wire 22 from and to the electrolytic detachment device 20), whereas a measured elapsed time *Tₘₑₐₛ* between a delivery wire decoupling event and a subsequent delivery wire coupling event that is greater than the single elapsed time threshold *Tₜₕ* can be assumed to involve physician actions that include a fluoroscope check, as well as loading a new vaso-occlusive device 24 into the delivery catheter 12 (and associated decoupling of the current delivery wire 22 from the electrolytic detachment device 20 and coupling of the new delivery wire 22 to the electrolytic detachment device 20).

As such, the controller 72 will reset the measured electrolytic work Wₘₑₐₛ and the electrolytic work threshold *Wₜₕ* (e.g., the measured electrolytic work *Wₘₑₐₛ* to zero, and the electrolytic work threshold *Wₜₕ* to a default value) only if the measured elapsed time *Tₘₑₐₛ* reaches the single elapsed time threshold *Tₜₕ*, such that the reset measured electrolytic work *Wₘₑₐₛ* and the electrolytic work threshold *Wₜₕ* will be applied when subsequently assessing whether a successful electrolytic detachment event has occurred for the assumed new vaso-occlusive device 24. Thus, the controller 72 will not automatically reset the measured electrolytic work *Wₘₑₐₛ* and the electrolytic work threshold *Wₜₕ* if the measured elapsed time *Tₘₑₐₛ* does not reach the single elapsed time threshold *Tₜₕ*, thereby minimizing the chance that a false electrolytic detachment negative will be detected and reported for the current vaso-occlusive device 24 at the end of the next electrolytic detachment cycle.

In another embodiment of the electrolytic detachment device 20 for use with, e.g., the dual-lumen delivery catheter 12 illustrated in **Fig. 7****,** the controller 72 is configured for resetting the measured electrolytic work *Wₘₑₐₛ* and the electrolytic work threshold *Wₜₕ* if the measured elapsed time *Tₘₑₐₛ* reaches a relatively short elapsed time threshold *Tₜₕ₁* if a successful electrolytic detachment event has been assessed and reported to occur, and resetting the measured electrolytic work *Wₘₑₐₛ* and the electrolytic work threshold *Wₜₕ* if the measured elapsed time *Tₘₑₐₛ* reaches a second relatively long elapsed time threshold *Tₜₕ₂* if a successful electrolytic detachment event has been assessed to have not occurred and reported.

The relatively short elapsed time threshold *Tₜₕ₁* is selected to temporally distinguish between 1) physician actions that merely include quickly decoupling and recoupling the current delivery wire 22 from and to electrolytic detachment device 20 (indicative of a double-press event where the current vaso-occlusive device 24 has electrolytically detached from its delivery wire 22, but will be subjected to one more electrolytic detachment cycle); and 2) physician actions that include a fluoroscopic visualization check (and associated decoupling of the current delivery wire 22 from the electrolytic detachment device 20 and coupling of a new delivery wire 22 to the electrolytic detachment device 20) (indicative that the current vaso-occlusive device 24 has electrolytically detached from its delivery wire 22, and that the next electrolytic detachment cycle will be applied to a new vaso-occlusive device 24).

In general, physician actions that include a fluoroscopic visualization check (and associated decoupling of the current delivery wire 22 from the electrolytic detachment device 20 and coupling of a new delivery wire 22 to the electrolytic detachment device 20) will take longer than physician actions that merely include quickly decoupling and recoupling the current delivery wire 22 from and to electrolytic detachment device 20 (in the case of a double press event). Notably, the fluoroscopic visualization check can either be followed by loading a new vaso-occlusive device into a single-lumen delivery catheter 12 and coupling its delivery wire 22 to the electrolytic detachment device 20 or loading two new vaso-occlusive devices into a dual-lumen delivery catheter 12' and coupling one of their delivery wires 22 to the electrolytic detachment device 20, or can be followed merely be coupling a delivery wire 22 to the electrolytic detachment device 20 in the case where the new vaso-occlusive device 24 has been previously loaded as the second vaso-occlusive device 24 into the dual-lumen delivery catheter 12'). However, when selecting the first relatively short elapsed time threshold *Tₜₕ₁*, only the alternative case where the fluoroscopic visualization check is not followed by loading a new vaso-occlusive device 24 into a delivery catheter (in this case, when only the first vaso-occlusive device 24 previously loaded into the dual-lumen delivery catheter 12' has been electrolytically detached from its delivery wire 12, such that the next electrolytic detachment cycle will be applied to the second vaso-occlusive device 24 previously loaded into the dual-lumen delivery catheter 12') should be considered.

Thus, the relatively short elapsed time threshold *Tₜₕ₁* may be selected (e.g., in the range of 3-10 seconds), such that a measured elapsed time *Tₘₑₐₛ* between a delivery wire decoupling event and a subsequent delivery wire coupling event that is less than the relatively short elapsed time threshold *Tₜₕ₁* can be assumed to involve physician actions that merely include quickly decoupling and recoupling the delivery wire 22 from and to electrolytic detachment device 20 (in the case of a double press event), whereas a measured elapsed time *Tₘₑₐₛ* between a delivery wire decoupling event and a subsequent delivery wire coupling event that is greater than the relatively short elapsed time threshold *Tₜₕ₁* can be assumed to involve physician actions that include a fluoroscopic visualization check (and associated decoupling of the current delivery wire 22 from the electrolytic detachment device 20 and coupling of the new delivery wire 22 to the electrolytic detachment device 20).

As such, if a successful electrolytic detachment event has been assessed and reported to occur, the controller 72 will only reset the measured electrolytic work *Wₘₑₐₛ* and the electrolytic work threshold *Wₜₕ* only if the measured elapsed time *Tₘₑₐₛ* reaches the relatively short elapsed time threshold *Tₜₕ₁*, such that the reset measured electrolytic work *Wₘₑₐₛ* and the electrolytic work threshold *Wₜₕ* will be applied when subsequently assessing whether a successful electrolytic detachment event has occurred for the assumed new vaso-occlusive device 24. Thus, the controller 72 will not automatically reset the measured electrolytic work *Wₘₑₐₛ* and the electrolytic work threshold *Wₜₕ* if the measured elapsed time *Tₘₑₐₛ* does not reach the relatively short elapsed time threshold *Tₜₕ₁*, thereby preventing a confusing false electrolytic detection negative from being assessed and reported after a true electrolytic detection positive has already been detected and reported (e.g., in a double-press event).

The relatively long elapsed time threshold *Tₜₕ₂* is selected to temporally distinguish 1) physician actions that merely include quickly decoupling and recoupling the current delivery wire 22 from and to electrolytic detachment device 20 (indicative of a double-press event where the current vaso-occlusive device 24 has electrolytically detached from its delivery wire 22, but will be subjected to one more electrolytic detachment cycle) or merely include a fluoroscopic visualization check and/or taking corrective action (and associated decoupling and recoupling of the current delivery wire 22 from and to the electrolytic detachment device 20) (indicative that the current vaso-occlusive device 24 has not electrolytically detached from its delivery wire 22, and thus, at least one more electrolytic detachment cycle would need to be performed for the current vaso-occlusive device 24); and 2) physician actions that include a fluoroscope check, as well as loading a new vaso-occlusive device 24 into the delivery catheter 12 (and associated decoupling of the current delivery wire 22 from the electrolytic detachment device 20 and coupling of the new delivery wire 22 to the electrolytic detachment device 20) (indicative that the current vaso-occlusive device 24 has electrolytically detached from its delivery wire 22, and that the next electrolytic detachment cycle will be applied to a new vaso-occlusive device 24).

In general, physician actions that include a fluoroscopic visualization check followed by loading a new vaso-occlusive device 24 into the delivery catheter 12 (and associated decoupling of the current delivery wire 22 from the electrolytic detachment device 20 and coupling of the new delivery wire 22 to the electrolytic detachment device 20) will take longer than physician actions that merely include quickly decoupling and recoupling the current delivery wire 22 from and to electrolytic detachment device 20 (in the case of a double press event) or merely include a fluoroscopic visualization check and/or corrective action (and associated decoupling and recoupling of the current delivery wire 22 from and to the electrolytic detachment device 20).

Thus, the relatively long elapsed time threshold *Tₜₕ₂* may be selected (e.g., in the range of 25-45 seconds), such that a measured elapsed time *Tₘₑₛₛ* between a delivery wire decoupling event and a subsequent delivery wire coupling event that is less than the relatively long elapsed time threshold *Tₜₕ₂* can be assumed to involve physician actions that merely include quickly decoupling and recoupling the delivery wire 22 from and to electrolytic detachment device 20 (in the case of a double press event) or a fluoroscopic visualization check and/or corrective action (and associated decoupling and recoupling of the current delivery wire 22 from and to the electrolytic detachment device 20), whereas a measured elapsed time *Tₘₑₐₛ* between a delivery wire decoupling event and a subsequent delivery wire coupling event that is greater than the relatively long elapsed time threshold *Tₜₕ₂* can be assumed to involve physician actions that include a fluoroscope check, as well as loading a new vaso-occlusive device 24 into the delivery catheter 12 (and associated decoupling of the current delivery wire 22 from the electrolytic detachment device 20 and coupling of the new delivery wire 22 to the electrolytic detachment device 20).

As such, if a successful electrolytic detachment event has been assessed to have not occurred and reported, the controller 72 will reset the measured electrolytic work *Wₘₑₐₛ* and the electrolytic work threshold *Wₜₕ* only if the measured elapsed time *Tₘₑₐₛ* reaches the relatively long elapsed time threshold *Tₜₕ₂*, such that the reset measured electrolytic work *Wₘₑₐₛ* and the electrolytic work threshold *Wₜₕ* will be applied when subsequently assessing whether a successful electrolytic detachment event has occurred for the assumed new vaso-occlusive device 24. Thus, the controller 72 will not automatically reset the measured electrolytic work *Wₘₑₐₛ* and the electrolytic work threshold *Wₜₕ* if the measured elapsed time *Tₘₑₐₛ* does not reach the relatively long elapsed time threshold *Tₜₕ₂*, thereby minimizing the chance that a false electrolytic detachment negative will be detected and reported for the current vaso-occlusive device 24 at the end of the next electrolytic detachment cycle.

Significantly, as briefly discussed above, the electrolytic detachment device 20 incrementally increases the electrolytic work threshold electrolytic work threshold *Wₜₕ* over time to which the measured electrolytic work is compared measured electrolytic work *Wₘₑₐₛ* to assess whether a successful electrolytic detachment event has occurred.

In particular, the electrolytic detachment detection circuit 78, under control of the controller 72, is configured for incrementally measuring electrolytic work performed by the electrical current delivery circuitry 76 over time, thereby generating a plurality of measured electrolytic work values *Wₘₑₐₛ₋₁* to *Wₘₑₐₛ₋ₙ* (where *Wₘₑₐₛ₋₁* is the initial measured electrolytic work value, and *Wₘₑₛₛ₋ₙ* is the last measured electrolytic work value, during an electrolytic detachment cycle 90), while the controller 72 is configured for incrementally increasing the electrolytic work threshold *Wₜₕ,* thereby generating a plurality of electrolytic work threshold values *Wₜₕ₋₁* to *Wₜₕ₋ₙ* (where *Wₜₕ₋₁* is the initial electrolytic work threshold value, and *Wₜₕ₋ₙ* is the last electrolytic work threshold value), respectively comparing the measured electrolytic work values *Wₘₑₐₛ* to *Wₘₑₐₛ* to the electrolytic work threshold values *Wₜₕ₋₁* to *Wₜₕ₋ₙ,* and assessing if a successful electrolytic detachment event has occurred based on the comparisons. In this manner, the incremental increases in the electrolytic work threshold *Wₜₕ* will be commensurate with the increased quantity of electrolytic work required to effect successful detachment of the vaso-occlusive device 24 from the delivery wire 22, thereby decreasing the detection and reporting of false electrolytic detachment positives.

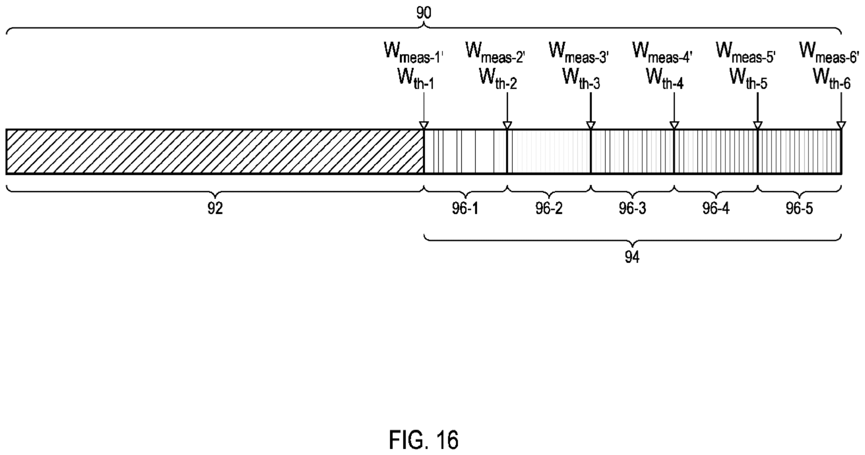

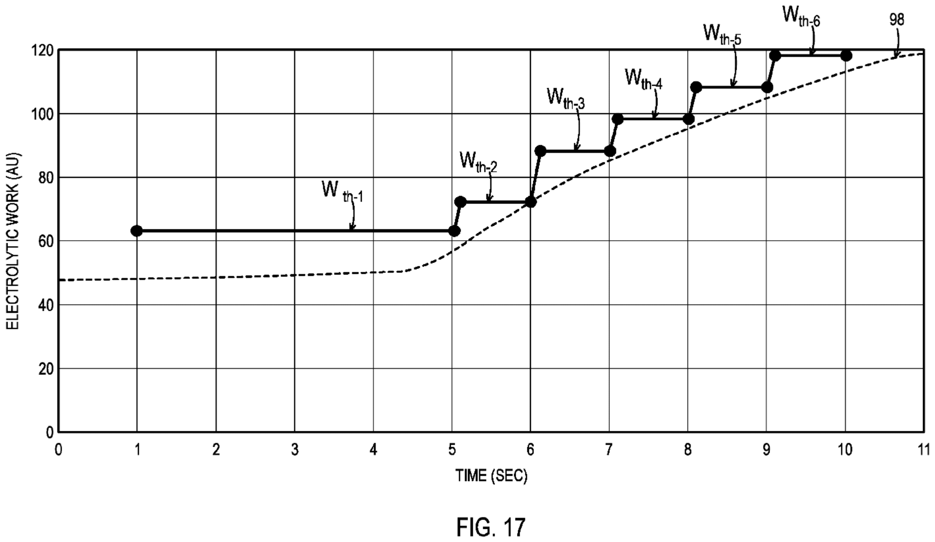

In the illustrated embodiment illustrated in Fig. 16, the electrolytic detachment detection circuit 78 measures the electrolytic work performed by the electrical current delivery circuitry 76 at by the end of the initial fixed time period 92 of an electrolytic detachment cycle 90 (in this case, at five seconds), and if the electrolytic detachment cycle 90 is extended, at the end of each fixed time increment 96 of the subsequent variable time period 94 (in this case, at six seconds, seventh seconds, and so forth until the termination of the subsequent variable time period 94) of the electrolytic detachment cycle 90, thereby generating a relatively short measured electrolytic work value *Wₘₑₐₛ₋₁* corresponding to the initial fixed time period 92, a second measured electrolytic work value *Wₘₑₐₛ₋₂* corresponding to the first fixed time increment 96, a third measured electrolytic work value *W*_{*meas*-*3*} corresponding to the second fixed time increment 96, and so forth until the termination of the subsequent variable time period 94. If the electrolytic detachment cycle 90 is extended, the controller 72 incrementally increases the electrolytic work threshold for each fixed time increment 96 of the subsequent variable time period 94 of the electrolytic detachment cycle 90, thereby generating a first electrolytic work threshold value *W*_{*th*-*1*} corresponding to the initial fixed time period 92, a second electrolytic work threshold value *W*_{*th*-*2*} corresponding to the first fixed time increment 96, a third electrolytic work threshold value *Wₜₕ₋₃* corresponding to the second fixed time increment 96, and so forth until the termination of the subsequent variable time period 94, as illustrated in **Fig. 17****.**

Thus, the controller 72 assesses if a successful electrolytic detachment event has occurred by the end of the initial fixed time period 92 if the first measured electrolytic work value *W*_{*meas*-1} reaches the first electrolytic work threshold value *Wₜₕ₋₁*; assesses if a successful electrolytic detachment event has occurred by the end of the first fixed time increment 96-1 of the subsequent variable time period 94 (assuming that a successful electrolytic detachment event has not been assessed to have occurred by the end of the initial fixed time period 92) if the second measured electrolytic work value *Wₘₑₐₛ₋₂* reaches the second electrolytic work threshold value *Wₜₕ₋₂;* assesses if a successful electrolytic detachment event has occurred by the end of the second fixed time increment 96-2 of the subsequent variable time period 94 (assuming that a successful electrolytic detachment event has not been assessed to have occurred by the end of the second fixed time increment 96-2) if the third measured electrolytic work value *Wₘₑₐₛ₋₃* reaches the third electrolytic work threshold value *Wₜₕ₋₃;* assesses if a successful electrolytic detachment event has occurred by the end of the third fixed time increment 96-3 of the subsequent variable time period 94 (assuming that a successful electrolytic detachment event has not been assessed to have occurred by the end of the second fixed time increment 96-2) if the third measured electrolytic work value *Wₘₑₐₛ₋₃* reaches the third electrolytic work threshold value *Wₜₕ₋₃*; assesses if a successful electrolytic detachment event has occurred by the end of the fourth fixed time increment 96-4 of the subsequent variable time period 94 (assuming that a successful electrolytic detachment event has not been assessed to have occurred by the end of the third fixed time increment 96-3) if the fourth measured electrolytic work value *W*_{*meas*-*4*} reaches the fourth electrolytic work threshold value *Wₜₕ₋₄;* and assesses if a successful electrolytic detachment event has occurred by the end of the fifth fixed time increment 96-5 of the subsequent variable time period 94 (assuming that a successful electrolytic detachment event has not been assessed to have occurred by the end of the fourth fixed time increment 96-4) if the fifth measured electrolytic work value *Wₘₑₐₛ₋₅* reaches the fifth electrolytic work threshold value *W*_{*th*-*5*}.

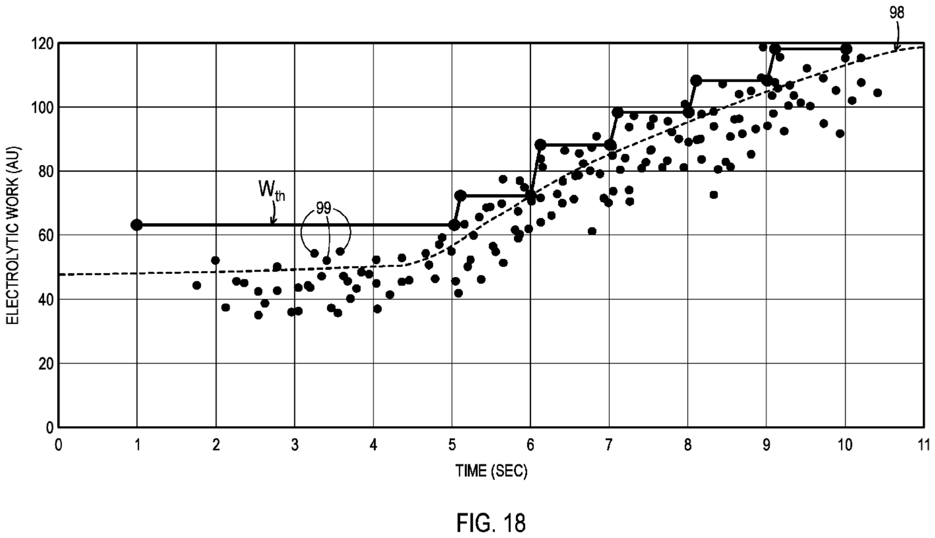

The measured electrolytic work values *Wₘₑₐₛ₋₁* to *Wₘₑₐₛ₋ₙ* will naturally increase over time as more electrolytic work is performed by the electrical current delivery circuitry 76 to effect the electrolytic detachment of the vaso-occlusive device 24 from its delivery wire 22, whereas the electrolytic work threshold values *Wₜₕ₋₁* to *Wₜₕ₋ₙ* may be selected in accordance with a known electrolytic work-time detachment curve 98 that generally increases over time (shown as a dashed line in Fig. 17). Such electrolytic work-time detachment curve 98 contains the magnitude of electrolytic work (in this case, cumulative current) required (with tolerance) to electrolytically detach a particular type of the vaso-occlusive device 24 from its delivery wire 22 as a function of time. The electrolytic work-time detachment curve 98 may be generated by experimentally testing a relatively large number of vaso-occlusive assemblies (e.g., one hundred) of a particular type (i.e., delivering electrical current to each vaso-occlusive assembly while visualizing the vaso-occlusive device, and noting the electrolytic work and elapsed time at which the vaso-occlusive device electrolytically detaches from its delivery wire), thereby generating a set of points 99, as illustrated in **Fig. 18****.** An electrolytic work-time detachment curve 98 may then be generated by fitting a continuous curve to the set of points 99, and then fitting the discrete electrolytic work threshold values *Wₜₕ₋₁* to *Wₜₕ₋ₙ* to the electrolytic work-time detachment curve 98. Notably, the discrete electrolytic work threshold values *Wₜₕ₋₁* to *Wₜₕ₋ₙ* are fitted with some tolerance in the electrolytic work, such that the discrete electrolytic work threshold values *Wₜₕ₋₁* to *Wₜₕ₋ₙ* are generally higher than the electrolytic work-time detachment curve 98 by a certain magnitude. As such, the discrete electrolytic work threshold values *Wₜₕ₋₁* to *Wₜₕ₋ₙ* are conservative estimates indicative of a successful electrolytic detachment event, thereby minimizing false positive electrolytic detachment detections.

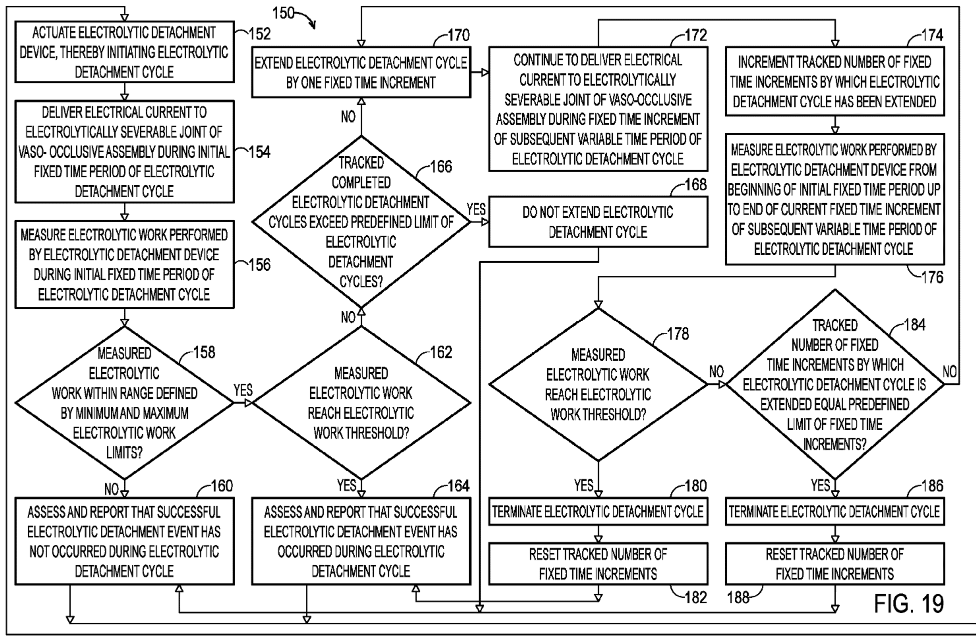

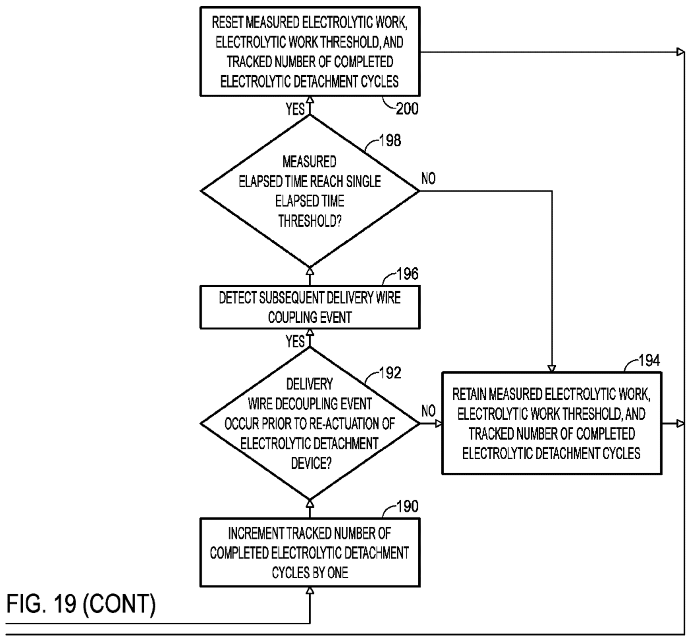

Having described the structure and function of the electrolytic detachment device 20, one method 150 of operating the electrolytic detachment device 20 to perform a series of electrolytic detachment cycles 90 to electrolytically detach a vaso-occlusive device 24 from its delivery wire 22 will now be described with respect to **Figs. 16** and **19****.**

Prior to initiating the method 150, it is assumed that the vaso-occlusive assembly 14 is disposed in the delivery catheter 12, with the vaso-occlusive device 24 deployed within an aneurysmal sac 100, and the electrolytically severable joint 26 located distal to the distal port 36 of the delivery catheter 22 and exposed to the blood within the blood vessel 104, as illustrated in **Fig. 10****,** while the proximal portion 52 of the core wire 48 of the delivery wire 22 is electrically coupled to the power terminal (and in particular, inserted into the power port 64) of the electrolytic detachment device 20, and the ground electrode 16 is placed into firm contact with the patient and electrically coupled to the ground terminal (and in particular, inserted into the ground port 66) of the electrolytic detachment device 20 via the electrical cable 18, as illustrated in **Figs. 3-4****.**

First, a single actuation of the electrolytic detachment device 20 is performed (and in this case, the push button 70 (shown in **Fig. 8****)** is quickly depressed and released), thereby initiating an electrolytic detachment cycle 90 (step 152). As a result, the electrolytic detachment device 20 delivers electrical current to the electrolytically severable joint 26 during an initial fixed time period 92 of the electrolytic detachment cycle 90 (step 154).

The electrolytic detachment device 20 then assesses if a successful electrolytic detachment event has occurred by the end of the initial fixed time period 92 of the electrolytic detachment cycle 90. If a successful electrolytic detachment event has been assessed to have occurred by the end of the initial fixed time period 92 of the electrolytic detachment cycle 90, the electrolytic detachment device 20 reports the result to the physician. If a successful electrolytic detachment event has not been assessed to have occurred by the end of the initial fixed time period 92 of the electrolytic detachment cycle 90, the electrolytic detachment device 20 may extend the electrolytic detachment cycle 90 by a subsequent variable time period 94 if it is deemed to be useful to do so, reassesses if successful electrolytic detachment event has been assessed to have occurred during the subsequent variable time period 94 of the electrolytic detachment cycle 90, and then reports the result to the physician at the end of the extended electrolytic detachment cycle 90.

In particular, the electrolytic detachment device 20 measures the electrolytic work *Wₘₑₐₛ* performed by the electrolytic detachment device 20 (in this case, the cumulative electrical current delivered by the electrolytic detachment device 20 through the delivery wire 22) during the initial fixed time period 92 of the electrolytically detachable cycle 90 (step 156). As a result, a first measured electrolytic work value *Wₘₑₛₛ₋₁* is generated.

The electrolytic detachment device 20 then determines if an electrolytic fault has occurred during the delivery of the electrical current to the electrolytically severable joint 26 during the initial fixed time period 92 of the electrolytic detachment cycle 90, and in particular, determines if the measured electrolytic work *Wₘₑₐₛ* (in this case, the first measured electrolytic work value *Wₘₑₐₛ₋₁*) is within the electrolytic work range defined by the minimum electrolytic work limit *Wₗᵢₘ₋ₘᵢₙ* and the maximum electrolytic work limit *Wₗᵢₘ₋ₘₐₓ* (step 158). If the measured electrolytic work is outside the electrolytic work range defined by the minimum electrolytic work limit *Wₗᵢₘ₋ₘᵢₙ* and the maximum electrolytic work limit *Wₗᵢₘ₋ₘₐₓ,* the electrolytic detachment device 20 assesses and reports (via the cycle complete indicator 86c) that a successful electrolytic detachment event has not occurred during the initial fixed time period 92 of the electrolytic detachment cycle 90 (step 160). If the measured electrolytic work is within the electrolytic work range defined by the minimum electrolytic work limit *Wₗᵢₘ₋ₘᵢₙ* and the maximum electrolytic work limit *Wₗᵢₘ₋ₘₐₓ,* the electrolytic detachment device 20 determines if the measured electrolytic work *Wₘₑₐₛ* (in this case, the measured electrolytic work value *Wₘₑₐₛ₋₁*) reaches the electrolytic work threshold *Wₜₕ* (in this case, the first electrolytic work threshold value *Wₜₕ₋₁*) (step 162). If the measured electrolytic work *Wₘₑₐₛ* does reach the first electrolytic work threshold *Wₜₕ,* the electrolytic detachment device 20 assesses and reports (via the cycle complete indicator 86c) that a successful electrolytic detachment event has occurred during the electrolytic detachment cycle 90 (step 164).

If the measured electrolytic work *Wₘₑₐₛ* does not reach the electrolytic work threshold *Wₜₕ,* the electrolytic detachment device 20 determines if the tracked number of completed electrolytic detachment cycles *N_{cycle}* (as tracked by one of the counters 82) exceeds the predefined limit of completed electrolytic detachment cycles *N_{cycle-lim}* (step 166). If the tracked number of completed electrolytic detachment cycles *N_{cycle}* does exceed the predefined limit of completed electrolytic detachment cycles *N_{cycle-lim},* the electrolytic detachment device 20 does not extend the electrolytic detachment cycle 90 (step 168) and assesses and reports (via the cycle complete indicator 86c) that a successful electrolytic detachment event has not occurred during the initial fixed time period 92 of the electrolytic detachment cycle 90 (step 160).

If the tracked number of completed electrolytic detachment cycles *N_{cycle}* does not exceed the predefined limit of completed electrolytic detachment cycles *N_{cycle-lim},* the electrolytic detachment device 20 then incrementally assesses if a successful electrolytic detachment event has occurred respectively during the time increments 96 of the subsequent variable time period 94, terminates the electrolytic detachment cycle 90 at the end of the time increment 96 at which the successful electrolytic detachment event has been assessed to have occurred, and reports the result to the physician.

In particular, the electrolytic detachment device 20 extends the electrolytic detachment cycle 90 by the subsequent variable time period 94, and in particular, extends the electrolytic detachment cycle 90 by one fixed time increment 96 (step 170). As a result, the electrolytic detachment device 20 continues to deliver the electrical current to the electrolytic severable joint 26 of the vaso-occlusive assembly 14 during the fixed time increment 96 of the variable time period 94 of the electrolytic detachment cycle 90 (step 172). The electrolytic detachment device 20 then increments the tracked number of fixed time increments *N_{TI}* by which the current electrolytic cycle 90 has been extended (i.e., via another one of counters 82) by one (step 174).

The electrolytic detachment device 20 then measures the electrolytic work *Wₘₑₐₛ* performed by the electrolytic detachment device 20 (in this case, the cumulative electrical current delivered by the electrolytic detachment device 20 through the delivery wire 22) from the beginning of the initial fixed time period 92 up to the end of the current fixed time increment 96 of the subsequent variable time period 94 of the electrolytically detachable cycle 90 (step 176), and increases the electrolytic work threshold *Wₜₕ* (step 178). In particular, one of the second through sixth measured electrolytic work values *W*_{*meas*-*2*} to *Wₘₑₐₛ₋₆* corresponding to the current fixed time increment 96 of the subsequent variable time period 94 of the electrolytic detachment cycle 90 is generated, and one of the incrementally increased electrolytic work threshold values *Wₜₕ₋₂* to *Wₜₕ₋₆* corresponding to the current fixed time increment 96 of the subsequent variable time period 94 of the electrolytic detachment cycle 90 is selected (e.g., in accordance with the incrementally increased electrolytic work threshold values *Wₜₕ₋₂* to *W*_{*th*-*6*} illustrated in **Fig. 17****).**

Thus, in the case where the electrolytic detachment cycle 90 is initially extended, the tracked number of fixed time increments *N_{TI}* by which the current electrolytic cycle 90 has been extended will be incremented from zero to one (i.e., the current fixed time increment 96 will be the first fixed time increment 96-1), the second measured electrolytic work value *Wₘₑₐₛ₋₂* will be generated, and the second electrolytic work threshold *Wₜₕ₋₂* will be selected. In the next iteration, the tracked number of fixed time increments *N_{TI}* by which the current electrolytic cycle 90 has been extended will be incremented from one to two (i.e., the current fixed time increment 96 will be the second fixed time increment 96-2), the third measured electrolytic work value *Wₘₑₐₛ₋₃* will be generated, and the third electrolytic work threshold *Wₜₕ₋₃* will be selected. In the next iteration, the tracked number of fixed time increments *N_{TI}* by which the current electrolytic cycle 90 has been extended will be incremented from two to three (i.e., the current fixed time increment 96 will be the third fixed time increment 96-3), the fourth measured electrolytic work value *Wₘₑₐₛ₋₄* will be generated, and the fourth electrolytic work threshold *Wₜₕ₋₄* will be selected. In the next iterations, the tracked number of fixed time increments *N_{TI}* by which the current electrolytic cycle 90 has been extended will be incremented from three to four (i.e., the current fixed time increment 96 will be the fourth fixed time increment 96-4), the fifth measured electrolytic work value *Wₘₑₐₛ₋₅* will be generated, and the fifth electrolytic work threshold *Wₜₕ₋₅* will be selected. In the next iterations, the tracked number of fixed time increments *N_{TI}* by which the current electrolytic cycle 90 has been extended will be incremented from four to five (i.e., the current fixed time increment 96 will be the fifth fixed time increment 96-5), the sixth measured electrolytic work value *Wₘₑₐₛ₋₆* will be generated, and the sixth electrolytic work threshold *Wₜₕ₋₆* will be selected.

The electrolytic detachment device 20 then determines if the measured electrolytic work *Wₘₑₐₛ* (the currently generated one of the measured electrolytic work values *Wₘₑₐₛ₋₂* to *Wₘₑₛₛ₋₆*) reaches the electrolytic work threshold (the currently selected one of the electrolytic work threshold values *Wₜₕ₋₂* to *Wₜₕ₋₆*) (step 178). If the measured electrolytic work *Wₘₑₐₛ* does reach the selected electrolytic work threshold *Wₜₕ,* the electrolytic detachment device 20 terminates the electrolytic detachment cycle 90 (step 180), resets the tracked number of fixed time increments *N_{TI}* (via one of the counters 82) (step 182), and assesses and reports (via the cycle complete indicator 86c) that a successful electrolytic detachment event has occurred during the electrolytic detachment cycle 90 (step 164). If the measured electrolytic work *Wₘₑₐₛ* does not reach the selected electrolytic work threshold *Wₜₕ,* the electrolytic detachment device 20 determines if the tracked number of fixed time increments *N_{TI}* by which the current electrolytic cycle 90 has been extended (as tracked by one of the counters 82) equals the predefined limit of fixed time increments *N_{TI-lim}* (step 184).

If the tracked number of fixed time Increments *N_{TI}* by which the current electrolytic cycle 90 has been extended equals the predefined limit of fixed time increments *N_{TI-lim},* the electrolytic detachment device 20 terminates the electrolytic detachment cycle 90 (step 186), resets the tracked number of fixed time increments *N_{TI}* (via one of the counters 82) (step 188), and assesses and reports (via the cycle complete indicator 86c) that a successful electrolytic detachment event has not occurred during the electrolytic detachment cycle 90 (step 160). If the tracked number of fixed time increments *N_{TI}* by which the current electrolytic cycle 90 has been extended is less than the predefined limit of fixed time increments *N_{TI-lim},* the method 150 returns back to step 170.

After the electrolytic detachment cycle 90 (whether or not extended) has been completed and the occurrence or non-occurrence of a successful electrolytic event has been reported to the physician at steps 160 or 164, the electrolytic detachment device 20 resets the tracked number of completed electrolytic detachment cycles *N_{cycle}* (step 190), and detects if a delivery wire decoupling event occurs (i.e., whether proximal end of the delivery wire 22 has been decoupled from the electrolytic detachment device 20) prior to re-actuation of the electrolytic detachment device 20 (i.e., initiation of another electrolytic detachment cycle) (step 192). If a delivery wire decoupling event has not occurred prior to the re-actuation of the electrolytic detachment device 20, the electrical parameter information, including the measured electrolytic work *Wₘₑₐₛ* and the electrolytic work threshold *Wₜₕ,* as well as the tracked number of completed electrolytic detachment cycles *N_{cycle},* are retained (i.e., not reset) (step 194).

If a delivery wire decoupling event has occurred, the electrolytic detachment device 20 then detects the occurrence of a subsequent delivery wire coupling event (i.e., whether proximal end of a delivery wire 22 (either the previous delivery wire 22 or the next delivery wire 22) or has been coupled to the electrolytic detachment device 20) (step 196), and determines the measured elapsed time *Tₘₑₐₛ* between the delivery wire decoupling event and the delivery wire coupling event (step 198). For example, the countdown timer 80 may be started, such that it counts down from an elapsed time threshold *Tₜₕ*. The electrolytic detachment device 20 may then determine if the measured elapsed time *Tₘₑₐₛ* reaches an elapsed time threshold *Tₜₕ* by determining whether or not the countdown timer 80 has expired prior to the delivery wire coupling event. In the method 150 illustrated in **Fig. 19****,** the electrolytic detachment device 20 determines if the measured elapsed time *Tₘₑₐₛ* reaches a single elapsed time threshold *Tₜₕ* without regard to the whether or not the occurrence of a successful electrolytic detachment event has been assessed and reported. Thus, the method 150 lends itself well to the single-lumen delivery catheter 12 illustrated in **Fig. 6****.**

If the measured elapsed time *Tₘₑₐₛ* does not reach the elapsed time threshold *Tₜₕ* (i.e., the countdown timer 80 did not expire prior to the delivery wire coupling event), the electrolytic detachment device 20 retains (i.e., does not reset) the electrical parameter information, including the measured electrolytic work *Wₘₑₐₛ* and the electrolytic work threshold *Wₜₕ,* as well as the tracked number of completed electrolytic detachment cycles *N_{cycle}* (step 194). In such case, it is assumed that, immediately prior to the delivery wire coupling event, the physician has performed no action (indicative of a double-press event where the current vaso-occlusive device 24 has electrolytically detached from its delivery wire 22, but will be subjected to one more electrolytic detachment cycle) or the physician has performed a fluoroscopic visualization check and/or taken corrective action without subsequently loading a new vaso-occlusive device 24 into the delivery catheter 12 (indicative that the current vaso-occlusive device 24 has not electrolytically detached from its delivery wire 22, and thus, at least one more electrolytic detachment cycle would need to be performed for the current vaso-occlusive device 24). Thus, resetting of the measured electrolytic work *Wₘₑₐₛ* and the electrolytic work threshold *Wₜₕ,* as well as the tracked number of completed electrolytic detachment cycles *N_{cycle},* would be undesirable in this case.

If the measured elapsed time *Tₘₑₐₛ* does reach the elapsed time threshold *Tₜₕ* (i.e., the countdown timer 80 expired prior to the delivery wire coupling event), the electrolytic detachment device 20 resets the electrical parameter information, including the measured electrolytic work *Wₘₑₐₛ* and the electrolytic work threshold *Wₜₕ,* as well as the tracked number of completed electrolytic detachment cycles *N_{cycle}* (step 200). In such case, it is assumed that, immediately prior to the delivery wire coupling event, the physician has performed a fluoroscopic visualization check, followed by loading a new vaso-occlusive device 24 into the delivery catheter 12 (indicative that the current vaso-occlusive device 24 has electrolytically detached from its delivery wire 22, and that the next electrolytic detachment cycle will be applied to the new vaso-occlusive device 24). Thus, resetting of the measured electrolytic work *Wₘₑₐₛ* and the electrolytic work threshold *Wₜₕ,* as well as the tracked number of completed electrolytic detachment cycles *N_{cycle},* would be desirable in this case.

After the electrical parameter information, including the measured electrolytic work *Wₘₑₐₛ* and the electrolytic work threshold *Wₜₕ,* as well as the tracked number of completed electrolytic detachment cycles *N_{cycle}* is either retained at step 194 or reset at 200, the method 150 returns to step 152 to initiate anther electrolytic detachment cycle 90.

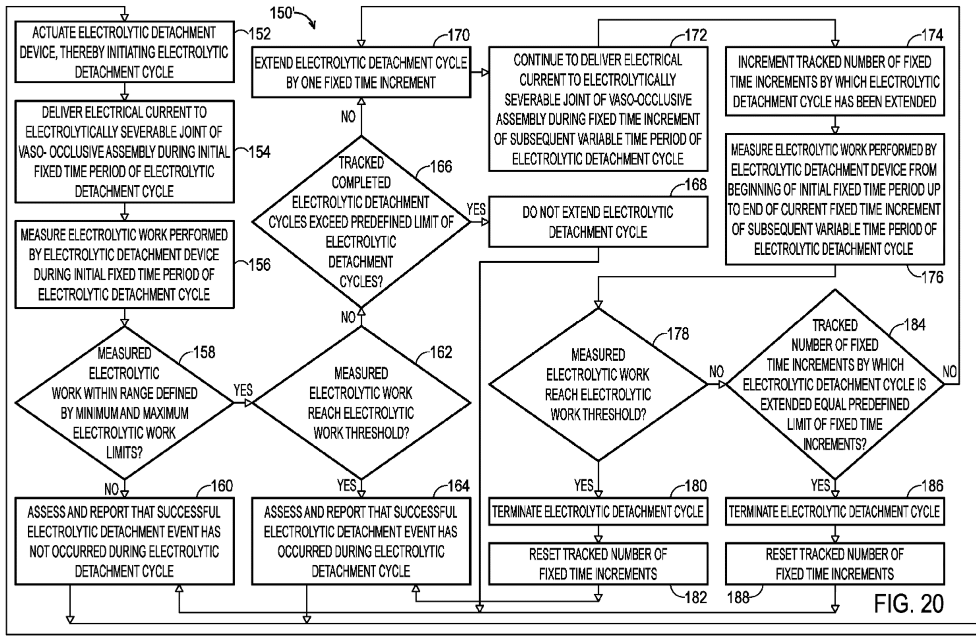

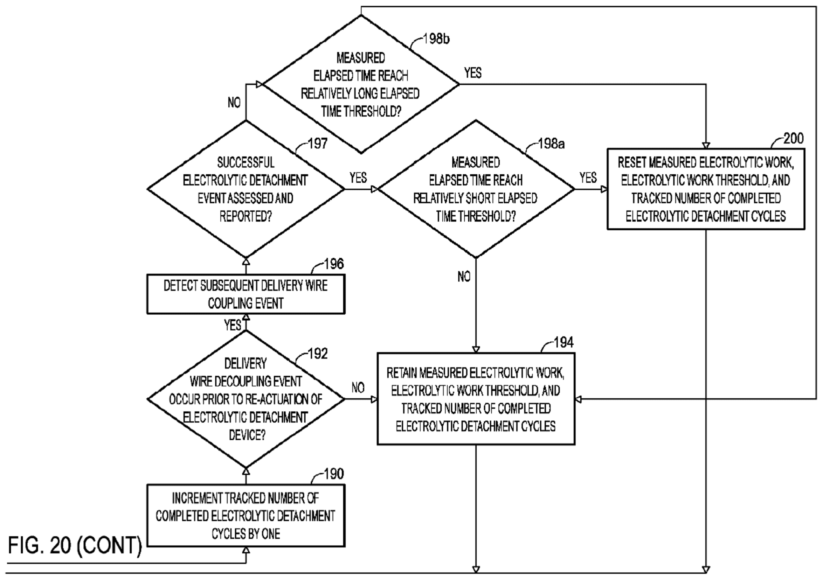

Referring now to **Fig. 20****,** an alternative method 150' is similar to the method 150 illustrated in **Fig. 19****,** with the exception that the electrolytic detachment device 20 determines if the measured elapsed time *Tₘₑₐₛ* reaches a relatively short elapsed time threshold *Tₜₕ₁* if a successful electrolytic detachment event has been assessed and reported as occurring (step 198a), and determines if the measured elapsed time *Tₘₑₐₛ* reaches a relatively long elapsed time threshold *Tₜₕ₂* if a successful electrolytic detachment event has not been assessed and reported as occurring (step 198b). For example, the countdown timer 80 may be started, such that it counts down from either the relatively short elapsed time threshold *Tₜₕ₁* or the relatively long elapsed time threshold *Tₜₕ₂*. The electrolytic detachment device 20 may then determine if the measured elapsed time *Tₘₑₐₛ* reaches relatively short elapsed time threshold *Tₜₕ₁* or the relatively long elapsed time threshold *Tₜₕ₂* by determining whether or not the countdown timer 80 has expired prior to the delivery wire coupling event. The method 150' lends itself well to the dual-lumen delivery catheter 12' illustrated in **Fig. 7****.**

If a successful electrolytic detachment event has been assessed and reported (step 197), and if the measured elapsed time *Tₘₑₐₛ* does not reach the relatively short elapsed time threshold *Tₜₕ₁* (i.e., the countdown timer 80 did not expire prior to the delivery wire coupling event) (step 198a), the electrolytic detachment device 20 retains (i.e., does not reset) the electrical parameter information, including the measured electrolytic work *Wₘₑₐₛ* and the electrolytic work threshold *Wₜₕ,* as well as the tracked number of completed electrolytic detachment cycles *N*_{*c*y*cle*} (step 194). In such case, it is assumed that, immediately prior to the delivery wire coupling event, the physician has performed no action (indicative of a double-press event where the current vaso-occlusive device 24 has electrolytically detached from its delivery wire 22, but will be subjected to one more electrolytic detachment cycle). Thus, resetting of the measured electrolytic work *Wₘₑₐₛ* and the electrolytic work threshold *Wₜₕ,* as well as the tracked number of completed electrolytic detachment cycles *N_{cycle},* would be undesirable in this case.

If a successful electrolytic detachment event has been assessed and reported (step 197), and if the measured elapsed time *Tₘₑₐₛ* reaches the relatively short elapsed time threshold *Tₜₕ₁* (i.e., the countdown timer 80 expires prior to the delivery wire coupling event) (step 198a), the electrolytic detachment device 20 resets the electrical parameter information, including the measured electrolytic work *Wₘₑₐₛ* and the electrolytic work threshold *Wₜₕ,* as well as the tracked number of completed electrolytic detachment cycles *N_{cycle}* (step 200). In such case, it is assumed that, immediately prior to the delivery wire coupling event, the physician has performed a fluoroscopic visualization check (indicative that the current vaso-occlusive device 24 has electrolytically detached from its delivery wire 22, and that the next electrolytic detachment cycle will be applied to a new vaso-occlusive device 24 that has been previously loaded into the delivery catheter 12'). Thus, resetting of the measured electrolytic work *Wₘₑₐₛ* and the electrolytic work threshold *Wₜₕ,* as well as the tracked number of completed electrolytic detachment cycles *N_{cycle}*, would be desirable in this case.

If a successful electrolytic detachment event has been not been assessed and reported (step 197), and if the measured elapsed time *Tₘₑₐₛ* does not reach the relatively long elapsed time threshold *Tₜₕ₂* (i.e., the countdown timer 80 did not expire prior to the delivery wire coupling event) (step 198b), the electrolytic detachment device 20 retains (i.e., does not reset) the electrical parameter information, including the measured electrolytic work *Wₘₑₐₛ* and the electrolytic work threshold *Wₜₕ,* as well as the tracked number of completed electrolytic detachment cycles *N_{cycle}* (step 194). In such case, it can be assumed that, immediately prior to the delivery wire coupling event, the physician has performed no action (indicative of a double-press event where the current vaso-occlusive device 24 has electrolytically detached from its delivery wire 22, but will be subjected to one more electrolytic detachment cycle) or the physician has performed a fluoroscopic visualization check and/or taken corrective action without subsequently loading a new vaso-occlusive device 24 into the delivery catheter 12 (indicative that the current vaso-occlusive device 24 has not electrolytically detached from its delivery wire 22, and thus, at least one more electrolytic detachment cycle would need to be performed for the current vaso-occlusive device 24). Thus, resetting of the measured electrolytic work *Wₘₑₐₛ* and the electrolytic work threshold *Wₜₕ,* as well as the tracked number of completed electrolytic detachment cycles *N_{cycle},* would be undesirable in this case.

**If** a successful electrolytic detachment event has been not been assessed and reported (step 197), and if the measured elapsed time *Tₘₑₐₛ* reaches the relatively long elapsed time threshold *Tₜₕ₂* (i.e., the countdown timer 80 expires prior to the delivery wire coupling event) (step 198b), the electrolytic detachment device 20 resets the electrical parameter information, including the measured electrolytic work *Wₘₑₐₛ* and the electrolytic work threshold *Wₜₕ,* as well as the tracked number of completed electrolytic detachment cycles *N_{cycle}* (step 200). In such case, it can be assumed that, immediately prior to the delivery wire coupling event, the physician has performed a fluoroscopic visualization check, followed by a loading of two new vaso-occlusive devices 24 into the delivery catheter 12' (indicative that the current vaso-occlusive device 24 has electrolytically detached from its delivery wire 22, and that the next electrolytic detachment cycle will be applied to one of the two new vaso-occlusive devices 24). Thus, resetting of the measured electrolytic work *Wₘₑₐₛ* and the electrolytic work threshold *Wₜₕ,* as well as the tracked number of completed electrolytic detachment cycles *N_{cycle}*, would be desirable in this case.

Although particular embodiments have been shown and described herein, it will be understood by those skilled in the art that they are not intended to limit the disclosed inventions, and it will be obvious to those skilled in the art that various changes, permutations, and modifications may be made (e.g., the dimensions of various parts, combinations of parts) without departing from the scope of the disclosed inventions, which is to be defined only by the following claims and their equivalents. The specification and drawings are, accordingly, to be regarded in an illustrative rather than restrictive sense. The various embodiments shown and described herein are intended to cover alternatives, modifications, and equivalents of the disclosed inventions, which may be included within the scope of the appended claims.

### NUMBERED EMBODIMENTS OF THE INVENTION

1. An electrolytic detachment device for use with an electrically conductive delivery wire having a distal end to which a respective vaso-occlusive device is attached and an electrolytically severable joint located proximal to the vaso-occlusive device, comprising:
   a power terminal to which a proximal end of the delivery wire is configured for being electrically coupled, and from which the proximal end of the delivery wire is configured for being electrically decoupled;
   electrical current delivery circuitry configured for delivering electrical current to the electrolytically severable joint of the vaso-occlusive assembly while the vaso-occlusive device is disposed within a vasculature of a patient, such that the vaso-occlusive device electrolytically detaches from the distal end of the delivery wire;
   an electrolytic detachment detection circuit configured for generating electrical parameter information indicative of a successful electrolytic detachment event;
   a timer configured for measuring an elapsed time between a delivery wire decoupling event and a subsequent delivery wire coupling event; and
   a controller configured for assessing if the successful electrolytic detachment event has occurred based on the generated electrical parameter information, and resetting the electrical parameter information if the measured elapsed time reaches a first elapsed time threshold.
2. The electrolytic detachment device of claim 1, further comprising a ground terminal to which a ground electrode in electrical contact with the patient is configured for being electrically coupled via an electrical cable.
3. The electrolytic detachment device of claim 1, wherein the power terminal is a power port into which the proximal end of the delivery wire is configured for being inserted.
4. The electrolytic detachment device of claim 1, wherein the electrical parameter information comprises a measured cumulative electrical parameter.
5. The electrolytic detachment device of claim 4, wherein the cumulative electrical parameter comprises measured cumulative current.
6. The electrolytic detachment device of claim 1, wherein the electrical parameter information comprises measured electrolytic work performed by the electrical current delivery circuitry.
7. The electrolytic detachment device of claim 1, wherein the first elapsed time threshold is equal to or greater than three seconds.
8. The electrolytic detachment device of claim 1, wherein the first elapsed time threshold is in the range of 25-45 seconds.
9. The electrolytic detachment device of claim 1,
   wherein the controller is configured for resetting the electrical parameter information if the measured elapsed time reaches the first elapsed time threshold and the successful electrolytic detachment event has been assessed to have occurred; and
   wherein the controller is configured for resetting the electrical parameter information if the measured elapsed time reaches a second elapsed time threshold longer than the first elapsed time threshold and the successful electrolytic detachment event has been assessed to have not occurred.
10. The electrolytic detachment device of claim 9, wherein the first elapsed time threshold is in the range of 3-10 seconds, and the second elapsed time threshold is in the range of 25-45 seconds.
11. The electrolytic detachment device of claim 1, wherein:
   the controller is configured for generating a first user-discernible notification if the successful electrolytic detachment event has been assessed to have occurred; and
   the controller is configured for generating a second user-discernible notification different from the first user-discernible notification if the successful electrolytic detachment event has been assessed to have not occurred.
12. The electrolytic detachment device of claim 1, wherein the electrical parameter information comprises measured electrolytic work performed by the electrical current delivery circuitry, and wherein the controller is configured for assessing that the successful electrolytic detachment event has not occurred if the measured electrolytic work has not reached an electrolytic work threshold, and assessing that the successful electrolytic detachment event has occurred if the measured electrolytic work has reached the electrolytic work threshold.
13. The electrolytic detachment device of claim 12, wherein the controller is configured for incrementally increasing the electrolytic work threshold over time.
14. The electrolytic detachment device of claim 1, further comprising an electrical current delivery actuator, wherein the controller is configured for operating the electrical current delivery circuitry to deliver the electrical current to the electrolytically severable joint during an electrolytic detachment cycle in response to a single actuation of the electrical current delivery actuator.
15. The electrolytic detachment device of claim 14, wherein the controller is configured for operating the electrical current delivery circuitry to deliver the electrical current to the electrolytically severable joint during another electrolytic detachment cycle in response to another single actuation of the electrical current delivery actuator.
16. The electrolytic detachment device of claim 14, wherein the electrolytic detachment cycle has a fixed time period, and the controller is configured for assessing if the successful electrolytic detachment event has occurred during the fixed time period.
17. The electrolytic detachment device of claim 16, wherein the controller is further configured for extending the electrolytic detachment cycle only if the successful electrolytic detachment event has been assessed to have not occurred during the fixed time period.
18. The electrolytic detachment device of claim 17, wherein the fixed time period is an initial fixed time period, wherein the controller is configured for assessing if the successful electrolytic detachment event has occurred by the end of the initial fixed time period, and extending the electrolytic detachment cycle by a subsequent variable time period only if the successful electrolytic detachment event has been assessed to have not occurred during the initial fixed time period.
19. The electrolytic detachment device of claim 18, wherein the controller is configured for incrementally assessing if the successful electrolytic detachment event has occurred during the subsequent variable time period, and terminating the electrolytic detachment cycle when the successful electrolytic detachment event is assessed to have occurred.
20. The electrolytic detachment device of claim 19, wherein the subsequent variable time period has a plurality time increments, each of which is shorter than the initial fixed time period of the electrolytic detachment cycle, and wherein the controller is configured for incrementally assessing if the successful electrolytic detachment event has occurred respectively during the plurality of time increments, and terminating the electrolytic detachment cycle at the end of the time increment at which the successful electrolytic detachment event has been assessed to have occurred.
21. The electrolytic detachment device of claim 17, wherein the controller is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint.
22. The electrolytic detachment device of claim 21, wherein the electrical parameter information comprises measured electrolytic work performed by the electrical current delivery circuitry wherein the controller is configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.
23. The electrolytic detachment device of claim 17, further comprising a counter configured for tracking a number of completed electrical detachment cycles, wherein the controller is configured for not extending the electrolytic detachment cycle if the tracked number of completed electrolytic detachment cycles exceeds a predefined number of electrolytic detachment cycles.
24. A vaso-occlusive treatment system, comprising:
   a delivery catheter configured for being introduced into a vasculature of a patient, the delivery catheter comprising an elongate sheath body, an inner lumen extending through the elongate sheath body, and a distal port in respective communication with the inner lumen;
   a vaso-occlusive assembly configured for being disposed within inner lumen of the delivery catheter, the vaso-occlusive assembly comprising a delivery wire and a vaso-occlusive device detachably coupled to the delivery wire via an electrolytically severable joint, the vaso-occlusive device configured for being deployed out from the distal port of the delivery catheter into the vasculature of the patient; and
   an electrolytic detachment device to which a proximal end of the delivery wire of the vaso-occlusive assembly is configured for being electrically coupled, and from which the proximal end of the delivery wire of the vaso-occlusive assembly is configured for being electrically decoupled, delivering electrical current to the electrolytically severable joint of the vaso-occlusive assembly while the vaso-occlusive device is disposed within the vasculature of the patient, such that the vaso-occlusive device electrolytically detaches from the distal end of the delivery wire, generating electrical parameter information indicative of a successful electrolytic detachment event, assessing if the successful electrolytic detachment event has occurred based on the generated electrical parameter information, measuring an elapsed time between a delivery wire decoupling event and a subsequent delivery wire coupling event, and resetting the electrical parameter information if the measured elapsed time reaches a first elapsed time threshold.
25. The vaso-occlusive treatment system of claim 24, further comprising a ground electrode configured for being placed in electrical contact with the patient and for being electrically coupled to the electrolytic detachment device via an electrical cable.
26. The vaso-occlusive treatment system of claim 24, wherein the electrolytic detachment device is a handheld electrolytic detachment device.
27. The vaso-occlusive treatment system of claim 24, wherein the electrical parameter information comprises a measured cumulative electrical parameter.
28. The vaso-occlusive treatment system of claim 27, wherein the cumulative electrical parameter comprises measured cumulative current.
29. The vaso-occlusive treatment system of claim 24, wherein the electrical parameter information comprises measured electrolytic work performed by the electrolytic detachment device.
30. The vaso-occlusive treatment system of claim 24, wherein the first elapsed time threshold is equal to or greater than three seconds.
31. The vaso-occlusive treatment system of claim 24, wherein the first elapsed time threshold is in the range of 25-45 seconds.
32. The vaso-occlusive treatment system of claim 24, wherein the electrolytic detachment device is configured for resetting the electrical parameter information if the measured elapsed time reaches the first elapsed time threshold and the successful electrolytic detachment event has been assessed to have occurred, and for resetting the electrical parameter information if the measured elapsed time reaches a second elapsed time threshold longer than the first elapsed time threshold and the successful electrolytic detachment event has been assessed to have not occurred.
33. The vaso-occlusive treatment system of claim 32, wherein the first elapsed time threshold is in the range of 3-10 seconds, and the second elapsed time threshold is in the range of 25-45 seconds.
34. The vaso-occlusive treatment system of claim 32,
   wherein the delivery catheter further comprises another inner lumen extending through the elongate sheath body, and another distal port in respective communication with the other inner lumen;
   the vaso-occlusive treatment system further comprising another vaso-occlusive assembly configured for being disposed within inner lumen of the delivery catheter, the other vaso-occlusive assembly comprising a delivery wire and a vaso-occlusive device detachably coupled to the delivery wire via an electrolytically severable joint, the other vaso-occlusive device configured for being deployed out from the other distal port of the delivery catheter into the vasculature of the patient;
   wherein a proximal end of the delivery wire of the other vaso-occlusive assembly is configured for being electrically coupled to the electrolytic detachment device, and the proximal end of the delivery wire of the other vaso-occlusive assembly is configured for being electrically decoupled from the electrolytic detachment device,
   wherein the electrolytic detachment device is configured for delivering electrical current to the electrolytically severable joint of the vaso-occlusive assembly while the other vaso-occlusive device is disposed within the vasculature of the patient, such that the vaso-occlusive device electrolytically detaches from the distal end of the delivery wire; and
   wherein the delivery wire decoupling event comprises decoupling the delivery wire of the vaso-occlusive assembly from the electrolytic detachment device, and the subsequent delivery wire coupling event comprises either recoupling the delivery wire of the vaso-occlusive assembly to the electrolytic detachment device or coupling the delivery wire of the other vaso-occlusive assembly to the electrolytic detachment device.
35. The vaso-occlusive treatment system of claim 24, wherein the electrolytic detachment device is configured for generating a first user-discernible notification if the successful electrolytic detachment event has been assessed to have occurred, and for generating a second user-discernible notification different from the first user-discernible notification if the successful electrolytic detachment event has been assessed to have not occurred.
36. The vaso-occlusive treatment system of claim 24, wherein the electrical parameter information comprises measured electrolytic work performed by the electrolytic detachment device, and wherein the electrolytic detachment device is configured for assessing that the successful electrolytic detachment event has not occurred if the measured electrolytic work has not reached an electrolytic work threshold, and assessing that the successful electrolytic detachment event has occurred if the measured electrolytic work has reached the electrolytic work threshold.
37. The vaso-occlusive treatment system of claim 36, wherein the electrolytic detachment device is configured for incrementally increasing the electrolytic work threshold over time.
38. The vaso-occlusive treatment system of claim 24, wherein the electrolytic detachment device is configured for delivering the electrical current to the electrolytically severable joint during an electrolytic detachment cycle in response to a single actuation of the electrolytic detachment device.
39. The vaso-occlusive treatment system of claim 38, wherein the electrolytic detachment device is configured for delivering the electrical current to the electrolytically severable joint during another electrolytic detachment cycle in response to another single actuation of the electrolytic detachment device.
40. The vaso-occlusive treatment system of claim 38, wherein the electrolytic detachment cycle has a fixed time period, and the electrolytic detachment device is configured for assessing if the successful electrolytic detachment event has occurred during the fixed time period.
41. The vaso-occlusive treatment system of claim 40, wherein the electrolytic detachment device is further configured for extending the electrolytic detachment cycle only if the successful electrolytic detachment event has been assessed to have not occurred during the fixed time period.
42. The vaso-occlusive treatment system of claim 41, wherein the fixed time period is an initial fixed time period, wherein the electrolytic detachment device is configured for assessing if the successful electrolytic detachment event has occurred by the end of the initial fixed time period, and extending the electrolytic detachment cycle by a subsequent variable time period only if the successful electrolytic detachment event has been assessed to have not occurred during the initial fixed time period.
43. The vaso-occlusive treatment system of claim 42, wherein the electrolytic detachment device is configured for incrementally assessing if the successful electrolytic detachment event has occurred during the subsequent variable time period, and terminating the electrolytic detachment cycle when the successful electrolytic detachment event is assessed to have occurred.
44. The vaso-occlusive treatment system of claim 43, wherein the subsequent variable time period has a plurality time increments, each of which is shorter than the initial fixed time period of the electrolytic detachment cycle, and wherein the electrolytic detachment device is configured for incrementally assessing if the successful electrolytic detachment event has occurred respectively during the plurality of time increments, and terminating the electrolytic detachment cycle at the end of the time increment at which the successful electrolytic detachment event has been assessed to have occurred.
45. The vaso-occlusive treatment system of claim 41, wherein the electrolytic detachment device is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint.
46. The vaso-occlusive treatment system of claim 45, wherein the electrical parameter information comprises measured electrolytic work performed by the electrolytic detachment device, and wherein the electrolytic detachment device is configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.
47. The vaso-occlusive treatment system of claim 41, wherein the electrolytic detachment device is configured for tracking a number of completed electrical detachment cycles, wherein the electrolytic detachment device is configured for not extending the electrolytic detachment cycle if the tracked number of completed electrolytic detachment cycles exceeds a predefined limit of electrolytic detachment cycles.
48. The vaso-occlusive treatment system of claim 47, wherein the vaso-occlusive device is a vaso-occlusive coil.
49. A method of occluding a vasculature of a patient using a vaso-occlusive assembly comprising a delivery wire and a vaso-occlusive device detachably coupled to a distal end of the delivery wire via an electrolytically severable joint, comprising:
   introducing a delivery catheter configured into a vasculature of a patient,
   disposing the vaso-occlusive assembly within the delivery catheter, such that the vaso-occlusive device is disposed outside of the delivery catheter within the vasculature of the patient;
   electrically coupling a proximal end of the delivery wire of the vaso-occlusive assembly to a power source;
   delivering electrical current from the power source to the electrolytically severable joint of the vaso-occlusive assembly while the vaso-occlusive device is disposed within the vasculature of the patient;
   generating electrical parameter information indicative of a successful electrolytic detachment event;
   assessing if a successful electrolytic detachment event has occurred based on the generated electrical parameter information;
   measuring an elapsed time between a delivery wire decoupling event and a subsequent delivery wire coupling event; and
   resetting the electrical parameter information if the measured elapsed time reaches a first elapsed time threshold.
50. The method of claim 49, further comprising:
   contacting a ground electrode to the patient; and
   electrically coupling the ground electrode to the power source, such that the electrical current is delivered between the delivery wire and the ground electrode.
51. The method of claim 49, wherein the electrical parameter information comprises a measured cumulative electrical parameter.
52. The method of claim 51, wherein the cumulative electrical parameter comprises measured cumulative current.
53. The method of claim 49, wherein the electrical parameter information comprises measured electrolytic work performed by the power source.
54. The method of claim 49, wherein the first elapsed time threshold is equal to or greater than three seconds.
55. The method of claim 49, wherein the first elapsed time threshold is in the range of 25-45 seconds.
56. The method of claim 49, wherein the electrical parameter information is reset if the measured elapsed time reaches the first elapsed time threshold and the successful electrolytic detachment event has been assessed to have occurred, and the electrical parameter information is reset if the measured elapsed time reaches a second elapsed time threshold longer than the first elapsed time threshold and the successful electrolytic detachment event has been assessed to have not occurred.
57. The method of claim 56, wherein the first elapsed time threshold is in the range of 3-10 seconds, and the second elapsed time threshold is in the range of 25-45 seconds.
58. The method of claim 56, further comprising:
   disposing another vaso-occlusive assembly within the delivery catheter, such that both the vaso-occlusive device and other vaso-occlusive device are disposed outside of the delivery catheter within the vasculature of the patient, the other vaso-occlusive assembly comprising a delivery wire and a vaso-occlusive device detachably coupled to a distal end of the delivery wire via an electrolytically severable joint; and
   electrically coupling a proximal end of the delivery wire of the other vaso-occlusive assembly to the power source after assessing if the successful electrolytic detachment event has occurred; and
   resetting the electrical parameter information after the proximal end of the delivery wire of the other vaso-occlusive assembly has been electrically coupled to the power source.
59. The method of claim 49, further comprising:
   generating a first user-discernible notification if the successful electrolytic detachment event has been assessed to have occurred; and
   generating a second user-discemible notification different from the first user-discernible notification if the successful electrolytic detachment event has been assessed to have not occurred.
60. The method of claim 49, wherein the electrical parameter information comprises measured electrolytic work performed by the power source, and wherein the electrolytic detachment device is configured for assessing that the successful electrolytic detachment event has not occurred if the measured electrolytic work has not reached an electrolytic work threshold, and assessing that the successful electrolytic detachment event has occurred if the measured electrolytic work has reached the electrolytic work threshold.
61. The method of claim 60, further comprising incrementally increasing the electrolytic work threshold over time.
62. The method of claim 49, wherein the electrical current is delivered to the electrolytically severable joint during an electrolytic detachment cycle.
63. The method of claim 62, wherein the electrical current is delivered to the electrolytically severable joint during another electrolytic detachment cycle.
64. The method of claim 62, wherein the electrolytic detachment cycle has a fixed time period, and wherein the occurrence of the successful electrolytic detachment device during the fixed time period is assessed.
65. The method of claim 64, further comprising extending the electrolytic detachment cycle only if the successful electrolytic detachment event has been assessed to have not occurred during the fixed time period.
66. The method of claim 65, wherein the fixed time period is an initial fixed time period, wherein the occurrence of the successful electrolytic detachment event by the end of the initial fixed time period is assessed, wherein the electrolytic detachment cycle is extended by a subsequent variable time period only if the successful electrolytic detachment event has been assessed to have not occurred during the initial fixed time period.
67. The method of claim 66, further comprising:
   incrementally assessing if the successful electrolytic detachment event has occurred during the subsequent variable time period; and
   terminating the electrolytic detachment cycle when the successful electrolytic detachment event is assessed to have occurred.
68. The method of claim 67, wherein the subsequent variable time period has a plurality time increments, each of which is shorter than the initial fixed time period of the electrolytic detachment cycle, and wherein if the successful electrolytic detachment event is incrementally assessed to have occurred respectively during the plurality of time increments, and terminating the electrolytic detachment cycle at the end of the time increment at which the successful electrolytic detachment event has been assessed to have occurred.
69. The method of claim 65, further comprising determining if an electrolytic fault has occurred during the delivery of the electrical current to the electrolytically severable joint, wherein the electrolytic detachment cycle is extended only if the electrolytic fault has not been determined to have occurred during the delivery of the electrical current to the electrolytically severable joint.
70. The method of claim 69, wherein the electrical parameter information comprises measured electrolytic work performed by the power source, and the electrolytic fault is determined to have occurred during the delivery of the electrical current to the electrolytically severable joint if the measured electrolytic work is outside of an electrolytic work range defined by a minimum electrolytic work limit and a maximum electrolytic work limit.
71. The method of claim 65, further comprising tracking a number of completed electrical detachment cycles, wherein the electrolytic detachment cycle is not extended if the tracked number of extended electrolytic detachment cycles exceeds a predefined limit of electrolytic detachment cycles.
72. The method of claim 49, wherein the vaso-occlusive device is a vaso-occlusive coil.
73. The method of claim 49, wherein the vaso-occlusive device is disposed within an aneurysmal sac in the vasculature of the patient.
74. An electrolytic detachment device for use with an electrically conductive delivery wire having a distal end to which a respective vaso-occlusive device is attached and an electrolytically severable joint located proximal to the vaso-occlusive device, comprising:
   a power terminal to which a proximal end of each of the delivery wires is configured for being electrically coupled;
   electrical current delivery circuitry configured for delivering electrical current to the electrolytically severable joint of the vaso-occlusive assembly when disposed within the vasculature of the patient, such that the vaso-occlusive device electrolytically detaches from the distal end of the delivery wire;
   an electrolytic detachment detection circuit configured for generating electrical parameter information indictive of an electrolytic detachment event; and
   a controller configured for operating the electrical current delivery circuitry to deliver the electrical current to the electrolytically severable joint during an electrolytic detachment cycle having a fixed time period, assessing if a successful electrolytic detachment event has occurred during the fixed time period based on the generated electrical parameter information, and extending the electrolytic detachment cycle only if the successful electrolytic detachment event has been assessed to have not occurred during the fixed time period.
75. The electrolytic detachment device of claim 74, further comprising a ground terminal to which a ground electrode in electrical contact with the patient is configured for being electrically coupled via an electrical cable.
76. The electrolytic detachment device of claim 74, wherein the power terminal is a power port into which the proximal end of the delivery wire is configured for being inserted.
77. The electrolytic detachment device of claim 74, wherein the electrical parameter information comprises a measured cumulative electrical parameter.
78. The electrolytic detachment device of claim 77, wherein the cumulative electrical parameter comprises measured cumulative current.
79. The electrolytic detachment device of claim 74, wherein the electrical parameter information comprises measured electrolytic work performed by the electrical current delivery circuitry.
80. The electrolytic detachment device of claim 74, wherein the fixed time period is an initial fixed time period, and wherein the controller is configured for extending the electrolytic detachment cycle by a subsequent variable time period.
81. The electrolytic detachment device of claim 80, wherein the controller is configured for incrementally assessing if the successful electrolytic detachment event has occurred during the subsequent variable time period, and terminating the electrolytic detachment cycle when the successful electrolytic detachment event is assessed to have occurred.
82. The electrolytic detachment device of claim 81, wherein the subsequent variable time period has a plurality time increments, each of which is shorter than the initial fixed time period of the electrolytic detachment cycle, and wherein the controller is configured for incrementally assessing that if the successful electrolytic detachment event has occurred respectively during the plurality of time increments, and terminating the electrolytic detachment cycle at the end of the time increment at which the successful electrolytic detachment event has been assessed to have occurred.
83. The electrolytic detachment device of claim 74, wherein the controller is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint.
84. The electrolytic detachment device of claim 83, wherein the electrical parameter information comprises measured electrolytic work performed by the electrical current delivery circuitry, and wherein the controller is configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.
85. The electrolytic detachment device of claim 74, further comprising an electrical current delivery actuator, wherein the controller is configured for operating the electrical current delivery circuitry to deliver the electrical current to the electrolytically severable joint during the electrolytic detachment cycle in response to a single actuation of the electrical current delivery actuator.
86. The electrolytic detachment device of claim 85, wherein the controller is configured for operating the electrical current delivery circuitry to deliver the electrical current to the electrolytically severable joint during another electrolytic detachment cycle having an initial fixed time period in response to another single actuation of the electrical current delivery actuator.
87. The electrolytic detachment device of claim 74, further comprising a counter configured for tracking a number of completed electrical detachment cycles, wherein the controller is configured for not extending the electrolytic detachment cycle if the tracked number of completed electrolytic detachment cycles exceeds a predefined number of electrolytic detachment cycles.
88. The electrolytic detachment device of claim 74, further comprising a timer configured for measuring an elapsed time between a delivery wire decoupling event and a subsequent delivery wire coupling event, wherein the controller is configured for resetting the electrical parameter information if the measured elapsed time reaches a first elapsed time threshold.
89. The electrolytic detachment device of claim 88, wherein the first elapsed time threshold is equal to or greater than three seconds.
90. The electrolytic detachment device of claim 88, wherein the first elapsed time threshold is in the range of 25-45 seconds.
91. The electrolytic detachment device of claim 88,
   wherein the controller is configured for resetting the electrical parameter information if the measured elapsed time reaches the first elapsed time threshold and the successful electrolytic detachment event has been assessed to have occurred; and
   wherein the controller is configured for resetting the electrical parameter information if the measured elapsed time reaches a second elapsed time threshold longer than the first elapsed time threshold and the successful electrolytic detachment event has been assessed to have not occurred.
92. The electrolytic detachment device of claim 91, wherein the first elapsed time threshold is in the range of 3-10 seconds, and the second elapsed time threshold is in the range of 25-45 seconds.
93. The electrolytic detachment device of claim 74, wherein:
   the controller is configured for generating a first user-discernible notification if the successful electrolytic detachment event has been assessed to have occurred; and
   the controller is configured for generating a second user-discernible notification different from the first user-discernible notification if the successful electrolytic detachment event has been assessed to have not occurred.
94. The electrolytic detachment device of claim 74, wherein the electrical parameter information comprises measured electrolytic work performed by the electrical current delivery circuitry, and wherein the controller is further configured for assessing that the successful electrolytic detachment event to has not occurred during the fixed time period of the electrolytic detachment cycle if the measured electrolytic work has not reached an electrolytic work threshold, and assessing that the successful electrolytic detachment event has occurred during the fixed time period of the electrolytic detachment cycle if the measured electrolytic work has reached the electrolytic work threshold.
95. The electrolytic detachment device of claim 94, wherein the controller is configured for incrementally increasing the electrolytic work threshold over the extended electrolytic detachment cycle.
96. A vaso-occlusive treatment system, comprising:
   a delivery catheter configured for being introduced into a vasculature of a patient, the delivery catheter comprising an elongate sheath body, an inner lumen extending through the elongate sheath body, and a distal port in respective communication with the inner lumen;
   a vaso-occlusive assembly configured for being disposed within inner lumen of the delivery catheter, the vaso-occlusive assembly comprising a delivery wire and a vaso-occlusive device detachably coupled to the delivery wire via an electrolytically severable joint, the vaso-occlusive device configured for being deployed out from the distal port of the delivery catheter into the vasculature of the patient; and
   an electrolytic detachment device to which a proximal end of the delivery wire of the vaso-occlusive assembly is configured for being electrically coupled, and from which the proximal end of the delivery wire of the vaso-occlusive assembly is configured for being electrically decoupled, delivering electrical current, during an electrolytic detachment cycle having a fixed time period, to the electrolytically severable joint of the vaso-occlusive assembly while the vaso-occlusive device is disposed within the vasculature of the patient, such that the vaso-occlusive device electrolytically detaches from the distal end of the delivery wire, generating electrical parameter information indicative of a successful electrolytic detachment event, assessing if the successful electrolytic detachment event has occurred during the fixed time period based on the generated electrical parameter information, and extending the electrolytic detachment cycle only if the successful electrolytic detachment event has been assessed to have not occurred during the fixed time period.
97. The vaso-occlusive treatment system of claim 96, further comprising a ground electrode configured for being placed in electrical contact with the patient and for being electrically coupled to the electrolytic detachment device via an electrical cable.
98. The vaso-occlusive treatment system of claim 96, wherein the electrolytic detachment device is a handheld electrolytic detachment device.
99. The vaso-occlusive treatment system of claim 96, wherein the electrical parameter information comprises a measured cumulative electrical parameter.
100. The vaso-occlusive treatment system of claim 99, wherein the cumulative electrical parameter comprises measured cumulative current.
101. The vaso-occlusive treatment system of claim 96, wherein the electrical parameter information comprises measured electrolytic work performed by the electrolytic detachment device.
102. The vaso-occlusive treatment system of claim 96, wherein the fixed time period is an initial fixed time period, and wherein the electrolytic detachment device is configured for extending the electrolytic detachment cycle by a subsequent variable time period.
103. The vaso-occlusive treatment system of claim 102, wherein the electrolytic detachment device is configured for incrementally assessing if the successful electrolytic detachment event has occurred during the subsequent variable time period, and terminating the electrolytic detachment cycle when the successful electrolytic detachment event is assessed to have occurred.
104. The vaso-occlusive treatment system of claim 103, wherein the subsequent variable time period has a plurality time increments, each of which is shorter than the initial fixed time period of the electrolytic detachment cycle, and wherein the electrolytic detachment device is configured for incrementally assessing that if the successful electrolytic detachment event has occurred respectively during the plurality of time increments, and terminating the electrolytic detachment cycle at the end of the time increment at which the successful electrolytic detachment event has been assessed to have occurred.
105. The vaso-occlusive treatment system of claim 96, wherein the electrolytic detachment device is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint.
106. The vaso-occlusive treatment system of claim 105, wherein the electrical parameter information comprises measured electrolytic work performed by the electrolytic detachment device, wherein the electrolytic detachment device is configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.
107. The vaso-occlusive treatment system of claim 96, wherein the electrolytic detachment device is configured for delivering the electrical current to the electrolytically severable joint during the electrolytic detachment cycle in response to a single actuation of the electrolytic detachment device.
108. The vaso-occlusive treatment system of claim 107, wherein the electrolytic detachment device is configured for delivering the electrical current to the electrolytically severable joint during another electrolytic detachment cycle having an initial fixed time period in response to another single actuation of the electrolytic detachment device.
109. The vaso-occlusive treatment system of claim 96, wherein the electrolytic detachment device is configured for tracking a number of completed electrical detachment cycles, and not extending the electrolytic detachment cycle if the tracked number of completed electrolytic detachment cycles exceeds a predefined limit of electrolytic detachment cycles.
110. The vaso-occlusive treatment system of claim 96, wherein the electrolytic detachment device is configured for measuring an elapsed time between a delivery wire decoupling event and a subsequent delivery wire coupling event, and for resetting the electrical parameter information if the measured elapsed time reaches a first elapsed time threshold.
111. The vaso-occlusive treatment system of claim 110, wherein the first elapsed time threshold is equal to or greater than three seconds.
112. The vaso-occlusive treatment system of claim 110, wherein the first elapsed time threshold is in the range of 25-45 seconds.
113. The vaso-occlusive treatment system of claim 110,
   wherein the controller is configured for resetting the electrical parameter information if the measured elapsed time reaches the first elapsed time threshold and the successful electrolytic detachment event has been assessed to have occurred; and
   wherein the controller is configured for resetting the electrical parameter information if the measured elapsed time reaches a second elapsed time threshold longer than the first elapsed time threshold and the successful electrolytic detachment event has been assessed to have not occurred.
114. The vaso-occlusive treatment system of claim 113, wherein the first elapsed time threshold is in the range of 3-10 seconds, and the second elapsed time threshold is in the range of 25-45 seconds.
115. The vaso-occlusive treatment system of claim 113,
   wherein the delivery catheter further comprises another inner lumen extending through the elongate sheath body, and another distal port in respective communication with the other inner lumen;
   the vaso-occlusive treatment system further comprising another vaso-occlusive assembly configured for being disposed within inner lumen of the delivery catheter, the other vaso-occlusive assembly comprising a delivery wire and a vaso-occlusive device detachably coupled to the delivery wire via an electrolytically severable joint, the other vaso-occlusive device configured for being deployed out from the other distal port of the delivery catheter into the vasculature of the patient;
   wherein a proximal end of the delivery wire of the other vaso-occlusive assembly is configured for being electrically coupled to the electrolytic detachment device, and the proximal end of the delivery wire of the other vaso-occlusive assembly is configured for being electrically decoupled from the electrolytic detachment device, delivering electrical current to the electrolytically severable joint of the vaso-occlusive assembly while the other vaso-occlusive device is disposed within the vasculature of the patient, such that the other vaso-occlusive device electrolytically detaches from the distal end of the delivery wire; and
   wherein the delivery wire decoupling event comprises decoupling the delivery wire of the vaso-occlusive assembly from the electrolytic detachment device, and the subsequent delivery wire coupling event comprises either recoupling the delivery wire of the vaso-occlusive assembly to the electrolytic detachment device or coupling the delivery wire of the other vaso-occlusive assembly to the electrolytic detachment device.
116. The vaso-occlusive treatment system of claim 96, wherein the electrolytic detachment device is configured for generating a first user-discernible notification if the successful electrolytic detachment event has been assessed to have occurred, and for generating a second user-discernible notification different from the first user-discernible notification if the successful electrolytic detachment event has been assessed to have not occurred.
117. The vaso-occlusive treatment system of claim 96, wherein the electrical parameter information comprises measured electrolytic work performed by the electrolytic detachment device, and wherein the electrolytic detachment device is further configured for assessing that the successful electrolytic detachment event has not occurred during the fixed time period of the electrolytic detachment cycle if the measured electrolytic work has not reached an electrolytic work threshold, and assessing that the successful electrolytic detachment event has occurred during the fixed time period of the electrolytic detachment cycle if the measured electrolytic work has reached the electrolytic work threshold.
118. The vaso-occlusive treatment system of claim 117, wherein the electrolytic detachment device is configured for incrementally increasing the electrolytic work threshold over the extended electrolytic detachment cycle.
119. The vaso-occlusive treatment system of claim 96, wherein the vaso-occlusive device is a vaso-occlusive coil.
120. A method of occluding a vasculature of a patient using a vaso-occlusive assembly comprising a delivery wire and a vaso-occlusive device detachably coupled to a distal end of the delivery wire via an electrolytically severable joint, comprising:
   introducing a delivery catheter configured into a vasculature of a patient,
   disposing the vaso-occlusive assembly within the delivery catheter, such that the vaso-occlusive device is disposed outside of the delivery catheter within the vasculature of the patient;
   electrically coupling a proximal end of the delivery wire of the vaso-occlusive assembly to a power source;
   delivering electrical current, during an electrolytic detachment cycle having a fixed time period, from the power source to the electrolytically severable joint of the vaso-occlusive assembly while the vaso-occlusive device is disposed within the vasculature of the patient;
   generating electrical parameter information indicative of a successful electrolytic detachment event;
   assessing if the successful electrolytic detachment event has occurred during the fixed time period of the electrolytic detachment cycle based on the generated electrical parameter information; and
   extending the electrolytic detachment cycle only if the successful electrolytic detachment event has been assessed to have not occurred during the fixed time period.
121. The method of claim 120, further comprising:
   contacting a ground electrode to the patient; and
   electrically coupling the ground electrode to the power source, such that the electrical current is delivered between the delivery wire and the ground electrode.
122. The method of claim 120, wherein the electrical parameter information comprises a measured cumulative electrical parameter.
123. The method of claim 122, wherein the cumulative electrical parameter comprises measured cumulative current.
124. The method of claim 120, wherein the electrical parameter information comprises measured electrolytic work performed by the power source.
125. The method of claim 120, wherein the fixed time period is an initial fixed time period, and wherein the electrolytic detachment cycle is extended by a subsequent variable time period.
126. The method of claim 125, further comprising:
   incrementally assessing if the successful electrolytic detachment event has occurred during the subsequent variable time period; and
   terminating the electrolytic detachment cycle when the successful electrolytic detachment event is assessed to have occurred.
127. The method of claim 126, wherein the subsequent variable time period has a plurality time increments, each of which is shorter than the initial fixed time period of the electrolytic detachment cycle, and wherein if the successful electrolytic detachment event has occurred is incrementally assessed respectively during the plurality of time increments, and terminating the electrolytic detachment cycle at the end of the time increment at which the successful electrolytic detachment event has been assessed to have occurred.
128. The method of claim 125, further comprising determining if an electrolytic fault has occurred during the delivery of the electrical current to the electrolytically severable joint, wherein the electrolytic detachment cycle is extended only if the electrolytic fault has not been determined to have occurred during the delivery of the electrical current to the electrolytically severable joint.
129. The method of claim 128, wherein the electrical parameter information comprises measured electrolytic work performed by the power source, and the electrolytic fault is determined to have occurred during the delivery of the electrical current to the electrolytically severable joint if the measured electrolytic work is outside of an electrolytic work range defined by a minimum electrolytic work limit and a maximum electrolytic work limit.
130. The method of claim 120, further comprising delivering electrical current, during another electrolytic detachment cycle having a fixed time period, to the electrolytically severable joint of the vaso-occlusive assembly while the vaso-occlusive device is disposed within the vasculature of the patient.
131. The method of claim 120, further comprising tracking a number of completed electrical detachment cycles, wherein the electrolytic detachment cycle is not extended if the tracked number of completed electrolytic detachment cycles exceeds a predefined limit of electrolytic detachment cycles.
132. The method of claim 120, further comprising:
   measuring an elapsed time between a delivery wire decoupling event and a subsequent delivery wire coupling event; and
   resetting the electrical parameter information if the measured elapsed time reaches a first elapsed time threshold.
133. The method of claim 132, wherein the first elapsed time threshold is equal to or greater than three seconds.
134. The method of claim 132, wherein the first elapsed time threshold is in the range of 25-45 seconds.
135. The method of claim 132, wherein the electrical parameter information is reset if the measured elapsed time reaches the first elapsed time threshold and the successful electrolytic detachment event has been assessed to have occurred, and the electrical parameter information is reset if the measured elapsed time reaches a second elapsed time threshold longer than the first elapsed time threshold and the successful electrolytic detachment event has been assessed to have not occurred.
136. The method of claim 135, wherein the first elapsed time threshold is in the range of 3-10 seconds, and the second elapsed time threshold is in the range of 25-45 seconds.
137. The method of claim 135, further comprising:
   disposing another vaso-occlusive assembly within the delivery catheter, such that both the vaso-occlusive device and other vaso-occlusive device are disposed outside of the delivery catheter within the vasculature of the patient, the other vaso-occlusive assembly comprising a delivery wire and a vaso-occlusive device detachably coupled to a distal end of the delivery wire via an electrolytically severable joint; and
   electrically coupling a proximal end of the delivery wire of the other vaso-occlusive assembly to the power source after assessing if the successful electrolytic detachment event has occurred; and
   resetting the electrical parameter information after the proximal end of the delivery wire of the other vaso-occlusive assembly has been electrically coupled to the power source.
138. The method of claim 120, further comprising:
   generating a first user-discernible notification if the successful electrolytic detachment event has been assessed to have occurred; and
   generating a second user-discernible notification different from the first user-discernible notification if the successful electrolytic detachment event has been assessed to have not occurred.
139. The method of claim 120, wherein the electrical parameter information comprises measured electrolytic work performed by the electrolytic detachment device, and wherein the successful electrolytic detachment event is assessed to have not occurred during the fixed time period of the electrolytic detachment cycle if the measured electrolytic work has not reached an electrolytic work threshold, and assessed to have occurred to have occurred during the fixed time period of the electrolytic detachment cycle if the measured electrolytic work has reached the electrolytic work threshold.
140. The method of claim 120, further comprising incrementally increasing the electrolytic work threshold over the extended electrolytic detachment cycle.
141. The method of claim 120, wherein the vaso-occlusive device is a vaso-occlusive coil.
142. The method of claim 120, wherein the vaso-occlusive device is disposed within an aneurysmal sac in the vasculature of the patient.
143. An electrolytic detachment device for use with an electrically conductive delivery wire having a distal end to which a respective vaso-occlusive device is attached and an electrolytically severable joint located proximal to the vaso-occlusive device, comprising:
   a power terminal to which a proximal end of each of the delivery wires is configured for being electrically coupled;
   electrical current delivery circuitry configured for delivering electrical current to the electrolytically severable joint of the vaso-occlusive assembly when disposed within the vasculature of the patient, such that the vaso-occlusive device electrolytically detaches from the distal end of the delivery wire;
   an electrolytic detachment detection circuit configured for incrementally measuring electrolytic work performed by the electrical current delivery circuitry over time, thereby generating a plurality of measured electrolytic work values; and
   a controller configured for operating the electrical current delivery circuitry to deliver the electrical current to the electrolytically severable joint, incrementally increasing an electrolytic work threshold over time, thereby generating a plurality of electrolytic work threshold values, respectively comparing the plurality of measured electrolytic work values to the plurality of electrolytic work threshold values, and assessing if a successful electrolytic detachment event has occurred based on the comparisons.
144. The electrolytic detachment device of claim 143, further comprising a ground terminal to which a ground electrode in electrical contact with the patient is configured for being electrically coupled via an electrical cable.
145. The electrolytic detachment device of claim 143, wherein the power terminal is a power port into which the proximal end of the delivery wire is configured for being inserted.
146. The electrolytic detachment device of claim 143, wherein the electrolytic detachment detection circuit is configured for incrementally measuring the electrolytic work performed by the electrical current delivery circuitry over time by incrementally measuring cumulative current delivered by the electrical current delivery circuitry over time.
147. The electrolytic detachment device of claim 143, further comprising an electrical current delivery actuator, wherein the controller is configured for operating the electrical current delivery circuitry to deliver the electrical current to the electrolytically severable joint during an electrolytic detachment cycle in response to a single actuation of the electrical current delivery actuator.
148. The electrolytic detachment device of claim 147, wherein the controller is configured for operating the electrical current delivery circuitry to deliver the electrical current to the electrolytically severable joint during another electrolytic detachment cycle in response to another single actuation of the electrical current delivery actuator.
149. The electrolytic detachment device of claim 147, wherein the electrolytic detachment cycle has an initial fixed time period, and the controller is configured for assessing if the successful electrolytic detachment event has occurred during the initial fixed time period based on at least one of the comparisons.
150. The electrolytic detachment device of claim 149, wherein the controller is configured extending the electrolytic detachment cycle only if the successful electrolytic detachment event has been assessed to have not occurred during the initial fixed time period, and assessing if the successful electrolytic detachment event has occurred after the initial fixed time period based on at least another one of the comparisons.
151. The electrolytic detachment device of claim 150, wherein the initial fixed time period is an initial fixed time period, wherein the controller is configured for assessing if the successful electrolytic detachment event has occurred during the initial fixed time period, and extending the electrolytic detachment cycle by a subsequent variable time period only if the successful electrolytic detachment event has been assessed to have not occurred during the initial fixed time period, wherein the controller is configured for assessing if the successful electrolytic detachment event has occurred during the subsequent variable time period based on the at least one other one of the comparisons.
152. The electrolytic detachment device of claim 151, wherein the at least one other one of the comparisons comprises an incremental number of comparisons, and wherein the controller is configured for incrementally assessing if the successful electrolytic detachment event has occurred during the subsequent variable time period based on the incremental number of comparisons, and terminating the electrolytic detachment cycle when the successful electrolytic detachment event is assessed to have occurred.
153. The electrolytic detachment device of claim 152, wherein the subsequent variable time period has a plurality time increments, each of which is shorter than the initial fixed time period of the electrolytic detachment cycle, and wherein the controller is configured for incrementally assessing if the successful electrolytic detachment event has occurred respectively during the plurality of time increments respectively based on the incremental number of comparisons, and terminating the electrolytic detachment cycle at the end of the time increment at which the successful electrolytic detachment event has been assessed to have occurred.
154. The electrolytic detachment device of claim 150, wherein the controller is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint.
155. The electrolytic detachment device of claim 154, wherein the controller is configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.
156. The electrolytic detachment device of claim 150, further comprising a counter configured for tracking a number of completed electrical detachment cycles, wherein the controller is configured for not extending the electrolytic detachment cycle if the tracked number of completed electrolytic detachment cycles exceeds a predefined number of electrolytic detachment cycles.
157. The electrolytic detachment device of claim 143, further comprising a timer configured for measuring an elapsed time between a delivery wire decoupling event and a subsequent delivery wire coupling event, wherein the controller is configured for resetting the incrementally measured electrolytic work and the incrementally increased electrolytic work threshold if the measured elapsed time reaches a first elapsed time threshold.
158. The electrolytic detachment device of claim 157, wherein the first elapsed time threshold is equal to or greater than three seconds.
159. The electrolytic detachment device of claim 157, wherein the first elapsed time threshold is in the range of 25-45 seconds.
160. The electrolytic detachment device of claim 157,
   wherein the controller is configured for resetting the incrementally measured electrolytic work and the incrementally increased electrolytic work threshold if the measured elapsed time reaches the first elapsed time threshold and the successful electrolytic detachment event has been assessed to have occurred; and
   wherein the controller is configured for resetting the incrementally measured electrolytic work and the incrementally increased electrolytic work threshold if the measured elapsed time reaches a second elapsed time threshold longer than the first elapsed time threshold and the successful electrolytic detachment event has been assessed to have not occurred.
161. The electrolytic detachment device of claim 160, wherein the first elapsed time threshold is in the range of 3-10 seconds, and the second elapsed time threshold is in the range of 25-45 seconds.
162. The electrolytic detachment device of claim 143, wherein:
   the controller is configured for generating a first user-discernible notification if the successful electrolytic detachment event has been assessed to have occurred; and
   the controller is configured for generating a second user-discernible notification different from the first user-discernible notification if the successful electrolytic detachment event has been assessed to have not occurred.
163. A vaso-occlusive treatment system, comprising:
   a delivery catheter configured for being introduced into a vasculature of a patient, the delivery catheter comprising an elongate sheath body, an inner lumen extending through the elongate sheath body, and a distal port in respective communication with the inner lumen;
   a vaso-occlusive assembly configured for being disposed within inner lumen of the delivery catheter, the vaso-occlusive assembly comprising a delivery wire and a vaso-occlusive device detachably coupled to the delivery wire via an electrolytically severable joint, the vaso-occlusive device configured for being deployed out from the distal port of the delivery catheter into the vasculature of the patient; and
   an electrolytic detachment device to which a proximal end of the delivery wire of the vaso-occlusive assembly is configured for being electrically coupled, and from which the proximal end of the delivery wire of the vaso-occlusive assembly is configured for being electrically decoupled, delivering electrical current to the electrolytically severable joint of the vaso-occlusive assembly while the vaso-occlusive device is disposed within the vasculature of the patient, such that the vaso-occlusive device electrolytically detaches from the distal end of the delivery wire, incrementally measuring electrolytic work performed by the electrolytic detachment device over time, thereby generating a plurality of measured electrolytic work values, incrementally increasing an electrolytic work threshold over time, thereby generating a plurality of electrolytic work threshold values, respectively comparing the plurality of measured electrolytic work values to the plurality of electrolytic work threshold values, and assessing if a successful electrolytic detachment event has occurred based on the comparisons.
164. The vaso-occlusive treatment system of claim 163, further comprising a ground electrode configured for being placed in electrical contact with the patient and for being electrically coupled to the electrolytic detachment device via an electrical cable.
165. The vaso-occlusive treatment system of claim 163, wherein the electrolytic detachment device is a handheld electrolytic detachment device.
166. The vaso-occlusive treatment system of claim 163, wherein the electrolytic detachment device is configured for incrementally measuring the electrolytic work performed by the electrolytic detachment device over time by incrementally measuring cumulative current delivered by the electrolytic detachment device over time.
167. The vaso-occlusive treatment system of claim 163, wherein the electrolytic detachment device is configured for delivering the electrical current to the electrolytically severable joint during an electrolytic detachment cycle in response to a single actuation of the electrolytic detachment device.
168. The vaso-occlusive treatment system of claim 167, wherein the electrolytic detachment device is configured for delivering the electrical current to the electrolytically severable joint during another electrolytic detachment cycle in response to another single actuation of the electrolytic detachment device.
169. The vaso-occlusive treatment system of claim 167, wherein the electrolytic detachment cycle has an initial fixed time period, and the electrolytic detachment device is configured for assessing if the successful electrolytic detachment event has occurred during the initial fixed time period based on at least one of the comparisons.
170. The vaso-occlusive treatment system of claim 169, wherein the electrolytic detachment device is configured extending the electrolytic detachment cycle only if the successful electrolytic detachment event has been assessed to have not occurred during the initial fixed time period, and assessing if the successful electrolytic detachment event has occurred after the initial fixed time period based on at least another one of the comparisons.
171. The vaso-occlusive treatment system of claim 170, wherein the initial fixed time period is an initial fixed time period, wherein the electrolytic detachment device is configured for assessing if the successful electrolytic detachment event has occurred during the initial fixed time period, and extending the electrolytic detachment cycle by a subsequent variable time period only if the successful electrolytic detachment event has been assessed to have not occurred during the initial fixed time period, wherein the electrolytic detachment device is configured for assessing if the successful electrolytic detachment event has occurred during the subsequent variable time period based on the at least one other one of the comparisons.
172. The vaso-occlusive treatment system of claim 171, wherein the at least one other one of the comparisons comprises an incremental number of comparisons, and wherein the electrolytic detachment device is configured for incrementally assessing if the successful electrolytic detachment event has occurred during the subsequent variable time period based on the incremental number of comparisons, and terminating the electrolytic detachment cycle when the successful electrolytic detachment event is assessed to have occurred.
173. The vaso-occlusive treatment system of claim 172, wherein the subsequent variable time period has a plurality time increments, each of which is shorter than the initial fixed time period of the electrolytic detachment cycle, and wherein the electrolytic detachment device is configured for incrementally assessing if the successful electrolytic detachment event has occurred respectively during the plurality of time increments respectively based on the incremental number of comparisons, and terminating the electrolytic detachment cycle at the end of the time increment at which the successful electrolytic detachment event has been assessed to have occurred.
174. The vaso-occlusive treatment system of claim 170, wherein the electrolytic detachment device is configured for determining if an electrolytic fault has occurred during the delivery of the electrical current to the electrolytically severable joint, and extending the electrolytic detachment cycle only if the electrolytic fault has not been determined to have occurred during the delivery of the electrical current to the electrolytically severable joint.
175. The vaso-occlusive treatment system of claim 174, wherein the electrolytic detachment device is configured for determining that the electrolytic fault has occurred during the delivery of the electrical current to the electrolytically severable joint if the measured electrolytic work is outside of an electrolytic work range defined by a minimum electrolytic work limit and a maximum electrolytic work limit.
176. The vaso-occlusive treatment system of claim 170, wherein the electrolytic detachment device is configured for tracking a number of completed electrical detachment cycles, and for not extending the electrolytic detachment cycle if the tracked number of completed electrolytic detachment cycles exceeds a predefined limit of electrolytic detachment cycles.
177. The vaso-occlusive treatment system of claim 163, wherein the electrolytic detachment device is configured for measuring an elapsed time between a delivery wire decoupling event and a subsequent delivery wire coupling event, and for resetting the incrementally measured electrolytic work and the incrementally increased electrolytic work threshold if the measured elapsed time reaches a first elapsed time threshold.
178. The vaso-occlusive treatment system of claim 177, wherein the first elapsed time threshold is equal to or greater than three seconds.
179. The vaso-occlusive treatment system of claim 177, wherein the first elapsed time threshold is in the range of 25-45 seconds.
180. The vaso-occlusive treatment system of claim 177,
   wherein the electrolytic detachment device is configured for resetting the incrementally measured electrolytic work and the incrementally increased electrolytic work threshold if the measured elapsed time reaches the first elapsed time threshold and the successful electrolytic detachment event has been assessed to have occurred; and
   wherein the electrolytic detachment device is configured for resetting the incrementally measured electrolytic work and the incrementally increased electrolytic work threshold if the measured elapsed time reaches a second elapsed time threshold longer than the first elapsed time threshold and the successful electrolytic detachment event has been assessed to have not occurred.
181. The vaso-occlusive treatment system of claim 180, wherein the first elapsed time threshold is in the range of 3-10 seconds, and the second elapsed time threshold is in the range of 25-45 seconds.
182. The vaso-occlusive treatment system of claim 180,
   wherein the delivery catheter further comprises another inner lumen extending through the elongate sheath body, and another distal port in respective communication with the other inner lumen;
   the vaso-occlusive treatment system further comprising another vaso-occlusive assembly configured for being disposed within inner lumen of the delivery catheter, the other vaso-occlusive assembly comprising a delivery wire and a vaso-occlusive device detachably coupled to the delivery wire via an electrolytically severable joint, the other vaso-occlusive device configured for being deployed out from the other distal port of the delivery catheter into the vasculature of the patient;
   wherein a proximal end of the delivery wire of the other vaso-occlusive assembly is configured for being electrically coupled to the electrolytic detachment device, and the proximal end of the delivery wire of the other vaso-occlusive assembly is configured for being electrically decoupled from the electrolytic detachment device, delivering electrical current to the electrolytically severable joint of the vaso-occlusive assembly while the other vaso-occlusive device is disposed within the vasculature of the patient, such that the other vaso-occlusive device electrolytically detaches from the distal end of the delivery wire; and
   wherein the delivery wire decoupling event comprises decoupling the delivery wire of the vaso-occlusive assembly from the electrolytic detachment device, and the subsequent delivery wire coupling event comprises either recoupling the delivery wire of the vaso-occlusive assembly to the electrolytic detachment device or coupling the delivery wire of the other vaso-occlusive assembly to the electrolytic detachment device.
183. The vaso-occlusive treatment system of claim 163, wherein:
   the electrolytic detachment device is configured for generating a first user-discernible notification if the successful electrolytic detachment event has been assessed to have occurred; and
   the electrolytic detachment device is configured for generating a second user-discernible notification different from the first user-discernible notification if the successful electrolytic detachment event has been assessed to have not occurred.
184. A method of occluding a vasculature of a patient using a vaso-occlusive assembly comprising a delivery wire and a vaso-occlusive device detachably coupled to a distal end of the delivery wire via an electrolytically severable joint, comprising:
   introducing a delivery catheter configured into a vasculature of a patient,
   disposing the vaso-occlusive assembly within the delivery catheter, such that the vaso-occlusive device is disposed outside of the delivery catheter within the vasculature of the patient;
   electrically coupling a proximal end of the delivery wire of the vaso-occlusive assembly to a power source;
   delivering electrical current from the power source to the electrolytically severable joint of the vaso-occlusive assembly while the vaso-occlusive device is disposed within the vasculature of the patient;
   incrementally measuring electrolytic work performed by the power source over time, thereby generating a plurality of measured electrolytic work values;
   incrementally increasing an electrolytic work threshold over time, thereby generating a plurality of electrolytic work threshold values;
   respectively comparing the plurality of measured electrolytic work values to the plurality of electrolytic work threshold values; and
   assessing if a successful electrolytic detachment event has occurred based on the comparisons.
185. The method of claim 184, further comprising:
   contacting a ground electrode to the patient; and
   electrically coupling the ground electrode to the power source, such that the electrical current is delivered between the delivery wire and the ground electrode.
186. The method of claim 184, wherein the electrolytic work is incrementally measured over time by incrementally measuring cumulative current delivered by the power source over time.
187. The method of claim 184, wherein the electrical current is delivered from the power source to the electrolytically severable joint during an electrolytic detachment cycle.
188. The method of claim 187, wherein the electrical current is delivered from the power source to the electrolytically severable joint during another electrolytic detachment cycle.
189. The method of claim 187, wherein the electrolytic detachment cycle has a fixed time period, and wherein the occurrence of the successful electrolytic detachment device during the fixed time period is assessed based on at least one of the comparisons.
190. The method of claim 189, wherein the electrolytic detachment cycle is extended only if the successful electrolytic detachment event has been assessed to have not occurred during the fixed time period, the method further comprising assessing if the successful electrolytic detachment event has occurred after the fixed time period based on at least another one of the comparisons.
191. The method of claim 190, wherein the fixed time period is an initial fixed time period, wherein the occurrence of the successful electrolytic detachment event by the end of the initial fixed time period is assessed, and wherein the electrolytic detachment cycle is extended by a subsequent variable time period only if the successful electrolytic detachment event has been assessed to have not occurred during the initial fixed time period.
192. The method of claim 191, wherein the at least one other one of the comparisons comprises an incremental number of comparisons, the method further comprising incrementally assessing if the successful electrolytic detachment event has occurred during the subsequent variable time period based on the incremental number of comparisons, and terminating the electrolytic detachment cycle when the successful electrolytic detachment event is assessed to have occurred.
193. The method of claim 192, wherein the subsequent variable time period has a plurality time increments, each of which is shorter than the initial fixed time period of the electrolytic detachment cycle, and wherein the electrolytic detachment device is configured for incrementally assessing if the successful electrolytic detachment event has occurred respectively during the plurality of time increments respectively based on the incremental number of comparisons, and terminating the electrolytic detachment cycle at the end of the time increment at which the successful electrolytic detachment event has been assessed to have occurred.
194. The method of claim 189, further comprising determining if an electrolytic fault has occurred during the delivery of the electrical current to the electrolytically severable joint, wherein the electrolytic detachment cycle is extended only if the electrolytic fault has not been determined to have occurred during the delivery of the electrical current to the electrolytically severable joint.
195. The method of claim 194, wherein the electrolytic fault is determined to have occurred during the delivery of the electrical current to the electrolytically severable joint if the measured electrolytic work is outside of an electrolytic work range defined by a minimum electrolytic work limit and a maximum electrolytic work limit.
196. The method of claim 189, further comprising tracking a number of completed electrical detachment cycles, wherein the electrolytic detachment cycle is not extended if the tracked number of completed electrolytic detachment cycles exceeds a predefined limit of electrolytic detachment cycles.
197. The method of claim 184, further comprising:
   measuring an elapsed time between a delivery wire decoupling event and a subsequent delivery wire coupling event; and
   resetting the incrementally measured electrolytic work and the incrementally increased electrolytic work threshold if the measured elapsed time reaches a first elapsed time threshold.
198. The method of claim 14, wherein the first elapsed time threshold is equal to or greater than three seconds.
199. The method of claim 14, wherein the first elapsed time threshold is in the range of 25-45 seconds.
200. The method of claim 14, wherein the measuring electrolytic work is reset if the measured elapsed time reaches the first elapsed time threshold and the successful electrolytic detachment event has been assessed to have occurred, and the measuring electrolytic work is reset if the measured elapsed time reaches a second elapsed time threshold longer than the first elapsed time threshold and the successful electrolytic detachment event has been assessed to have not occurred.
201. The method of claim 200, wherein the first elapsed time threshold is in the range of 3-10 seconds, and the second elapsed time threshold is in the range of 25-45 seconds.
202. The method of claim 200, further comprising:
   disposing another vaso-occlusive assembly within the delivery catheter, such that both the vaso-occlusive device and other vaso-occlusive device are disposed outside of the delivery catheter within the vasculature of the patient, the other vaso-occlusive assembly comprising a delivery wire and a vaso-occlusive device detachably coupled to a distal end of the delivery wire via an electrolytically severable joint; and
   electrically coupling a proximal end of the delivery wire of the other vaso-occlusive assembly to the power source after assessing if the successful electrolytic detachment event has occurred; and
   resetting the measuring electrolytic work after the proximal end of the delivery wire of the other vaso-occlusive assembly has been electrically coupled to the power source.
203. The method of claim 184, further comprising:
   generating a first user-discernible notification if the successful electrolytic detachment event has been assessed to have occurred; and
   generating a second user-discernible notification different from the first user-discernible notification if the successful electrolytic detachment event has been assessed to have not occurred.
204. The method of claim 184, wherein the vaso-occlusive device is a vaso-occlusive coil.
205. The method of claim 184, wherein the vaso-occlusive device is disposed within an aneurysmal sac in the vasculature of the patient.

## Claims

1. An electrolytic detachment device (22) for use with an electrically conductive delivery wire (24) having a distal end to which a respective vaso-occlusive device (26) is attached and an electrolytically severable joint (28) located proximal to the vaso-occlusive device (26), comprising:
a power terminal (64) to which a proximal end of each of the delivery wire (24) is configured for being electrically coupled;
electrical current delivery circuitry (76) configured for delivering electrical current to the electrolytically severable joint (28) when disposed within the vasculature of the patient, such that the vaso-occlusive device (26) electrolytically detaches from the distal end of the delivery wire (24);
an electrolytic detachment detection circuit (78) configured for generating electrical parameter information indictive of an electrolytic detachment event; and
a controller (72) configured for operating the electrical current delivery circuitry (76) to deliver the electrical current to the electrolytically severable joint (28) during an electrolytic detachment cycle (90) having a fixed time period (92), assessing if a successful electrolytic detachment event has occurred during the fixed time period (92) based on the generated electrical parameter information, and extending the electrolytic detachment cycle (90) only if the successful electrolytic detachment event has been assessed to have not occurred during the fixed time period (92).

2. The electrolytic detachment device (22) of claim 1, wherein the electrical parameter information comprises a measured cumulative electrical parameter.

3. The electrolytic detachment device (22) of claim 2, wherein the cumulative electrical parameter comprises measured cumulative current.

4. The electrolytic detachment device (22) of claim 1, wherein the electrical parameter information comprises measured electrolytic work, Wₘₑₐₛ, performed by the electrical current delivery circuitry (76).

5. The electrolytic detachment device (22) of claim 1, wherein the fixed time period (92) is an initial fixed time period, and wherein the controller (72) is configured for extending the electrolytic detachment cycle (90) by a subsequent variable time period (94).

6. The electrolytic detachment device (22) of claim 5, wherein the controller (72) is configured for incrementally assessing if the successful electrolytic detachment event has occurred during the subsequent variable time period (94), and terminating the electrolytic detachment cycle (90) when the successful electrolytic detachment event is assessed to have occurred.

7. The electrolytic detachment device (22) of claim 6, wherein the subsequent variable time period (94) has a plurality time increments (96), each of which is shorter than the initial fixed time period (92) of the electrolytic detachment cycle (90), and wherein the controller (72) is configured for incrementally assessing that if the successful electrolytic detachment event has occurred respectively during the plurality of time increments (96), and terminating the electrolytic detachment cycle (90) at the end of the time increment (96) at which the successful electrolytic detachment event has been assessed to have occurred.

8. The electrolytic detachment device (22) of claim 1, wherein the controller (72) is configured for determining if an electrolytic fault has occurred during the delivery of the electrical current to the electrolytically severable joint (28), and extending the electrolytic detachment cycle (90) only if the electrolytic fault has not been determined to have occurred during the delivery of the electrical current to the electrolytically severable joint (28).

9. The electrolytic detachment device (22) of claim 8, wherein the electrical parameter information comprises measured electrolytic work, Wₘₑₐₛ, performed by the electrical current delivery circuitry (76), and wherein the controller (72) is configured for determining that the electrolytic fault has occurred during the delivery of the electrical current to the electrolytically severable joint (28) if the measured electrolytic work, Wₘₑₐₛ, is outside of an electrolytic work range defined by a minimum electrolytic work limit, Wₗᵢₘ₋ₘᵢₙ, and a maximum electrolytic work limit, Wₗᵢₘ₋ₘₐₓ.

10. The electrolytic detachment device (22) of claim 1, further comprising an electrical current delivery actuator (70), wherein the controller (72) is configured for operating the electrical current delivery circuitry (76) to deliver the electrical current to the electrolytically severable joint (28) during the electrolytic detachment cycle (90) in response to a single actuation of the electrical current delivery actuator (70).

11. The electrolytic detachment device (22) of claim 10, wherein the controller (72) is configured for operating the electrical current delivery circuitry (76) to deliver the electrical current to the electrolytically severable joint (28) during another electrolytic detachment cycle (90) having an initial fixed time period (92) in response to another single actuation of the electrical current delivery actuator (70).

12. The electrolytic detachment device (22) of claim 1, further comprising a counter (82) configured for tracking a number of completed electrical detachment cycles (90), wherein the controller (72) is configured for not extending the electrolytic detachment cycle (90) if the tracked number of completed electrolytic detachment cycles (90) exceeds a predefined number of electrolytic detachment cycles (90).

13. The electrolytic detachment device (22) of claim 1, further comprising a timer (80) configured for measuring an elapsed time between a delivery wire (24) decoupling event and a subsequent delivery wire (24) coupling event, wherein the controller (72) is configured for resetting the electrical parameter information if the measured elapsed time reaches a first elapsed time threshold, Tₜₕ, Tₜₕ₁, Tₜₕ₂.

14. The electrolytic detachment device (22) of claim 1, wherein the electrical parameter information comprises measured electrolytic work, Wₘₑₐₛ, performed by the electrical current delivery circuitry (76), and wherein the controller (72) is further configured for assessing that the successful electrolytic detachment event to has not occurred during the fixed time period (92) of the electrolytic detachment cycle (90) if the measured electrolytic work, Wₘₑₐₛ, has not reached an electrolytic work threshold, Wₜₕ, and assessing that the successful electrolytic detachment event has occurred during the fixed time period (92) of the electrolytic detachment cycle (90) if the measured electrolytic work has reached the electrolytic work threshold, Wₜₕ.

15. The electrolytic detachment device (22) of claim 14, wherein the controller (72) is configured for incrementally increasing the electrolytic work threshold, Wₜₕ, over the extended electrolytic detachment cycle (90).
